# EUROPEAN PATENT SPECIFICATION

(11) **EP 0 830 136 B1**
(45) Date of publication and mention of the grant of the patent: **20.10.2004**
(21) Application number: 96910399.3
(22) Date of filing: 07.03.1996
(51) Int. Cl.: A61K 38/00, C07K 5/12, A61K 31/395, C07D 273/00, A61P 35/00

(54) **NEW CRYPTOPHYCINS FROM SYNTHESIS**
NEUE SYNTHETISCHE CRYPTOPHYCINE
NOUVELLES CRYPTOPHYCINES OBTENUES PAR SYNTHESE

(30) Priority: 07.03.1995 US 400057; 07.06.1995 US 482141
(43) Date of publication of application: 25.03.1998
(73) Proprietor: UNIVERSITY OF HAWAII, Honolulu, HI 96822 (US); WAYNE STATE UNIVERSITY, Detroit, MI 48202 (US)
(72) Inventor: MOORE, Richard, E., Honolulu, HI 98816 (US); TIUS, Marcus, A., Kailua, HI 96734 (US); BARROW, Russell, A., Honolulu, HI 96813 (US); LIANG, Jian, Honolulu, HI 96813 (US); CORBETT, Thomas, H., Grosse Point, MI 48230 (US); VALERIOTE, Frederick, A., Shelby Township, MI 48316 (US); HEMSCHEIDT, Thomas, K., Honolulu, HI 96821 (US); Golakoti, Trimurtulu, Honolulu, HI 96822 (US)
(74) Representative: Baldock, Sharon Claire
(86) International application number: PCT/US1996/003246
(87) International publication number: WO 1996/040184

(56) References cited:
- EP-B- 0 735 819
- WO-A-95/17093
- TRIMURTULU ET AL.: "Total structures of cryptophycins, potent antitumor..." J. AM. CHEM. SOC., vol. 116, no. 11, 1994, pages 4729-4737, XP002153400
- SMITH ET AL.: "Cryptophycin: A new antimicrotubule agent active..." CANCER RESEARCH, vol. 54, 15 July 1994 (1994-07-15), pages 3779-3784, XP002153401
- FEBS LETTERS, issued 1995, KERKSIEK et al., "Interaction of Cryptophycin 1 with Tubulin and Microtubules", pages 59-61.
- CHEMICAL PHARMACEUTICAL BULLETIN, 42(10), issued 1994, KOBAYASHI et al., "The Absolute Stereostructure of Arenastatin A, A Potent Cytotoxic Depsipeptide from the Okinawan Marine Sponge Dysidea Arenaria", pages 2196-2198.

## Description

This invention was made in part with U.S. Government support under Grant Nos. CA12623 and CA53001 from The National Cancer Institute, Department of Health and Human Services. Accordingly, the U.S. Government may have certain rights in this invention.

### Background of the Invention

Neoplastic diseases, characterized by the proliferation of cells not subject to the normal control of cell growth, are a major cause of death in humans. Clinical experience in chemotherapy has demonstrated that new and more effective drugs are desirable to treat these diseases. Such experience has also demonstrated that drugs which disrupt the microtubule system of the cytoskeleton can be effective in inhibiting the proliferation of neoplastic cells.

The microtubule system of eucaryotic cells is a major component of the cytoskeleton and is in a dynamic state of assembly and disassembly; that is, heterodimers of tubulin are polymerized to form microtubules, and microtubules are depolymerized to their constituent components. Microtubules play a key role in the regulation of cell architecture, metabolism, and division. The dynamic state of microtubules is critical to their normal function. With respect to cell division, tubulin is polymerized into microtubules that form the mitotic spindle. The microtubules are then depolymerized when the mitotic spindle's use has been fulfilled. Accordingly, agents which disrupt the polymerization or depolymerization of microtubules, and thereby inhibit mitosis, comprise some of the most effective chemotherapeutic agents in clinical use.

Such anti-mitotic agents or poisons may be classified into three groups on the basis of their molecular mechanism of action. The first group consists of agents, including colchicine and colcemid, which inhibit the formation of microtubules by sequestering tubulin. The second group consist of agents, including vinblastine and vincristine, which induce the formation of paracrystalline aggregates of tubulin. Vinblastine and vincristine are well known anticancer drugs: Their action of disrupting mitotic spindle microtubules preferentially inhibits hyperproliferative cells. The third group consists of agents, including taxol, which promotes the polymerization of tubulin and thus stabilizes microtubule structures.

However, merely having activity as an antimitotic agent does not guarantee efficacy against a tumor cell, and certainly not a tumor cell which exhibits a drug-resistant phenotype. Vinca alkaloids such as vinblastine and vincristine are effective against neoplastic cells and tumors, yet they lack activity against some drug-resistant tumors and cells. One basis for a neoplastic cell displaying drug resistance (DR) or multiple-drug resistance (MDR) is through the over-expression of P-glycoprotein. Compounds which are poor substrates for transport of P-glycoprotein should be useful in circumventing such DR or MDR phenotypes.

Accordingly, the exhibition of the DR or MDR phenotype by many tumor cells and the clinically proven mode of action of anti-microtubule agents against neoplastic cells necessitates the development of anti-microtubule agents cytotoxic to non-drug resistant neoplastic cells as well as cytotoxic to neoplastic cells with a drug resistant phenotype. Agents which have shown promise in this regard include a class of compounds known as cryptophycins.

Trimurtula et al. "Total structures of cryptophycins, potent antitumor depsipeptides from the blue-green alga nostoc sp. Strain GSV 224", Journal of the American Chemical Society, vol. 116, no.11, 1994, pp. 4729-4737, discloses cryptophycin compounds isolated from nostoc sp. strain GSV 224. This reference also discloses derivatives and degradation products of the naturally occurring cryptophycins.

With respect to methods of producing cryptophycins, no method for total synthesis of cryptophycins is currently known. Cryptophycin compounds are presently produced via isolation from blue-green alga or are semi-synthetic variations of such naturally produced compounds. The lack of a total synthetic method necessarily makes it difficult to produce stereospecific cryptophycins which can maximize activity and increase the stability of the compound. For example, research has shown that cryptophycins with an intact macrocyclic ring are more active. Accordingly, a total synthetic method which could produce cryptophycins with a macrocyclic ring that is more stable than naturally derived cryptophycins would be desirable. The present invention solves these problems.

### Disclosure of the Invention

The present invention provides novel cryptophycin compounds having the following structure: 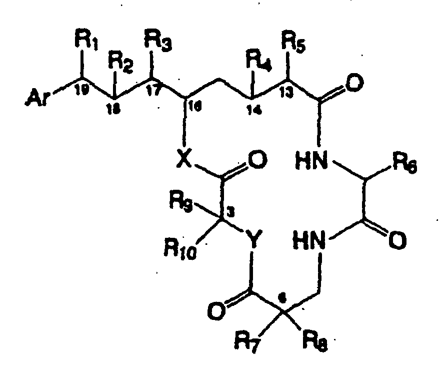 wherein
Ar is methyl or phenyl or any simple unsubstituted or substituted aromatic or heteroaromatic group;
R₁ is a halogen, SH, amino, monoalkylamino, dialkylamino, trialkylammonium, alkylthio, dialkylsulfonium, sulfate, or phosphate;
R₂ is OH or SH; or
R₁ and R₂ may be taken together to form an epoxide ring, an aziridine ring, an episulfide ring, a sulfate ring or a monoalkylphosphate ring; or
R₁ and R₂ may be taken together to form a double bond between C₁₈ and C₁₉;
R₃, R₇ and R₈ are each alkyl groups of one to five carbon atoms including linear and non linear hydrocarbon chains;
R₄ and R₅ are H; or
R₄ and R₅ may be taken together to form a double bond between C₁₃ and C₁₄;
R₆ is a benzyl, hydroxybenzyl, alkoxybenzyl, halohydroxybenzyl, dihalohydroxybenzyl, haloalkoxybenzyl, or dihaloalkoxybenzyl group;
R₉ and R₁₀ are each independently H or an alkyl group of one to five carbon atoms including linear and non linear hydrocarbon chains; and
X and Y are each independently O, NH or alkylamino.

The present invention further provides total synthetic methods for producing cryptophycins. The present invention also provides for the use of cryptophycins in pharmaceuticals, to inhibit the proliferation of mammalian cells and to treat neoplasia.

### Brief Description of the Drawirigs

Figure 1 provides a general structure of selected cryptophycin compounds of the present invention and a numbering system for the hydroxy acid units A and D and amino acid units B and C in selected embodiments;
Figures 2A and B graphically depict the effects of cryptophycin compounds and vinblastine on Jurkat cell proliferation and cell cycle progression. Jurkat cells were incubated with the indicated concentrations of cryptophycin compounds (A) or vinblastine (B) for 24 hours. For each sample, the number of viable cells (■) and the mitotic index (□) were determined as described in the Experimental Section. Values represent the means ± standard deviation (sd) for triplicate samples in one of three similar experiments;

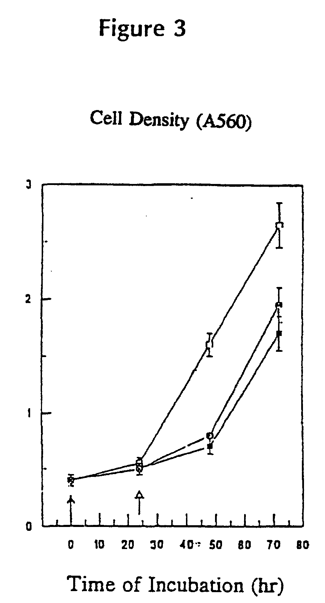

Figure 3 graphically depicts the reversibility of the effects of vinblastine, cryptophycins and taxol on cell growth. SKOV3 cells were treated with 0.1nM vinblastine (□), 0.1nM cryptophycins (■) or 1nM taxol ( 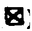 ) at time = 0. These concentrations inhibited cell growth by 50% for each compound. After 24 hours the cells were washed and incubated in drug-free medium for the time indicated. The cell density was determined by sulforhodamine B (SRB) staining as described in the Experimental Section, and is expressed as the mean ± sd absorbance at 560 nm for triplicate samples in one of three experiments;
Figure 4 provides Isobolograms for combinational effects of vinblastine and cryptophycins on cell proliferation. SKOV3 cells were treated with vinblastine (0-600pM) and/or cryptophycins (1-100pM) for 48 hours. Cell numbers were then determined by SRB staining as described in the Experimental Section, and the IC₅₀s (■) and the line of additivity (----) were determined for combinations of vinblastine and cryptophycin compounds. Values represent means for two experiments each containing triplicate samples;
Figure 5 provides a first scheme for synthesizing cryptophycins in accordance with the present invention;
Figure 6 provides a scheme for producing a hydroxy acid unit A;
Figure 7 provides a scheme for producing the subunit of a cryptophycin comprising a hydroxy acid unit A and amino acid B;
Figure 8 provides a scheme for producing the subunit of a cryptophycin comprising an amino acid unit C and hydroxy acid D;
Figure 9 provides a first scheme for synthesizing selected cryptophycins in accordance with the present invention;
Figure 10 provides a second scheme for synthesizing selected cryptophycins in accordance with the present invention;
Figure 11 provides a scheme for synthesizing a subunit of a cryptophycin comprising a hydroxy acid D;
Figure 12 provides a third scheme for synthesizing selected cryptophycins in accordance with the present invention;
Figure 13 provides a fourth scheme for synthesizing selected cryptophycins in accordance with the present invention; and
Figure 14 provides a fifth scheme for synthesizing selected cryptophycins in accordance with the present invention.

### Detailed Description of the Invention

The present invention provides novel cryptophycin compounds having the following structure: 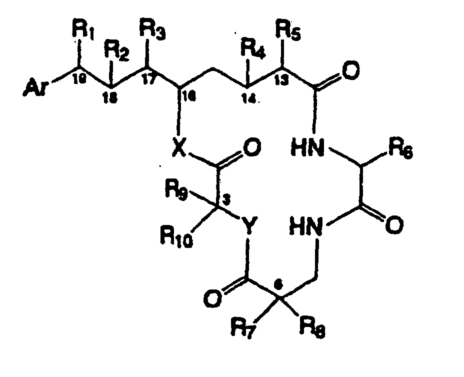 wherein
Ar is methyl or phenyl or any simple unsubstituted or substituted aromatic or heteroaromatic group;
R₁ is a halogen, SH, amino, monoalkylamino, dialkylamino, trialkylammonium, alkylthio, dialkylsulfonium, sulfate, or phosphate;
R₂ is OH or SH; or
R₁ and R₂ may be taken together to form an epoxide ring, an aziridine ring, an episulfide ring, a sulfate ring or a monoalkylphosphate ring; or
R₁ and R₂ may be taken together to form a double bond between C₁₈ and C₁₉;
R₃, R₇ and R₈ are each alkyl groups of one to five carbon atoms including linear and non linear hydrocarbon chains;
R₄ and R₅ are H; or
R₄ and R₅ may be taken together to form a double bond between C₁₃ and C₁₄;
R₆ is a benzyl, hydroxybenzyl, alkoxybenzyl, halohydroxybenzyl, dihalohydroxybenzyl, haloalkoxybenzyl, or dihaloalkoxybenzyl group;
R₉ and R₁₀ are each independently H or an alkyl group of one to five carbon atoms including linear and non linear hydrocarbon chains; and
X and Y are each independently O, NH or alkylamino.

As used herein, the following terms have the indicated meanings unless a contrary meaning is clearly intended from the use in context:
"lower β-amino acid" means any β-amino acid having three to eight carbons and includes linear and non-linear hydrocarbon chains; for example, 3-amino-2-methylpropionic acid.
"esterified lower β-amino acid" means any β-amino acid having three to eight carbons where the hydrogen of the carboxylic acid group is substituted with a methyl group; for example, 3-amino-2-methylpropionic acid methyl ester.
"lower alkanoyloxy group" means an alkanoyloxy group of one to seven carbons and includes linear and non-linear hydrocarbon chains.
"lower α-hydroxyalkanoyloxy group" means an α-hydroxyalkanoyloxy group of two to seven carbons and includes linear and non-linear hydrocarbon chains; for example, 2-hydroxy-4-methylvaleric acid.
"lower alkoxyl group" means any alkyl group of one to five carbons bonded to an oxygen atom.
"lower alkyl group" means an alkyl group of one to five carbons and includes linear and non-linear hydrocarbon chains including, for example, methyl, ethyl, propyl, isopropyl, butyl, isobutyl, tert-butyl, sec-butyl, methylated butyl groups, pentyl, and tert-pentyl groups.
"allylically substituted alkene" means any alkene which contains an alkyl substitution.
"epoxide ring" means a three-membered ring whose backbone consists of two carbons and an oxygen atom.
"aziridine ring" means a three-membered ring whose backbone consists of two carbons and a nitrogen atom.
"episulfide ring" means a three-membered ring whose backbone consists of two carbons and a sulfur atom.
"sulfate ring" means a five-membered ring consisting of a carbon-carbon-oxygen-sulfur-oxygen backbone with two additional oxygen atoms connected to the sulfur atom.
"monoalkylphosphate ring" means a five-membered ring consisting of a carbon-carbon-oxygen-phosphorus-oxygen backbone with two additional oxygen atoms, one of which bears a lower alkyl group, connected to the phosphorus atom.
"simple unsubstituted aromatic group" refers to common aromatic rings having 4n+2 pi electrons in a monocyclic conjugated system (for example, furyl, pyrrolyl, thienyl, pyridyl) or a bicyclic conjugated system (for example, indolyl or naphthyl).
"simple substituted aromatic group" refers to a phenyl group substituted with single group (e.g. a lower alkyl group or a halogen).
"heteroaromatic group" refers to aromatic rings which contain one or more non-carbon substituents such as oxygen, nitrogen, or sulfur.
"halogen" refers to those members of the group on the periodic table historically known as the halogens. Methods of halogenation include, but are not limited to, the addition of hydrogen halides, substitution at high temperature, photohalogenation, etc., and such methods are known to those of ordinary skill in the art.^{1,2}

Throughout the disclosure, reference is made to specific cryptophycins by number. Unless stated otherwise, the cryptophycin correspond to compounds having the following structure: 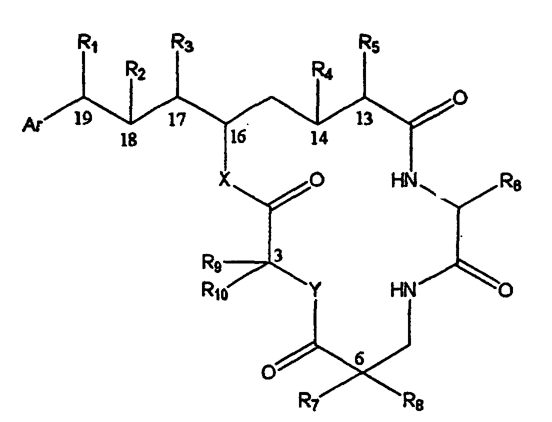 where the numbers refer to compounds as presented in the following table:

| Cpd | Ar | R¹ | R² | R³ | R⁴ | R⁵ | R⁶ | R⁷ | R⁸ | R⁹ | R¹⁰ | X | Y |
|---|---|---|---|---|---|---|---|---|---|---|---|---|---|
| 1 | phenyl | epoxide | epoxide | methyl | db | db | 3C4M | methyl | H | isobutyl | H | O | O |
| 2 | phenyl | epoxide | epoxide | methyl | db | db | 4-MB | methyl | H | isobutyl | H | O | O |
| 3 | phenyl | db | db | methyl | db | db | 3C4M | methyl | H | isobutyl | H | O | O |
| 4 | phenyl | db | db | methyl | db | db | 4-MB | methyl | H | isobutyl | H | O | O |
| 8 | phenyl | Cl | OH | methyl | db | db | 3C4M | methyl | H | isobutyl | H | O | O |
| 9 | phenyl | methoxy | OH | methyl | db | db | 3C4M | methyl | H | isobutyl | H | O | O |
| 15 | phenyl | OH | OH | methyl | db | db | 3C4M | methyl | H | isobutyl | H | O | O |
| 16 | phenyl | epoxide | epoxide | methyl | db | db | 3C4OH | methyl | H | isobutyl | H | O | O |
| 17 | phenyl | db | db | methyl | db | db | 3C4OH | methyl | H | isobutyl | H | O | O |
| 18 | phenyl | db | db | methyl | db | db | 3C4M | methyl | H | isobutyl | H | O | O |
| 19 | phenyl | db | db | methyl | db | db | 3C4M | methyl | H | isobutyl | H | O | O |
| 20 | phenyl | H | O | methyl | db | db | 3C4M | methyl | H | isobutyl | H | O | O |
| 21 | phenyl | epoxide | epoxide | methyl | db | db | 3C4M | H | H | isobutyl | H | O | O |
| 23 | phenyl | epoxide | epoxide | methyl | db | db | 3,5C4O4 | methyl | H | isobutyl | H | O | O |
| 24 | phenyl | epoxide | epoxide | methyl | db | db | 4-MB | H | H | isobutyl | H | O | O |
| 25 | phenyl | Br | OH | methyl | db | db | 3C4M | methyl | H | isobutyl | H | O | O |
| 27 | phenyl | Cl | OH | methyl | db | db | 3C4M | methyl | H | isobutyl | H | O | O |
| 28 | phenyl | db | db | H | db | db | 3C4M | methyl | H | isobutyl | H | O | O |
| 29 | phenyl | db | db | methyl | db | db | 3C4M | H | H | isobutyl | H | O | O |
| 30 | phenyl | db | db | methyl | OH | H | 3C4M | methyl | H | isobutyl | H | O | O |
| 31 | phenyl | epoxide | epoxide | methyl | db | db | 3,5C4M | methyl | H | isobutyl | H | O | O |
| 32 | phenyl | epoxide | epoxide | methyl | H | H | 3C4M | H | H | isobutyl | H | O | O |
| 33 | phenyl | H | OH | methyl | H | H | 3C4M | methyl | H | isobutyl | H | O | O |
| 34 | phenyl | H | H | methyl | H | H | 4-MB | methyl | H | isobutyl | H | O | O |
| 35 | phenyl | epoxide | epoxide | methyl | H | H | 3C4M | methyl | H | isobutyl | H | O | O |
| 37 | phenyl | Br | OH | methyl | db | db | 3C4M | methyl | H | isobutyl | H | O | O |
| 38 | phenyl | epoxide | epoxide | methyl | db | db | 3C4M | methyl | H | isobutyl | H | O | O |

| Cpto | Ar | R¹ | R² | R³ | R⁴ | R⁵ | R⁶ | R⁷ | R⁸ | R⁹ | R¹⁰ | X | Y |
|---|---|---|---|---|---|---|---|---|---|---|---|---|---|
| 39 | phenyl | epoxide | epoxide | methyl | db | db | 3C4M | methyl | H | isobutyl | H | O | O |
| 40 | phenyl | epoxide | epoxide | H | db | db | 3C4M | methyl | H | isobutyl | H | O | O |
| 41 | phenyl | epoxide | epoxide | methyl | OH | OH | 3C4M | methyl | H | isobutyl | H | O | O |
| 42 | phenyl | epoxide | epoxide | methyl | OH | OH | 3C4M | methyl | H | isobutyl | H | O | O |
| 45 | phenyl | db | db | methyl | db | db | 3,5C4OH | methyl | H | isobutyl | H | O | O |
| 48 | phenyl | H | OH | methyl | db | db | 3C4M | methyl | H | isobutyl | H | O | O |
| 49 | phenyl | epoxide | epoxide | methyl | db | db | 3C4M | methyl | H | propyl | H | O | O |
| 50 | phenyl | db | db | methyl | db | db | 3C4M | methyl | H | propyl | H | O | O |
| 51 | phenyl | db | db | methyl | db | db | 3C4M | methyl | methyl | isobutyl | H | O | O |
| 52 | phenyl | epoxide | epoxide | methyl | db | db | 3C4M | methyl | methyl | isobutyl | H | O | O |
| 53 | phenyl | epoxide | epoxide | methyl | db | db | 3C4M | methyl | methyl | isobutyl | H | O | O |
| 54 | phenyl | epoxide | epoxide | methyl | db | db | 3C4M | methyl | H | isobutyl | H | O | O |
| 55 | phenyl | Cl | OH | methyl | db | db | 3C4M | methyl | methyl | isobutyl | H | O | O |
| 57 | phenyl | epoxide | epoxide | methyl | H | H | 3C4M | methyl | methyl | isobutyl | H | O | O |
| 58 | phenyl | Cl | OH | methyl | H | H | 3C4M | methyl | methyl | isobutyl | H | O | O |
| 59 | phenyl | Cl | OH | methyl | H | H | 3C4M | methyl | H | isobutyl | H | O | O |
| 60 | phenyl | episulfid e | episulfid e | methyl | db | db | 3C4M | methyl | H | isobutyl | H | O | O |
| 61 | phenyl | episulfide | episulfide | methyl | db | db | 3C4M | methyl | methyl | isobutyl | H | O | O |
| 63 | phenyl | Cl | OH | H | db | db | 3C4M | methyl | H | isobutyl | H | O | O |
| 64 | phenyl | Cl | OH | methyl | H | H | 3C4M | methyl | H | isobutyl | H | O | O |
| 69 | phenyl | Cl | OH | methyl | db | db | 3C4M | methyl | H | isobutyl | H | O | O |
| 70 | phenyl | Cl | OH | methyl | db | db | 3C4M | methyl | H | isobutyl | H | O | O |
| 71 | phenyl | Br | OH | methyl | db | db | 3C4M | methyl | H | isobutyl | H | O | O |
| 72 | phenyl | Br | OH | methyl | db | db | 3C4M | methyl | H | isobutyl | H | O | O |
| 73 | phenyl | Cl | OH | methyl | db | db | 3C4M | H | H | isobutyl | H | O | O |
| 74 | phenyl | Cl | OH | methyl | db | db | 4-MB | mehtyl | H | isobutyl | H | O | O |
| 75 | phenyl | F | OH | methyl | db | db | 3C4M | methyl | H | isobutyl | H | O | O |
| 76 | phenyl | F | OH | methyl | db | db | 3C4M | methyl | H | isobutyl | H | O | O |
| 81 | MeO-phenyl | db | db | methyl | db | db | 3C4M | methyl | H | isobutyl | H | O | O |
| 82 | Me | db | db | methyl | db | db | 3C4M | methyl | H | isobutyl | H | O | O |
| 90 | Me | epoxid | epoxid | methyl | db | db | 3C4M | methyl | H | isobutyl | H | O | O |
| 91 | Me | epoxide | epoxid | methyl | db | db | 3C4M | methyl | H | isobutyl | H | O | O |
| 97 | phenyl | aziridine | aziridine | methyl | db | db | 3C4M | methyl | methyl | isobutyl | H | O | O |
| 108 | * | * | * | methyl | db | db | 3C4M | methyl | H | isobutyl | H | O | O |
| 110 | F-phenyl | db | db | methyl | db | db | 3C4M | methyl | H | isobutyl | H | O | O |
| 111 | Mephenyl | db | db | methyl | db | db | 3C4M | methyl | H | isobutyl | H | O | O |
| 112 | thiophene | db | db | methyl | db | db | 3C4M | methyl | H | isobutyl | H | O | O |
| 115 | F-phenyl | epoxide | epoxide | methyl | db | db | 3C4M | methyl | H | isobutyl | H | O | O |
| 116 | F-phenyl | epoxide | epoxide | methyl | db | db | 3C4M | methyl | H | isobutyl | H | O | O |
| 117 | Mephenyl | epoxide | epoxide | methyl | db | db | 3C4M | methyl | H | isobutyl | H | O | O |
| 118 | Mephenyl | epoxide | epoxide | methyl | db | db | 3C4M | methyl | H | isobutyl | H | O | O |
| 119 | thiophene | epoxide | epoxide | methyl | db | db | 3C4M | methyl | H | isobutyl | H | O | O |
| 120 | thiophene | epoxide | epoxide | methyl | db | db | 3C4M | methyl | H | isobutyl | H | O | O |
| 121 | phenyl | db | db | methyl | db | db | 3C4M | methyl | H | isobutyl | H | O | N |
| 122 | phenyl | epoxide | epoxide | methyl | db | db | 3C4M | methyl | H | isobutyl | H | O | N |
| 123 | phenyl | epoxide | epoxide | methyl | db | db | 3C4M | methyl | H | isobutyl | H | O | N |
| 124 | Cl-phenyl | db | db | methyl | db | db | 3C4M | methyl | H | isobutyl | H | O | O |
| 125 | Cl-phenyl | epoxide | epoxide | methyl | db | db | 3C4M | methyl | H | isobutyl | H | O | O |
| 126 | Cl-phenyl | epoxide | epoxide | methyl | db | db | 3C4M | methyl | H | isobutyl | H | O | O |
| 127 | phenyl | Cl | OH | methyl | db | db | 3C4M | methyl | H | isobutyl | H | O | N |
| 128 | Mephenyl | Cl | OH | methyl | db | db | 3C4M | methyl | H | isobutyl, | H | O | O |
| 130 | Mephenyl | Cl | OH | methyl | db | db | 3C4M | methyl | H | isobutyl | H | O | O |
| 131 | Mephenyl | Cl | OH | methyl | db | db | 3C4M | methyl | H | isobutyl | H | O | O |
| 132 | Cl-phenyl | Cl | OH | methyl | db | db | 3C4M | methyl | H | isobutyl | H | O | O |
| 133 | Cl-phenyl | Cl | OH | methyl | db | db | 3C4M | methyl | H | isobutyl | H | O | O |
| 134 | Cl-phenyl | Cl | OH | methyl | db | db | 3C4M | methyl | H | isobutyl | H | O | O |
| db = double bond (where either R₄ and R₅ together from a double bond between C₁₃ and C₁₄ or where R₁ and R₂ together form a double bond between C₁₈ and C₁₉), Me = methyl, 4-MB = 4-methoxy benzyl, 3C4M = 3-chloro-4-methoxybenzyl, 3C4OH = 3-chloro-4-hydroxybenzyl, 3,5C4OH = 3,5-dichloro-4-hydroxybenzyl, and 3,5C4M = 3,5-dichloro-4-methoxybenzyl. | | | | | | | | | | | | | |

| | | | | | | | | | | | | | |
|---|---|---|---|---|---|---|---|---|---|---|---|---|---|
| * C₁₈ is the final carbon of Cryptophycin 108. C₁₈ of cryptophycin 108 is substituted as follows OHC₁₈-C₁₇. See Figure 13. | | | | | | | | | | | | | |

Additional cryptophycins referred to throughout the disclosure include cryptophycins 5, 6, 7, 10, 12, 14, and 26, which are represented by the following structures:

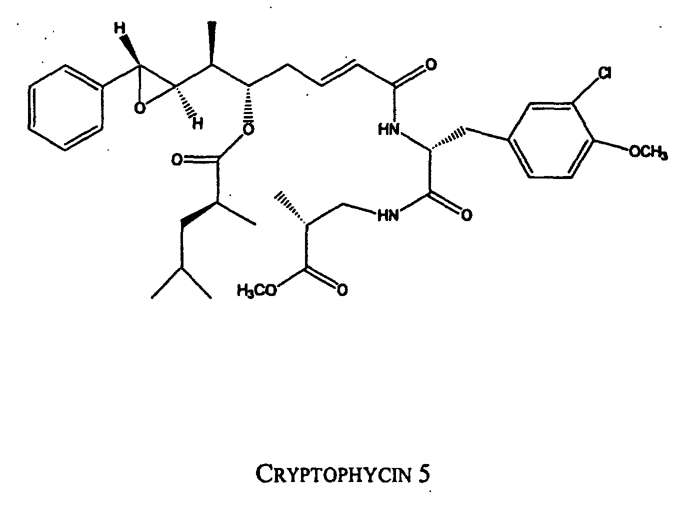

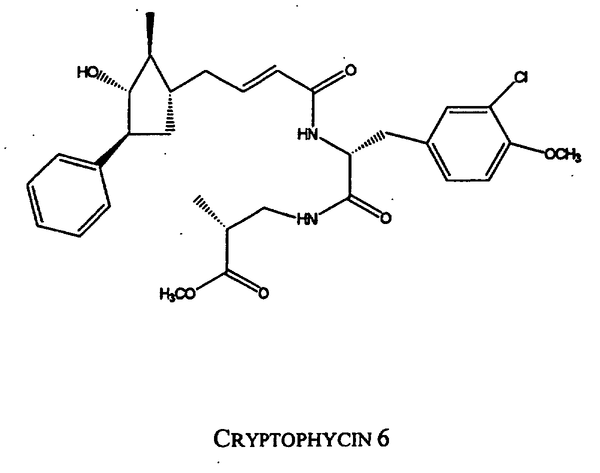

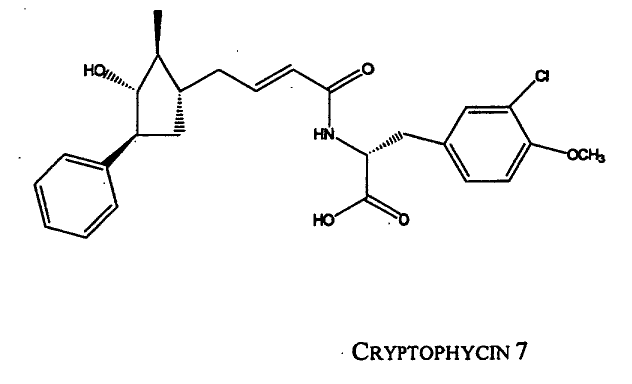

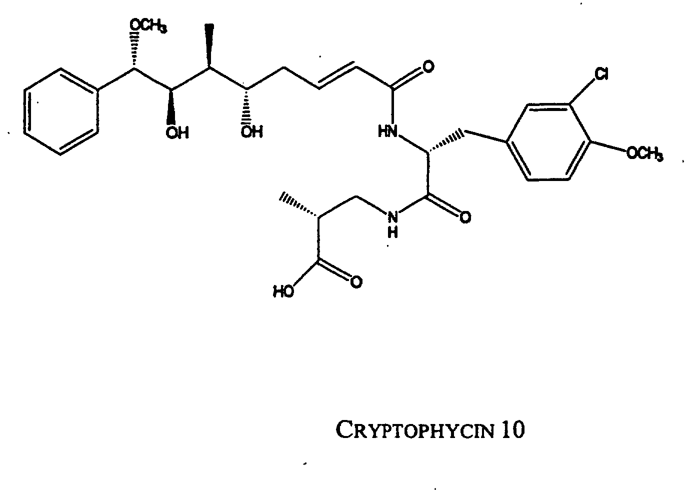

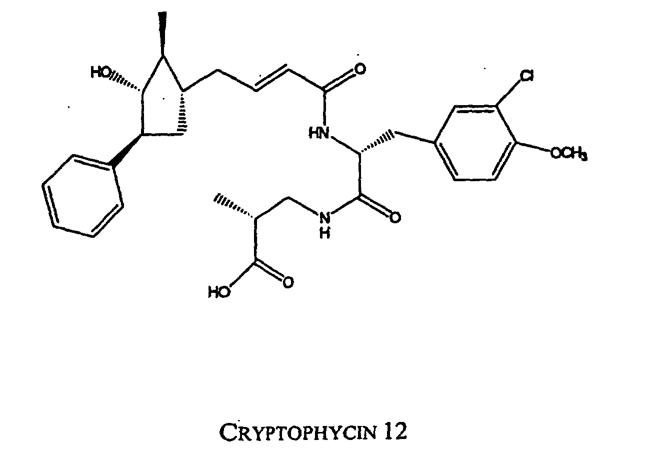

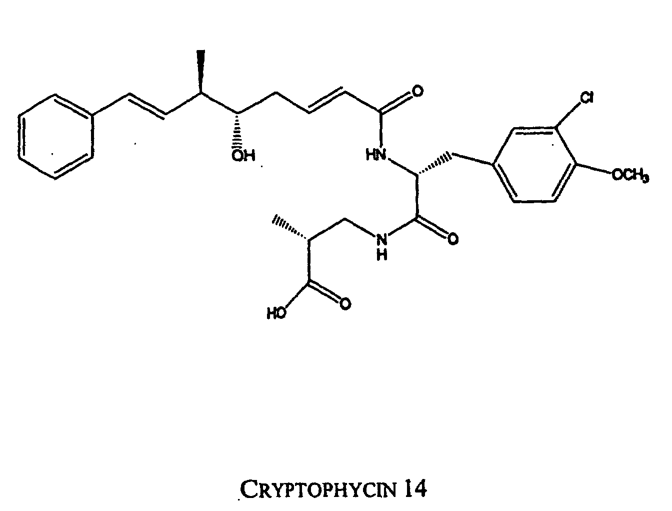

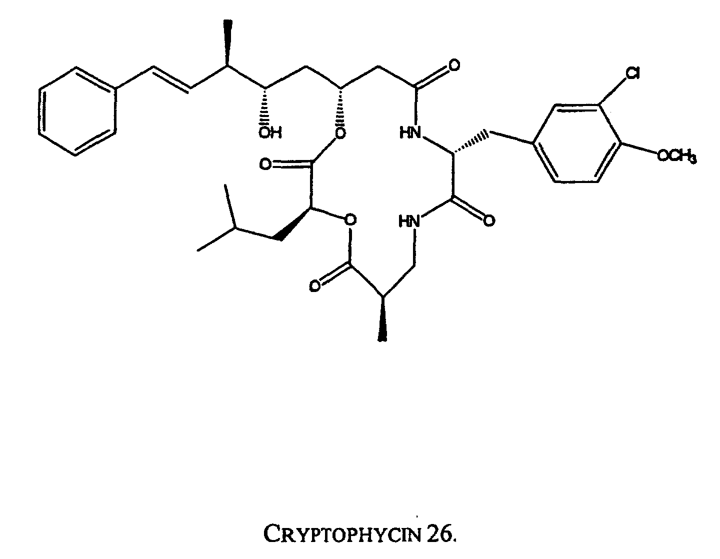

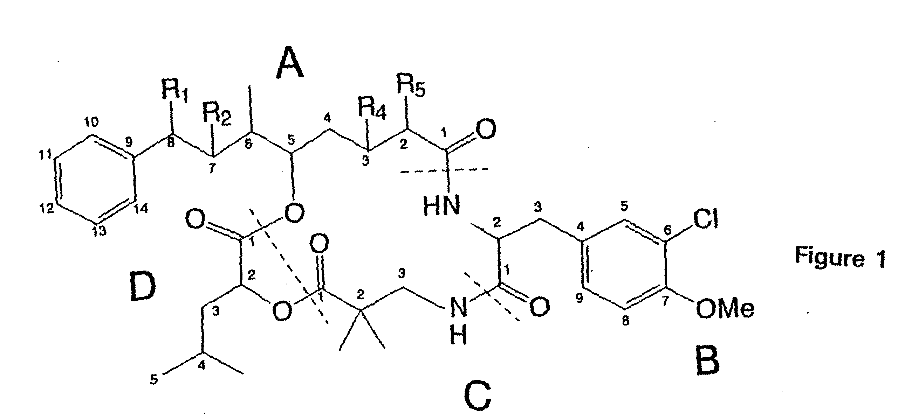

As will be apparent from their structures, the cryptophycin compounds have groups which are capable of chemical modification. The genus compound of the present invention contemplates those cryptophycins which exhibit anti-neoplastic activity. For example, the derivatives exemplified in the present invention include compounds having the epoxide oxygen or hydroxy groups on C-7 and C-8 of unit A or the leucic acid group of unit B of Figure 1. Such derivatives of the novel and previously disclosed compounds which display the desired anti-neoplastic activity are included in the claimed invention.
Moreover, the relationship between the structure of the cryptophycin compounds and anti-neoplastic activity is provided in the Experimental Section hereinbelow.

Twenty-two additional cryptophycin compounds, herein designated Cryptophycins 2, 4, 6, 7, 16-19, 21, 23, 24, 26, 28-31, 40, 43, 45, 49, 50 and 54 are disclosed in U.S. Patent Nos. 5, 955, 423 and 5,945,315, such compounds either being metabolites isolated from a strain of Nostoc sp. or having been semi-synthesized from such metabolites. Also disclosed in these patent applications is the characterization of selected cryptophycin compounds as anti-microtubule agents with clinical-type activity expressed toward a broad spectrum of tumors implanted in mice, including DR and MDR tumors.

The present invention provides novel cryptophycin compounds having the following structure: 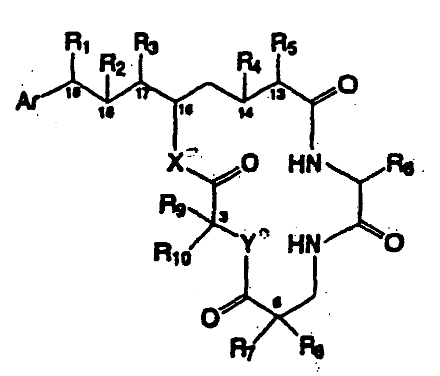 wherein
Ar is methyl or phenyl or any simple unsubsticuced or substituted aromatic or heteroaromatic group;
R₁ is a halogen, SH, amino, monoalkylamino, dialkylamino, trialkylammonium, alkylthio, dialkylsulfonium, sulfate, or phosphate;
R₂ is OH or SH; or
R₁ and R₂ may be taken together to form an epoxide ring, an aziridine ring, an episulfide ring, a sulfate ring or a monoalkylphosphate ring; or
R₁ and R₂ may be taken together to form a double bond between C₁₈ and C₁₉;
R₃, R₇ and R₉ are each alkyl groups of one to five carbon atoms including linear and non linear hydrocarbon chains;
R₄ and R₅ are H; or
R₄ and R₅ may be taken together to form a double bond between C₁₃ and C₁₄;
R₆ is a benzyl, hydroxybenzyl, alkoxybenzyl, halohydroxybenzyl, dihalohydroxybenzyl, haloalkoxybenzyl, or dihaloalkoxybenzyl group;
R₉ and R₁₀ are each independently H or an alkyl group of one to five carbon atoms including linear and non linear hydrocarbon chains; and
X and Y are each independently O, NH or alkylamino.

In a preferred embodiment of this cryptophycin, R₈ of the cryptophycin is ethyl, propyl, isopropyl, butyl, isobutyl, pentyl or isopentyl. In another preferred embodiment of this cryptophycin, R₇ is ethyl, propyl, isopropyl, butyl, isobutyl, pentyl or isopentyl. In an additional preferred embodiment of this cryptophycin, R₇ is H, R₈ is methyl, R₃ is methyl; X and Y are not both O.

The present invention provides an additional preferred embodiment of this cryptophycin wherein R₃ is ethyl, propyl, isopropyl, butyl, isobutyl, pentyl or isopentyl. In another preferred embodiment of this cryptophycin, R₉ is methyl, ethyl, propyl, butyl, isobutyl, pentyl or isopentyl. In a further preferred embodiment of this cryptophycin, R₁₀ is methyl, ethyl, propyl, butyl, isobutyl, pentyl or isopentyl.

The invention further provides cryptophycin compounds wherein at least one of the groups attached to C₃, C₆, C₁₀, C₁₆, C₁₇, and C₁₈ has *R* stereochemistry. In a further embodiment of the invention, at least one of the groups attached to C₃, C₆, C₁₀, C₁₆, C₁₇, and C₁₈ has S stereochemistry.

One embodiment of a cryptophycin compound of the present invention is when Ar is phenyl. R₁ and R₂ are taken together to form a double bond between C₁₈ and C₁₉, R₃, R₇ and R₈ are methyl, R₄ and R₅ are taken together to form a double bond between C₁₃ and C₁₄, R₆ is 3-chloro-4-methoxybenzyl, R₉ is isobutyl, R₁₀ is hydrogen, and X and Y are oxygen. The structure of this compound, Cryptophycin **51,** is as follows:

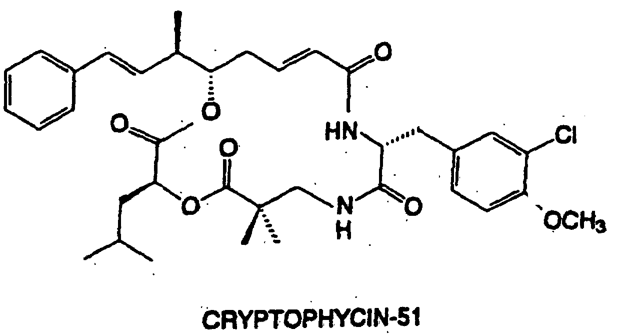

A further embodiment of a compound of the present invention is when Ar is phenyl, R₁ and R₂ are taken together to form an *R,R*-epoxide ring, R₃, R₇ and R₈ are methyl, R₄ and R₅ are taken together to form a double bond between C₁₃ and C₁₄, R₆ is 3-chloro-4-methoxybenzyl, R₉ is isobutyl, R₁₀ is hydrogen, and X and Y are oxygen. The structure of this compound, Cryptophycin **52**, is as follows:

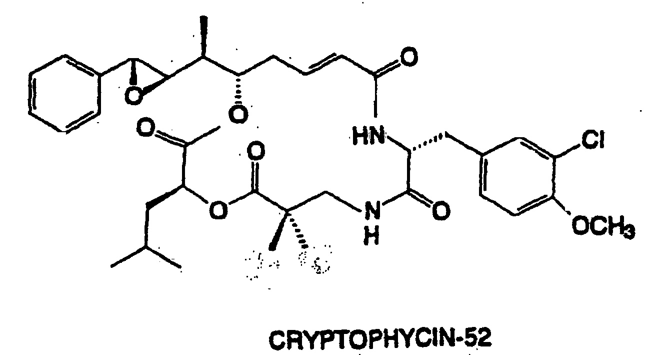

A further embodiment of a compound of the present invention is when Ar is phenyl, R₁ and R₂ are taken together to form an *S*,*S*-epoxide ring, R₃, R₇ and R₈ are methyl, R₄ and R₅ are taken together to form a double bond between C₁₃ and C₁₄, R₆ is 3-chloro-4-methoxybenzyl, R₉ is isobutyl, R₁₀ is hydrogen, and X and Y are oxygen. The structure of this compound, Cryptophycin **53**, is as follows:

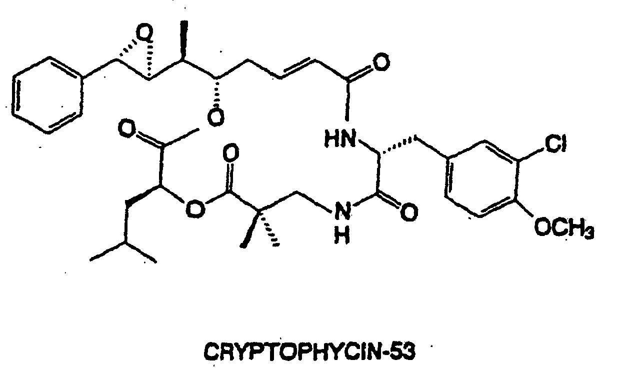

A further embodiment of a compound of the present invention is when Ar is phenyl, R₁ is *S*-chloro, R₂ is *R*-hydroxyl, R₃, R₇ and R₄ are methyl, R₄ and R₅ are taken together to form a double bond between C₁₃ and C₁₄, R₆ is 3-chloro-4-methoxybenzyl, R₉ is isobutyl, R₁₀ is hydrogen, and X and Y are oxygen. The structure of this compound, Cryptophycin **55**, is as follows:

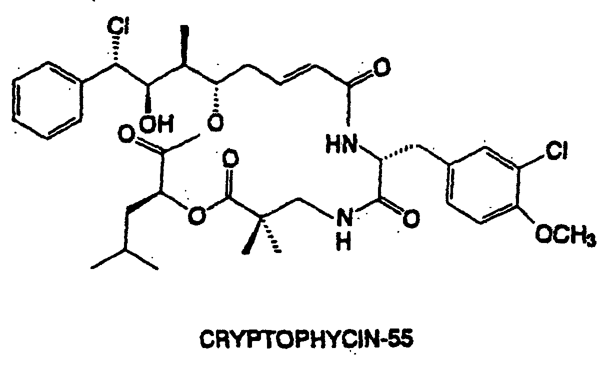

Another embodiment of a compound of the present invention is when Ar is phenyl, R₁ and R₂ are taken together to form an *R,R*-epoxide ring, R₃, R₇ and R₈ are methyl, R₄ and R₅ are hydrogen, R₆ is 3-chloro-4-methoxybenzyl, R₉ is isobutyl, R₁₀ is hydrogen, and X and Y are oxygen. The structure of this compound, Cryptophycin **57**, is as follows:

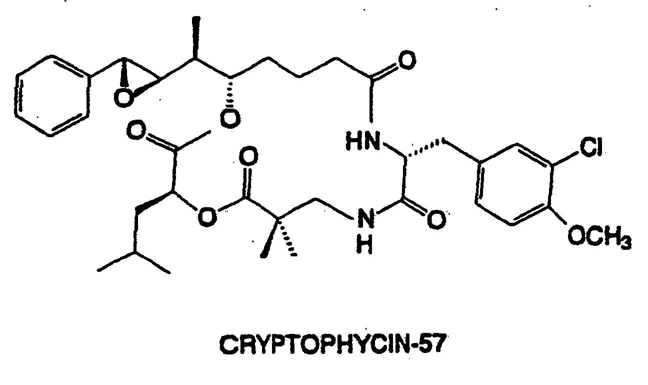

Another embodiment of a compound of the present invention is when Ar is phenyl, R₁ is *S*-chloro, R₂ is *R*-hydroxyl, R₃, R₇ and R₈ are methyl, R₄ and R₅ are hydrogen, R₆ is 3-chloro-4-methoxybenzyl, R₉ is isobutyl, R₁₀ is hydrogen, and X and Y are oxygen. The structure of this compound. Cryptophycin **58**, is as follows:

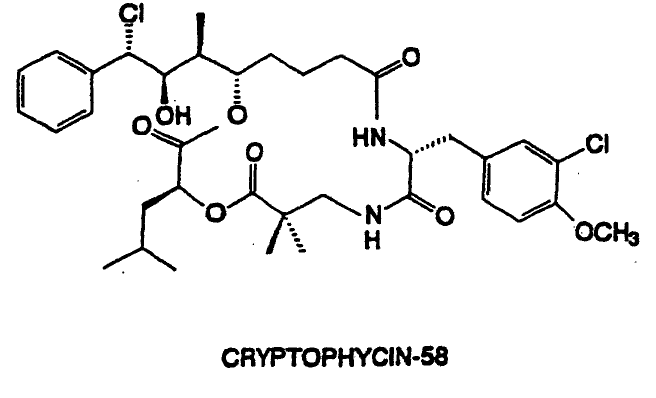

Another embodiment of a compound of the present invention is when Ar is phenyl, R₁ and R₂ are taken together to form an *R*,*R*-episulfide ring, R₃, R₇ and R₈ are methyl, R₄ and R₅ are taken together to form a double bond between C₁₃ and C₁₄, R₆ is 3-chloro-4-methoxybenzyl, R₉ is isobutyl, R₁₀ is hydrogen, and X and Y are oxygen. The structure of this compound, Cryptophycin **61**, is as follows:

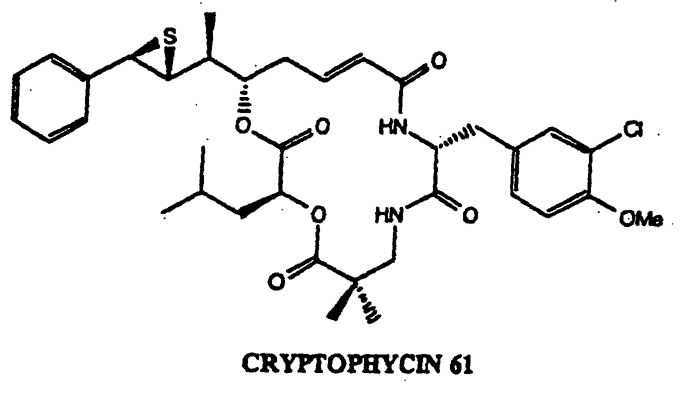

Another embodiment of a compound of the present invention is when Ar is phenyl, R₁ and R₂ are taken together to form an *R,R*-aziridine ring, R₃, R₇ and R₈ are methyl, R₄ and R₅ are taken together to form a double bond between C₁₃ and C₁₄, R₆ is 3-chloro-4-methoxybenzyl, R₉ is isobutyl, R₁₀ is hydrogen, and X and Y are oxygen. The structure of this compound, Cryptophycin **97**, is as follows:

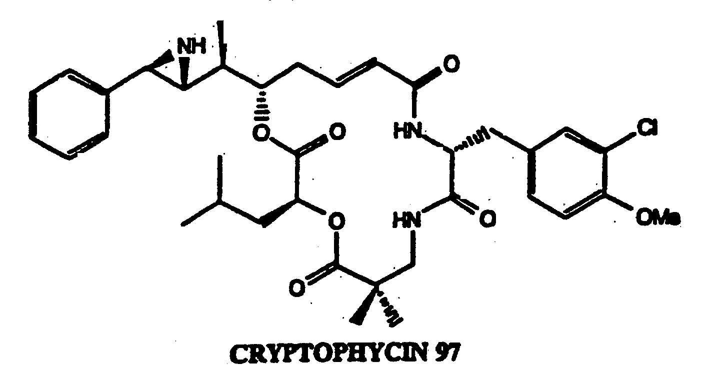

The present invention provides methods of producing the above cryptophycin compounds, as well as all previously known cryptophycins, through total synthesis.

The invention further provides that novel cryptophycin metabolites, as well as previously disclosed cryptophycin metabolites, may be synthesized using the methods provided in this invention.

The present invention provides a method for producing a cryptophycin of the structure. 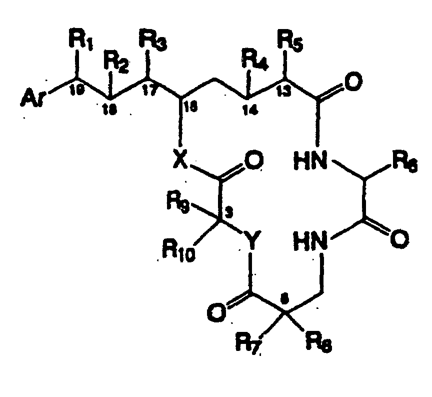 wherein
Ar is methyl or phenyl or any simple unsubstituted or substituted aromatic or heteroaromatic group;
R₁ is a halogen, SH, amino, monoalkylamino, dialkylamino, trialkylammonium, alkylthio, dialkylsulfonium, sulfate, or phosphate;
R₂ is OH or SH; or
R₁ and R₂ may be taken together to form an epoxide ring, an aziridine ring, an episulfide ring, a sulfate ring or a monoalkylphosphate ring; or
R₁ and R₂ may be taken together to form a double bond between C₁₈ and C₁₉;
R₃, R₇ and R₈ are each alkyl groups of one to five carbon atoms including linear and non linear hydrocarbon chains;
R₄ and R₅ are H; or
R₄ and R₅ may be taken together to form a double bond between C₁₃ and C₁₄;
R₆ is a benzyl, hydroxybenzyl, alkoxybenzyl, halohydroxybenzyl, dihalohydroxybenzyl, haloalkoxybenzyl, or dihaloalkoxybenzyl group;
R₉ and R₁₀ are each independently H or an alkyl group of one to five carbon atoms including linear and non linear hydrocarbon chains; and
X and Y are each independently O, NH or alkylamino comprising selecting an allylically substituted E alkene; rearranging the allylically substituted E alkene via a stereospecific Wittig rearrangement; converting this compound to a first δ-amino acid or δ-hydroxy acid; coupling the first acid to a second α-amino acid to form a first subunit; coupling a third β-amino acid to fourth α-hydroxy acid or α-amino acid to form a second subunit; and coupling the first subunit to the second subunit to form a said cryptophycin.

The present invention also provides a pharmaceutical composition useful for inhibiting the proliferation of a hyperproliferative mammalian cell comprising an effective amount of a cryptophycin with the following structure: 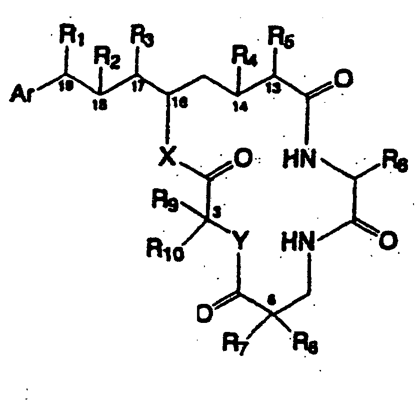 wherein
Ar is methyl of phenyl or any simple unsubsmuted or substituted aromatic or heteroaromatic group;
R₁ is a halogen, SH, amino, monoalkylamino, dialkylamino, trialkylammonium, alkylthio, dialkysulfonium, sulfate, or phosphate;
R₂ is OH or SH; or
R₁ and R₂ may be taken together to form an epoxide ring, an aziridine ring, an episulfide ring, a sulfate ring or a monoalkylphosphate ring; or
R₁ and R₂ may be taken together to form a double bond between C₁₈ and C₁₉;
R₃, R₇ and R₈ are each alkyl groups of one to five carbon atoms including linear and non linear hydrocarbon chains;
R₄ and R₅ are H; or
R₄ and R₅ may be taken together to form a double bond between C₁₃ and C₁₄;
R₆ is a benzyl, hydroxybenzyl, alkoxybenzyl, halohydroxybenzyl, dihalohydroxybenzyl, haloalkoxybenzyl, or dihaloalkoxybenzyl group;
R₉ and R₁₀ are each independently H or an alkyl group of one to five carbon atoms including linear and non linear hydrocarbon chains; and
X and Y are each independently O, NH or alkylamino; togther with a pharmaceutically acceptable carrier.

In a preferred embodiment of the present invention, the pharmaceutical composition further comprises at least one additional anti-neoplastic agent.

The present invention also provides for the use of a cryptophycin compound having the following structure: 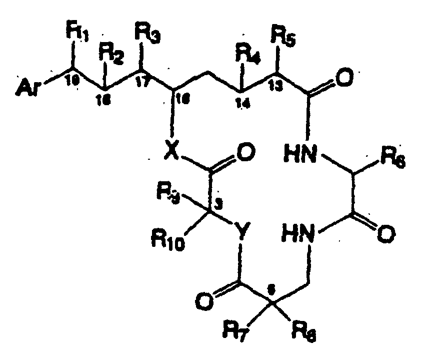 wherein
Ar is methyl or phenyl of any simple unsubstituted or substituted aromatic or heteroaromatic group;
R₁ is a halogen, SH, amino, monoalkylamino, dialkylamino, trialkylammonium, alkylthio, dialkylsulfonium, sulfate, or phosphate;
R₂ is OH or SH; or
R₁ and R₂ may be taken together to form an epoxide ring, an aziridine ring, an episulfide ring, a sulfate ring of a monoalkylphosphate ring; or
R₁ and R₂ may be taken together to form a double bond between C₁₈ and C₁₉;
R3, R7 and R8 are each alkyl groups of one to five carbon atoms including linear and non linear hydrocarbon chains;
R4 and R5 are H; or
R4 and R5 may be taken together to form a double bond between C13 and C14;
R6 is a benzyl, hydroxybenzyl, alkoxybenzyl, halohydroxybenzyl, dihalohydroxybenzyl, haloalkoxybenzyl, or dihaloalkoxybenzyl group;
R9 and R10 are each independently H; and
X and Y are each independently O, NH or alkylamino in the preparation of a medicament for inhibiting the proliferation of a mammalian cell.

In a preferred embodiment of the present invention the medicament further comprises at least one additional anti-neoplastic agent.

The present invention also provides the use of a cryptophycin compound having the following structure: 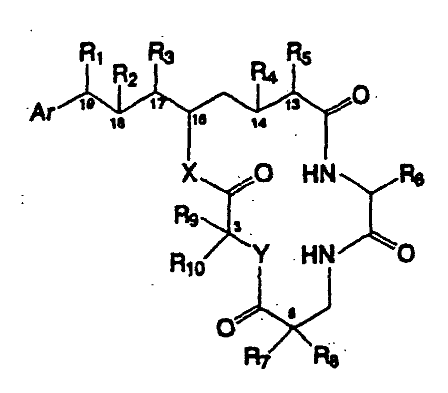 wherein
Ar is methyl or phenyl or any simple unsubstituted or substituted aromatic or heteroaromatic group;
R₁ is a halogen, SH, amino, monoalkylamino, dialkylamino, trialkylammonium, alkylthio, dialkylsulfonium, sulfate, or phosphate;
R₂ is OH or SH; or
R₁ and R₂ may be taken together to form an epoxide ring, an aziridine ring, an episulfide ring, a sulfate ring or a monoalkylphosphate ring; or
R₁ and R₂ may be taken together to form a double bond between C₁₈ and C₁₉;
R₃, R₇ and R₈ are each alkyl groups of one to five carbon atoms including linear and non linear hydrocarbon chains;
R₄ and R₅ are H; or
R₁ and R₅ may be taken together to form a double bond between C₁₃ and C₁₄;
R6 is a benzyl, hydroxybenzyl, alkoxybenzyl, halohydroxybenzyl, dihalohydroxybenzyl, haloalkoxybenzyl, or dihaloalkoxybenzyl group;
R₉, and R₁₀ are each independently H or an alkyl group of one to five carbon atoms including linear and non linear hydrocarbon chains; and
X and Y are each independently O, NH or alkylamino in the preparation of a medicament for inhibiting the proliferation of a hyperproliferative mammalian cell having a multiple drug resistant phenotype.

In a preferred embodiment of the present invention, the medicament further comprises at least one additional anti-neoplastic agent. In a further preferred embodiment of the present invention, the mammalian cell is human.

In a preferred embodiment of the present invention, the pathological condition is characterized by the formation of neoplasms. In a further preferred embodiment of the present invention, the neoplasms are selected from the group consisting of mammary, small-cell lung, non-small-cell lung, colorectal, leukemia, melanoma, pancreatic adenocarcinoma, central nervous system (CNS), ovarian, prostate, sarcoma of soft tissue or bone, head and neck, gastric which includes pancreatic and esophageal, stomach, myeloma, bladder, renal, neuroendocrine which includes thyroid and non-Hodgkin's disease and Hodgkin's disease neoplasms.

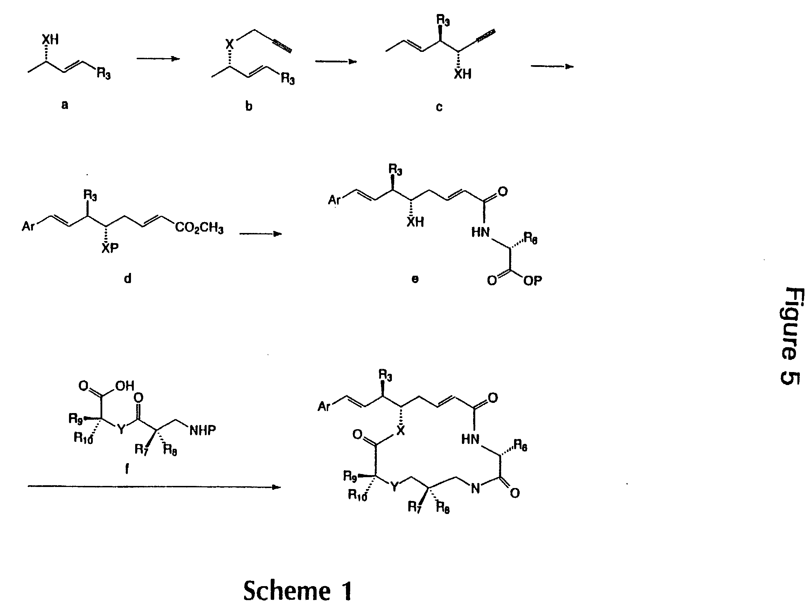

The method for preparing the cryptophycin compounds is summarized in Scheme 1 as depicted in Figure 5. The starting material is a 3*E*-alkene (a) substituted at C-2 with a XH group in the *S*-configuration where X is oxygen or NH. L-Alanine and L-lactic acid serve as inexpensive sources of the starting material **a**. The key step in the synthesis is a stereoselective [2,3] Wittig rearrangement (D.J-S. Tsai *et al*., *J*. *Org*. *Chem*. 1984, **49**:1842-1843; K. Mikami *et al*., *Tetrahedron* 1984, **25**:2303-2308; N. Sayo *et al*., *Chem Lett*. 1984, 259-262) of the propargyl ether of **a (b)** to the (3*R*,4*R*)-3-(XH-substituted)-4-alkylhept-5(*E*)-en-1-yne **(c)** where X is an oxygen or a protected nitrogen (e.g. *t*-butyldimethylsilylamino). Compound **c** can then be converted to the δ-hydroxy or amino acid unit A precursor of the cryptophycin, methyl (5*S*,6*R*)-5-(XP-substituted)-6-alkyl-8-aryl-octa-2*E*,7*E*-dienoate **(d)** where **P** is a suitable protecting group, using methods known to those of ordinary skill in the art.

One strategy for synthesizing a cryptophycin, which is composed of a δ-hydroxy or amino acid unit A, an α-amino acid unit B, a β-amino acid unit C, and an α-hydroxy or amino acid unit D, is to assemble the macrocycle from two precursors representing moieties of the cryptophycin molecule, for example, an A-B precursor **(e)** containing the δ-hydroxy or amino acid unit A and the α-amino acid unit B and a C-D precursor **(f)** containing the β-amino acid unit C and the α-hydroxy or amino acid unit D.

In the method described herein, a cryptophycin is assembled from A-B and C-D precursors in two steps by (1) connecting the termini of units A and D in the A-B and C-D precursors to form an acyclic C-D-A-B intermediate and (2) connecting the termini of units B and C to form the cyclic product.

In the synthesis of Cryptophycin **51** described in the Experimental Section, an ester linkage is formed between the δ-hydroxy group of unit A in the A-B moiety and the carboicylic acid group of unit D in the C-D fragment to form an acyclic C-D-A-B intermediate and then an amide linkage is formed between the carboxylic acid group of unit B in the A-B moiety and the β-amino group of unit C in the C-D moiety. Compound **K** is the A-B moiety precursor. Compound **P** is the C-D moiety precursor, and Compound **R** is the acyclic C-D-A-B precursor of Cryptophycin **51.** Compounds **K** and **P** have protecting groups on the carboxylic acid group of unit B and the β-amino group of unit C to limit coupling to ester formation between units A and D in step 1. These protecting groups are removed from the C-D-A-B intermediate so that amide formation can occur between units B and C in step 2.

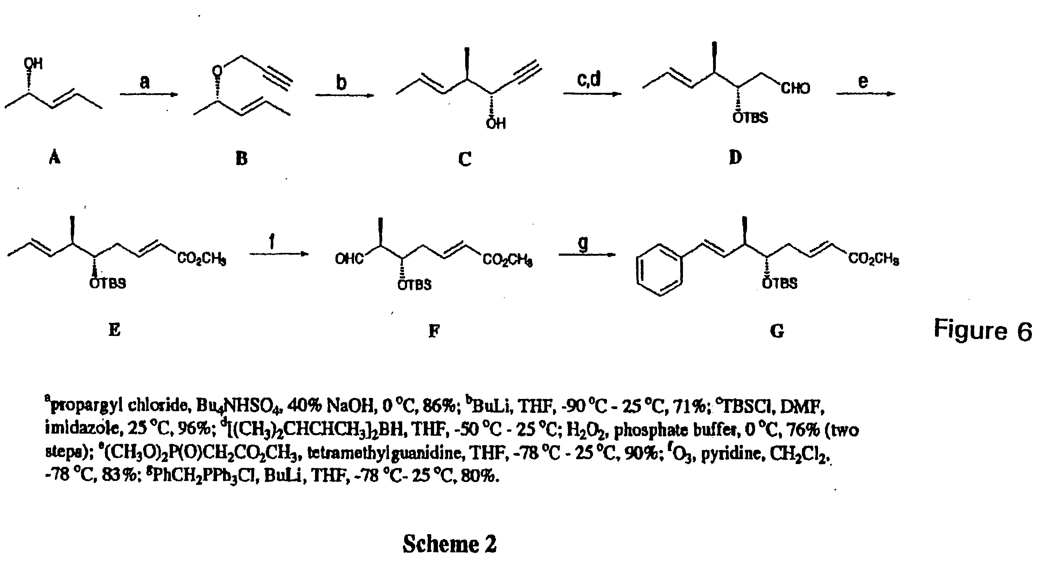

The synthesis of methyl (5*S*,6*R*)-5-*t*-butyldimethylsilyloxy-6-methyl-8-phenyl-octa-2*E*,7 *E*-dienoate (G), the unit A precursor of the Cryptophycin **51**, is summarized in Scheme 2 (Figure 6). (*S*)-*trans*-3-penten-2-ol (A), the starting material, was prepared by enzymatic resolution of the racemic compound. Reaction of **A** with propargyl chloride and base under phase-transfer condition formed propargyl ether **B** in 86% yield. Treatment of **B** with butyl lithium at -90°C led to alcohol C in 71% yield. The desired 3*R*,4*R anti* compound **C** was the only product formed in the Wittig rearrangement. After protection of the hydroxyl group of **C** as the *tert*-butyldimethylsilyl ether (or *tert*-butyldimethylsilyl ether), hydroboration of the triple bond (H.C. Brown, Organic Synthesis Via Boranes, Wiley, 1975) led to an aldehyde **D** in 73% yield from **C**. Next **D** was converted into the *trans* α, β-unsaturated ester **E** by a Horner-Emmons reaction in 90% yield. Selective ozonolysis of the C6-C7 double bond in **D** gave aldehyde **F** in 83% yield. Finally a Wittig reaction of **F** with benzyltriphenylphosphonium chloride in the presence of butyllithium produced **G** in 80% yield. The yield of **G** from **A** was 26%.

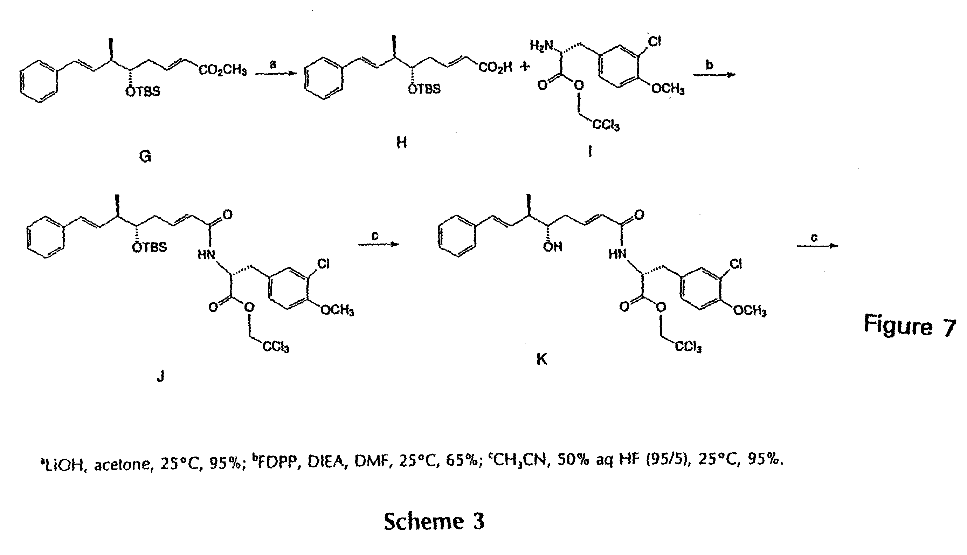

The coupling of the unit **A** precursor **G** with the D-3-(3-chloro-4-methoxyphenyl) alanine unit **B** to produce the A-B precursor (**K**) is summarized in Scheme 3 (Figure 7). Hydrolysis of the methyl ester group in **G** with lithium hydroxide in acetone produced carboxylic acid **H** in 95% yield. Coupling of **H** with trichloroethyl ester **I** to produce **J** could be accomplished in 65% yield by treating a solution of **H** in N,N-dimethylformamide (DMF) with a small excess of pentafluorophenyldiphenylphosphinate (FDPP), an equimolar quantity of the trifluoroacetate salt of **I**, followed by 3 equiv of diisopropylethylamine (DIEA) at 25°C (S. Chen *et al., Tetrahedron Lett*. 1991, **32**:6711-6714). Fluorodesilylation of **J** then led to **K** in 95% yield.

Protected amino acid **I** was prepared from D-tyrosine in five steps. First, D-tyrosine was chlorinated with sulfuryl chloride in glacial acetic acid (R. Zeynek, *Hoppe-Seyler's Z*. *f*. *Physiol*. *Chemie* 1926, **144**:247-254). Next *N*-(*tert*-butoxycarbonyl)-3-(3-chloro-4-hydroxyphenyl)-D-alanine was obtained in 94% yield by treating a suspension of the amino acid in 50% aqueous dioxaue with di-*tert*-butyldicarbonate in the presence of triethylamine. The resulting product was dimethylated with dimethyl sulfate in the presence of potassium carbonate in refluxing acetone in 84% yield. The methyl ester was then saponified with sodium hydroxide in aqueous dioxane to yield *N*-(*tert*-butoxycarbonyl)-3-(3-chloro-4-methoxyphenyl)-D-alanine in 86% yield. Exposure of the BOC-protected amino acid to trichloroethanol, pyridine and DCC in dichloromethane led to trichloroethyl ester **I** in 65 % yield. Treatment of this material with trifluoroacetic acid led to a quantitative yield of the trifluoroacetate salt of **I**.

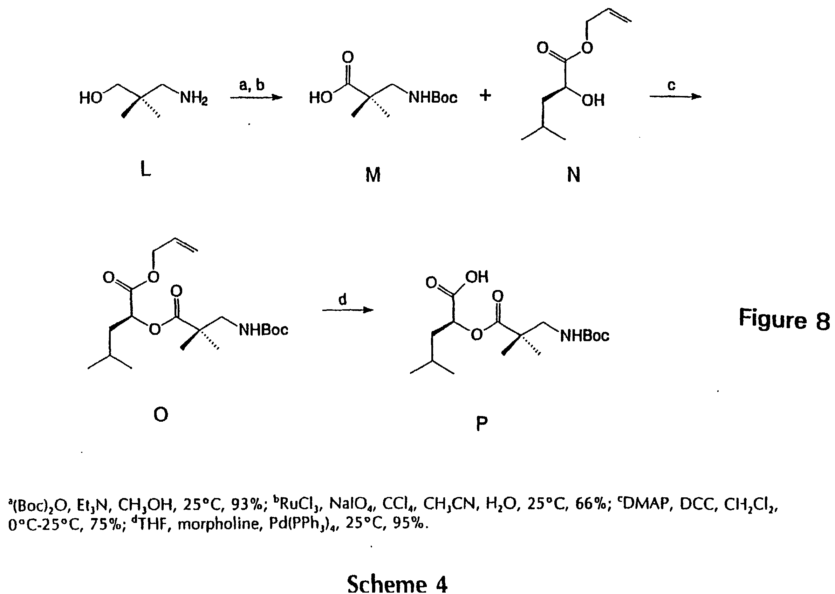

The synthesis of (2*S*)-2-[3'(*tert*-butoxycarbonyl)amino-2',2'-dimethylpropanoyloxy]-4-methylpentanoic acid (**P**), the C-D precursor, is summarized in Scheme 4 (Figure 8). The starting point for the unit C portion of **P** was the aminoalcohol **L**. Protection of the amino group in **L** by treatment with di-*tert*-butyldicarbonate in the presence of triethylamine (93% yield), followed by oxidation of the primary alcohol with ruthenium tetroxide (P.H.J. Carlsen *et al., J. Org*. *Chem*. 1981, **46**:3936-3938) gave carboxylic acid **M** (66% yield). L-Leucic acid was converted to allyl ester **N** in 93% yield under phase-transfer conditions, by exposing it to a mixture of allyl bromide in dichloromethane and aqueous sodium bicarbonate containing tetra-*n*-butylammonium chloride (S. Friedrich-Bochnitschek *et al., J. Org. Chem*. 1989, **54**:751-756). The coupling reaction of **M** with **N** was carried out with 4-dimethylaminopyridine (DMAP) and dicyclohexylcarbodiimide (DCC) in dichloromethane to produce **O** in 75% yield. Cleavage of the allyl ester in **O** was carried out in THF containing morpholine and catalytic tetrakis(triphenylphosphine)-palladium to give **P** in 95% yield (P.D. Jeffrey *et al.* , *J. Org*. *Chem*. 1982, **47**:587-590).

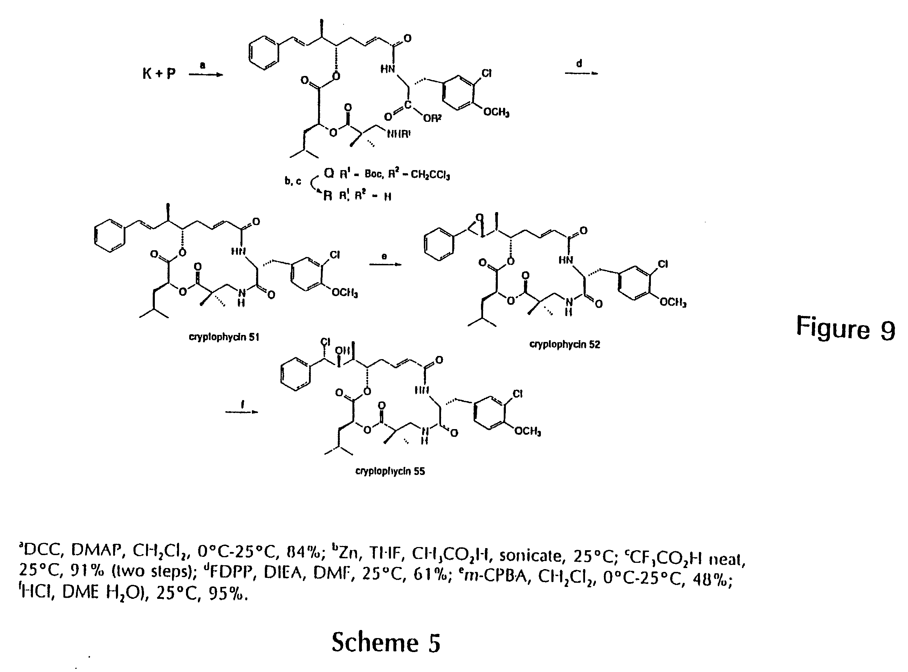

Coupling of the A-B precursor (**K**) and the C-D precursor (**P**) was accomplished as shown in Scheme 5 (Figure 9). Treatment of **K** and **P** with DCC/DMAP in dichloromethane led to the fully protected C-D-A-B intermediate (**Q**) in 84% yield. Reductive cleavage of the trichloroethyl ester group in **Q** was achieved using activated zinc dust in acetic acid. The BOC-protecting group was then removed by trifluoroacetic acid to give **R** as the trifluoroacetate salt in 91% overall yield from **Q**.
Macrolactamization of **R** with FDPP led to Cryptophycin **51** in 61 % yield (J. Dudash, Jr. *et al., Synth*. *Commun*. 1993, **23**:349-356). The overall yield from *S*-*trans*-3-penten-2-ol (**A**) was 7%.

Cryptophycin **51** served as the precursor of Cryptophycin **52,** the *R*,*R*-epoxide, and Cryptophycin **53,** the *S,S*-epoxide. In turn, Cryptophycin **52** served as the precursor of Cryptophycin **55,** the 18*R*,19*S*-chlorohydrin, and Cryptophycin **57,** the 13,14-dihydro analog. Cryptophycin **57** served as the precursor of Cryptophycin **58**. Cryptophycin **53** served as the precursor of Cryptophycin **61** using a method described by T.H. Chan and J.R. Finkenbine, *J*. *Am*. *Chem*. *Soc*. **1972, 94**:2880-2882 and Cryptophycin **97** using a method described by Y. Ittah *et al*., *J*. *Org*. *Chem*. **1978**, **43**:4271-4273.

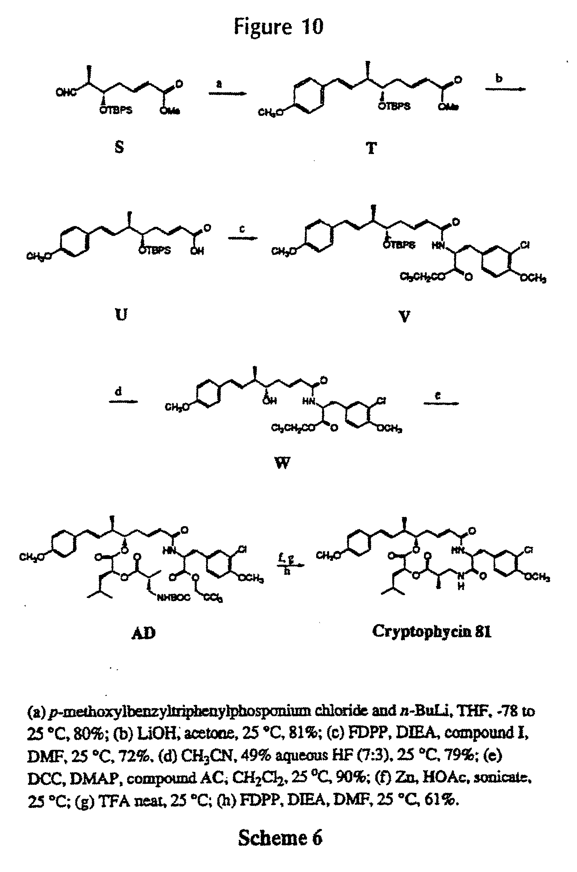

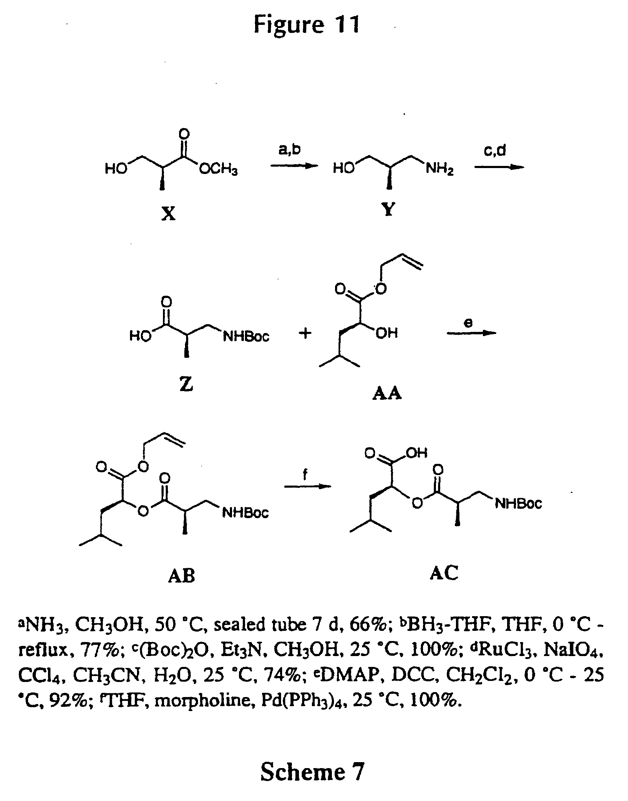

For the synthesis of cryptophycins that have Ar groups that are different from phenyl, unit A precursors of general structure **d** (Scheme 1, R₃ = Me) can be prepared by a Wittig reaction of aldehyde **F** (Scheme 2; TBS protecting group) or **S** (Scheme 6; TBPS protecting group) with the appropriate aryltriphenylphosphonium chloride in the presence of butyllithium. Cryptophycin **81** was prepared from precursor **d** (Ar = *p*-methoxyphenyl, R₃ = Me) as shown in Schemes 6 and 7 (Figures 10 and 11).

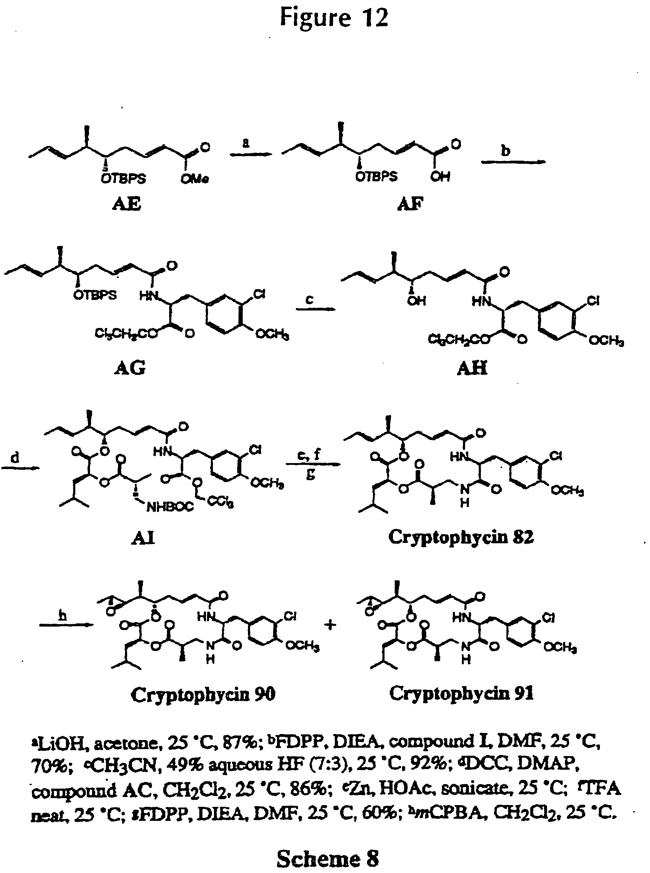

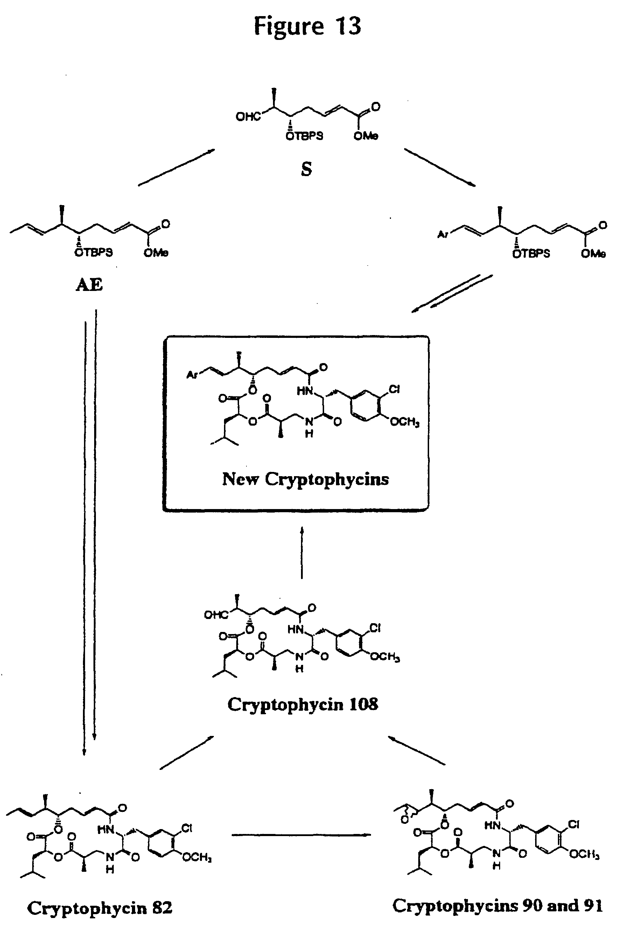

The Ar group can also be introduced into the new cryptophycin at a later step in the synthesis. First precursor **d** (Ar = R₃ = Me) was converted into Cryptophycin **82** by coupling the appropriate A-B (**e**) and C-D (**f**) precursors as shown in Scheme 8 (Figure 12). Selective ozonoiysis of Cryptophycin **82** or periodic acid oxidation of the corresponding epoxides Cryptophycins **90** and **91** led to an aldehyde, Cryptophycin **108.** A Wittig reaction of Cryptophycin **108** with the appropriate aryltriphenylphosphonium chloride in the presence of butyllithium gave the new cryptophycin (Scheme 9; Figure 13). Using this procedure, Cryptophycin **110** (Ar = *p*-fluorophenyl), Cryptophycin **111** (Ar = *p*-tolyl), Cryptophycin **112** (Ar = 2-thienyl) and Cryptophycin **124** (Ar = *p*-chlorophenyl) were prepared. Cryptophycin **110** served as the precursor of the epoxides Cryptophycins **115** and **116**. Cryptophycin **111** served as the precursor of the epoxides Cryptophycins **117** and **118** and the chlorohydrins Cryptophycins **128**, **130** and **131.** Cryptophycin **112** served as the precursor of the epoxides Cryptophycins **119** and **120**. Cryptophycin **124** served as the prccurcur of the epoxides Cryptophycins **125** and **126** and the chlorohydrins Cryptophycins **132, 133** and **134**.

Another strategy for synthesizing a cryptophycin is to assemble the macrocycle from three precursors, for example, an A-B precursor (**e**) containing the δ-hydroxy or amino acid unit A, a precursor containing the δ-hydroxy or amino acid unit D, and a precursor containing the β-amino acid unit C. In the method described herein, a cryptophycin is assembled from A-B, C and D precursors in three steps by (1) connecting the termini of units A and D in the A-B and D precursors to form an acyclic D-A-B intermediate, (2) connecting the termini of units D and C in the D-A-B and C precursors to form an acyctic C-D-A-B intermediate, and (3) connecting the termini of units B and C to form the cyclic product.

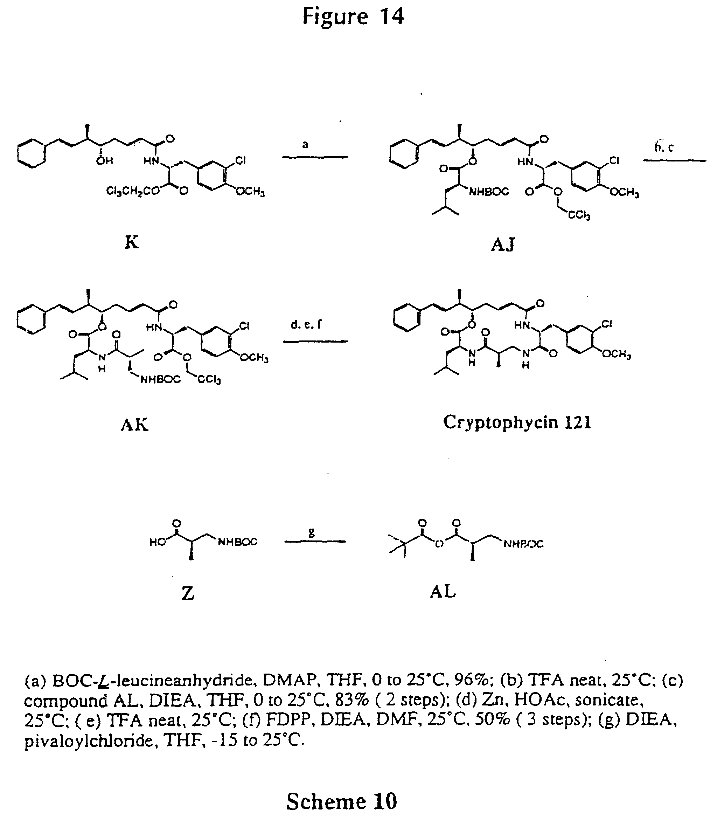

In the synthesis of Cryptophycin **121** described in the Experimental Section, an ester linkage is formed between the δ-hydroxy group of unit A in the A-B moiety and the carboxylic acid group of unit D in the D fragment to form an acyclic D-A-B intermediate. An amide linkage is then formed between the carboxylic acid group of unit C and the α-amino group of unit D in the D-A-B fragment. Finally an amide linkage is formed between the carboxylic acid group of unit B in the A-B moiety and the β-amino group of unit C in the C-D moiety. Compound **K** is the A-B moiety precursor, BOC-L-leucineanhydride is the D unit precursor, and Compound **AL** is the unit C precursor. Compound **AK** is the acyclic C-D-A-B precursor of Cryptophycin **121** (Scheme 10; Figure 14). Compounds **K** and BOC-L-leucineanhydride have protecting groups on the carboxylic acid group of unit B and the α-amino group of unit D to limit coupling to ester formation between units A and D in step 1. The protecting group is removed from the δ-amino group of unit D in the D-A-B intermediate so that amide formation can occur between the amino group in unit B and the carboxylic acid C in step 2. These protecting groups are removed from the C-D-A-B intermediate so that amide formation can occur between units B and C in step 2.

Novel cryptophycin compounds of the present invention have epoxide rings that are opened by nucleophiles at different rates than the epoxide ring in Cryptophycin **1**, or have chlorohydrin functionalities that form epoxide rings at different rates than the chlorohydrin functionality of Cryptophycin **8** is transformed into the epoxide ring of Cryptophycin **1**. The epoxide ring of Cryptophycin **1** or the chlorohydrin functionality (a masked epoxide ring) of Cryptophycin **8** is essential for optimum *in vivo* activity. If the epoxide oxygen is eliminated (such as found in Cryptophycin **3**) or the epoxide ring hydrolyzed to a diol (such as found in Cryptophycin **15**), antitumor activity is greatly diminished. Cryptophycin **1** shows appreciable toxicity in animals compared with Cryptophycin **8**. This is reflected in the T/C (mostly >0%) and gross log kill values (mostly <2.0) for Cryptophycin 1 compared with those for Cryptophycin **8** (mostly T/C values of 0% and gross log kill values of >2.8). Cryptophycin **25**, the corresponding bromohydrin analog, shows T/C and gross log kill values that are comparable with those for Cryptophycin **1**. This striking difference in *in vivo* activity suggests that the bromohydrin Cryptophycin **25** is converted more rapidly into Cryptophycin **1** *in vivo* than Cryptophycin **8**. This further suggests that the less toxic Cryptophycin **8** might have more time to accumulate at the tumor site before being transformed into the active compound Cryptophycin **1**. The epoxide group of Cryptophycin **1** probably binds covalently to its target receptor in the tumor cell. The novel cryptophycins in the present invention could potentially possess better *in vivo* activity than Cryptophycin **1** and Cryptophycin **8** by exhibiting more favorable rates of epoxide formation *in vivo* from the corresponding chlorohydrin pro-drugs and covalent binding to the target receptor in the tumor cell.

The compounds of the present invention are more stable towards hydrolysis and solvolysis than Cryptophycins **1** and **21**. The ester bond connecting units C and D in Cryptophycin **1** is relatively sensitive to mild base hydrolysis, cleaving at pH 11 to a hydroxy acid with a half-life of 0.83 hour. The C-D ester bond in Cryptophycin **21**, which lacks the methyl group on C-2 of unit C, opens at a faster rate with a half-life of 0.25 hour. The C-D ester bond is also sensitive to solvolysis. When methanol is used in the isolation scheme, considerable methanolysis of Cryptophycins **1** and **21** occurs. Cryptophycin **21** is much more susceptible to methanolysis than Cryptophycin **1**. Cryptophycin **1** shows antitumor activity whereas Cryptophycin **21** is inactive, probably because the C-D ester bond of Cryptophycin **21** is hydrolyzed faster than the C-D ester bond of Cryptophycin **1** *in vivo*. Hydrolysis of the C-D ester bond may also explain in part the diminished *in vivo* activity of Cryptophycin **1** by intraperitoneal and subcutaneous routes of drug administration. The C-D ester bond of cryptophycins possessing two methyl groups on C-2 of unit C, such as the one found in Cryptophycin **52**, is stable at pH 11.

The compounds of the present invention and the previously disclosed cryptophycin compounds can be therapeutically employed as anti-neoplastic agents and thereby used in methods to treat neoplastic diseases. As used herein, "neoplastic" pertains to a neoplasm, which is an abnormal growth, such growth occurring because of a proliferation of cells not subject to the usual limitations of growth. As used herein, "anti-neoplastic agent" is any compound, composition, admixture, co-mixture or blend which inhibits, eliminates, retards or reverses the neoplastic phenotype of a cell.

Chemotherapy, surgery, radiation therapy, therapy with biologic response modifiers, and immunotherapy are currently used in the treatment of cancer. Each mode of therapy has specific indications which are known to those of ordinary skill in the art, and one or all may be employed in an attempt to achieve total destruction of neoplastic cells. Chemotherapy utilizing one or more cryptophycins is provided by the present invention. Moreover, combination chemotherapy, chemotherapy utilizing cryptophycins in combination with other neoplastic agents, is also provided by the subject invention as combination therapy is generally more effective than the use of single anti-neoplastic agents. Thus, a further aspect of the present invention provides compositions containing a therapeutically effective amount of at least one new cryptophycin compound of the present invention, including nontoxic addition salts thereof, which serve to provide the above-recited therapeutic benefits. Such compositions can also be provided together with physiologically tolerable liquid, gel or solid carriers, diluents, adjuvants and excipients. Such carriers, diluents, adjuvants and excipients may be found in the United States *Pharmacopeia* Vol. XXII and National Formulary Vol XVII, U.S. *Pharmacopeia* Convention, Inc., Rockville, MD (1989). Additional modes of treatment are provided in AHFS Drug Information, 1993 ed. by the American Hospital Formulary Service, pp. 522-660.

The present invention further provides that the pharmaceutical composition used to treat neoplastic disease contains at least one cryptophycin compound and at least one additional and-neoplastic agent. Anti-neoplastic compounds which may be utilized in combination with cryptophycin include those provided in The Merck Index, 11th ed. Merck & Co., Inc. (1989) pp. Ther 16-17. In a further embodiment of the invention, anti-neoplastic agents may be antimetabolites which may include, but are not limited to, methotrexate, 5-fluorouracil, 6-mereaptopurine, cytosine arabinoside, hydroxyurea, and 2-chlorodeoxyadenosine. In another embodiment of the present invention, the anti-neoplastic agents contemplated are alkylating agents which may include, but are not limited to, cyclophosphamide, melphalan, busulfan, paraplatin, chlorambucil, and nitrogen mustard. In a further embodiment of the subject invention, the anti-neoplastic agents are plant alkaloids which may include, but are not limited to, vincristine, vinblastine, taxol, and etoposide. In a further embodiment of the present invention, the anti-neoplastic agents contemplated are antibiotics which may include, but are not limited to, doxorubicin (adriamycin), daunorubicin, mitomycin c, and bleomycin. In a further embodiment of the subject invention, the anti-neoptastic agents contemplated are hormones which may include, but are not limited to, calusterone, diomostavolone, propionate, epitiostanol, mepitiostane, testolactone, tamoxifen, polyestiadiol phosphate, megesterol acetate, flutamide, nilutamide, and trilotane. In a further embodiment of the subject invention, the anti-neoplastic agents contemplated include enzymes which may include, but are not limited to, L-Asparaginase or aminoacridine derivatives which may include, but are not limited to, amsacrine. Additional anti-neoplastic agents include those provided in Skeel, Roland T., "Antineoplastic Drugs and Biologic Response Modifier: Classification, Use and Toxicity of Clinically Useful Agents," Handbook of Cancer Chemotherapy (3rd ed.), Little Brown & Co. (1991).

The present cryptophycin compounds and compositions can be administered to mammals for veterinary use, such as for domestic animals, and clinical use in humans in a manner similar to other therapeutic agents. In general, the dosage required for therapeutic efficacy will vary according to the type of use and mode of administration, as well as the particularized requirements of individual hosts. Ordinarily, dosages will range from about 0.001 to 100mg/kg, more usually 0.01 to 10mg/kg, of the host body weight. Alternatively, dosages within these ranges can be administered by constant infusion over an extended period of time, usually exceeding 24 hours, until the desired therapeutic benefits have been obtained. Indeed, drug dosage, as well as route of administration, must be selected on the basis of relative effectiveness, relative toxicity, growth characteristics of tumor and effect of cryptophycins on cell cycle, drug pharmacokinetics, age, sex, physical condition of the patient, and prior treatment.

The cryptophycin compounds, with or without additional anti-neoplastic agents, may be formulated into therapeutic compositions as natural or salt forms.
Pharmaceutically acceptable non-toxic salts include the base addition salts (formed with free carboxyl or other anionic groups) which may be derived from inorganic bases such as, for example, sodium, potassium, ammonium, calcium, or ferric hydroxides, and such organic bases as isopropylamine, trimethylamine, 2-ethylamino ethanol, histidine, procaine, and the like. Such salts may also be formed as acid addition salts with any free cationic groups and will generally be formed with inorganic acids such as, for example, hydrochloric or phosphoric acids, or organic acids such as acetic, oxalic, tartaric, mandelic, and the like. Additional excipients which the further invention provides are those available to one of ordinary skill in the art, for example, that found in the United States *Pharmacopeia* Vol. XXII and National Formulary Vol XVII, U.S. *Pharmacopeia* Convention, Inc., Rockville, MD (1989).

The suitability of particular carriers for inclusion in a given therapeutic composition depends on the preferred route of administration. For example, anti-neoplastic compositions may be formulated for oral administration. Such compositions are typically prepared either as liquid solution or suspensions, or in solid forms. Oral formulations usually include such normally employed additives such as binders, fillers, carriers, preservatives, stabilizing agents, emulsifiers, buffers and excipients as, for example, pharmaceutical grades of mannitol, lactose, starch, magnesium stearate, sodium saccharin, cellulose, magnesium carbonate, and the like. These compositions take the form of solutions, suspensions, tablets, pills, capsules, sustained release formulations, or powders, and typically contain 1%-95% of active ingredient, preferably 2%-70%.

Compositions of the present invention may also be prepared as injectable, either as liquid solutions, suspensions, or emulsions; solid forms suitable for solution in, or suspension in, liquid prior to infection may be prepared. Such injectables may be administered subcutaneously, intravenously, intraperitoneally, intramuscularly, intrathecally, or intrapleurally. The active ingredient or ingredients are often mixed with diluents or excipients which are physiologically tolerable and compatible with the active ingredient(s). Suitable diluents and excipients are, for example, water, saline, dextrose, glycerol, or the like, and combinations thereof. In addition, if desired, the compositions may contain minor amounts of auxiliary substances such as wetting or emulsifying agents, stabilizing or pH buffering agents.

The invention further provides cryptophycin compounds encompassed by the genus structure for the preparation of a medicament to inhibit the proliferation of mammalian cells by contacting these cells with a cryptophycin compound in an amount sufficient to inhibit the proliferation of the mammalian cell. A preferred embodiment is a medicament to inhibit the proliferation of hyperproliferative mammalian cells. For purposes of this invention, "hyperproliferative mammalian cells" are mammalian cells which are not subject to the characteristic limitations of growth, e.g., programmed cell death (apoptosis). A further preferred embodiment is when the mammalian cell is human. The invention further provides contacting the mammalian cell with at least one cryptophycin compound and at least one additional anti-neoplastic agent. The types of anti-neoplastic agents contemplated are the same as those disclosed hereinabove.

The invention further provides cryptophycin compounds encompassed by the genus structure for the preparation of a medicament to inhibit the proliferation of hyperproliferative cells with drug-resistant phenotypes, including those with multiple drug-resistant phenotypes, by contacting said cell with a cryptophycin compound in an amount sufficient to inhibit the proliferation of a hyperproliferative mammalian cell. A preferred embodiment is when the mammalian cell is human. The invention further provides contacting the mammalian cell with a cryptophycin compound and at least one additional anti-neoplastic agent. The types of anti-neoplastic agents contemplated are the same as those disclosed hereinabove.

The pharmaceutical composition of the invention can be used in a method for alleviating pathological conditions caused by hyperproliferating mammalian cells, for example, neoplasia, by administering to a subject an effective amount of a pharmaceutical composition provided hereinabove to inhibit the proliferation of the hyperproliferating cells. As used herein "pathological condition" refers to any pathology arising from the proliferation of mammalian cells that are not subject to the normal limitations of cell growth. Such proliferation of cells may be due to neoplasms, including, but not limited to the following neoplasms; mammary, small-cell lung, non-small-cell lung, colorectal, leukemia, melanoma, central nervous system (CNS), ovarian, prostate, sarcoma of soft tissue or bone, head and neck, gastric which includes pancreatic and esophageal, stomach, myeloma, bladder, renal, neuroendocrine which includes thyroid and lymphoma, non-Hodgkin's and Hodgkin's. In a further embodiment of the invention, the neoplastic cells are human. The pharmaceutical composition of the invention can also be used in methods of alleviating such pathological conditions utilizing cryptophycin in combination with other therapies, as well as other anti-neoplastic agents. Such therapies and their appropriateness for different neoplasia may be found in Cancer Principles and Practice of Oncology, 4th ed., Editors DeVita, V., Hellman, S., and Rosenberg., S., Lippincott Co. (1993).

In the present disclosure, cryptophycin compounds are shown to potently disrupt the microtubule structure in cultured cells. In addition, and in contrast with the *Vinca* alkaloids, cryptophycin compounds appear to be a poor substrate for the drug-efflux pump P-glycoprotein. Cryptophycin **1** is the major cytotoxin in the blue-green alga (cyanobacteria) *Nostoc* sp. strain designated GSV 224 and shows excellent activity against tumors implanted in mice. This cyclic didepsipeptide had previously been isolated from *Nostoc* sp. ATCC accession no. 53789 as an antifungal agent and its gross structure was previously determined. The relative and absolute stereochemistry of this potentially important drug has now been established using a combination of chemical and spectral techniques. Twenty-four additional cryptophycin compounds, Cryptophycins **2-7, 16-19, 21, 23, 24, 26, 28-31, 40, 43, 45, 49, 50** and **54** have also been isolated from GSV 224 and their total structures and cytotoxicities determined. Several derivatives and degradation products are described, both chemically and pharmacologically.

The following examples serve to illustrate certain preferred embodiments and aspects of the present invention and are not to be construed as limiting the scope thereof.

### Experimental Section

In the experimental disclosure which follows, all weights are given in grams (g), milligrams (mg), micrograms (µg), nanograms (ng), picograms (pg), moles (mol) or millimoles (mmol), all concentrations are given as percent by volume (%), molar (M), millimolar (mM), micromolar (µM), nanomolar (nM), picomolar (pM), or normal (N). all volumes are given in liters (L), milliliters (mL) or microliters (µL), and linear measurements in millimeters (mm), unless otherwise indicated.

The following examples demonstrate the isolation and synthesis of cryptophycin compounds as well as their use as therapeutic agents in accordance with the invention.

### Example 1 Analysis of Microtubule Depolymerizing Activity of Cryptophycin Materials

Vinblastine, cytochalasin B, tetramethylrhodamine isothiocyanate (TRITC)-phalloidin, sulforhodamine B (SRB) and antibodies against β-tubulin and vimentin were obtained from the Sigma Chemical Company. Basal Medium Eagle containing Earle's salts (BME) was from Gibco and Fetal Bovine Serum (FBS) was purchased from Hyclone Laboratories.

### Cell Lines

The Jurkat T cell leukemia line and A-10 rat aortic smooth muscle cells were obtained from the American Type Culture Collection and were cultured in BME containing 10% FBS and 50µg/mL gentamycin sulfate. Human ovarian carcinoma cells (SKOV3) and a sub-line which has been selected for resistance to vinblastine (SKVLB1) were a generous gift from Dr. Victor Ling of the Ontario Cancer Institute. Both cell lines were maintained in BME containing 10% FBS and 50µg/mL gentamycin sulfate. Vinblastine was added to a final concentration of 1µg/mL to SKVLB1 cells 24 hours after passage to maintain selection pressure for P-glycoprotein-overexpressing cells.

### Cell Proliferation and Cycle Arrest Assays

Cell proliferation assays were performed as described by Skehan *et al*.¹¹ For Jurkat cells, cultures were treated with the indicated drugs as described in Skehan¹¹ and total cell numbers were determined by counting the cells in a hemacytometer. The percentage of cells in mitosis was determined by staining with 0.4% Giemsa in PBS followed by three rapid washes with PBS. At least 1000 cells per treatment were scored for the presence of mitotic figures and the mitotic index was calculated as the ratio of cells with mitotic figures to the total number of cells counted.

### Immunofluorescence Assays

A-10 cells were grown to near-confluency on glass coverslips in BME/10% FBS. Compounds in PBS were added to the indicated final concentrations and cells were incubated for an additional 24 hours. For the staining of microtubules and intermediate filaments, the cells were fixed with cold methanol and incubated with PBS containing 10% calf serum to block nonspecific binding sites. Cells were then incubated at 37°C for 60 min with either monoclonal anti-β-tubulin or with monoclonal anti-vimentin at dilutions recommended by the manufacturer. Bound primary antibodies were subsequently visualized by a 45-minute incubation with fluorescein-conjugated rabbit antimouse IgG. The coverslips were mounted on microscope slides and the fluorescence patterns were examined and photographed using a Zeiss Photomicroscope ill equipped with epifluorescence optics for fluorescein. For staining of microfilaments, cells were fixed with 3% paraformaldehyde, permeabilized with 0.2% Triton X-100 and chemically reduced with sodium borohydride (1mg/mL). PBS containing 100nM TRITC-phalloidin was then added and the mixture was allowed to incubate for 45 min at 37° C. The cells were washed rapidly three times with PBS before the coverslips were mounted and immediately photographed as described above.

### Effects of cryptophycins and vinblastine on Jurkat cell proliferation and cell cycle

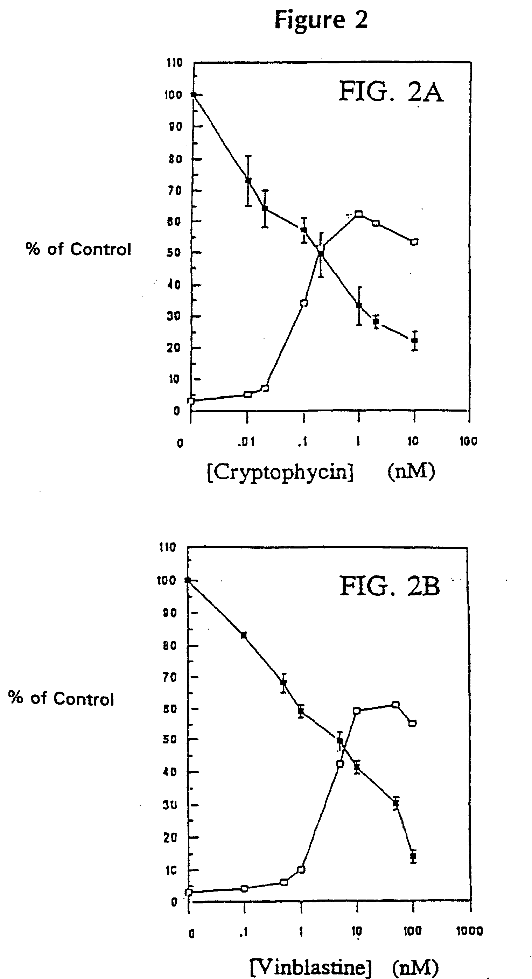

Dose-response curves for the effects of cryptophycin compounds and vinblastine on cell proliferation and the percentage of cells in mitosis are indicated in Figures 2A and 2B. respectively. Less than 3% of untreated cells displayed mitotic figures. Both the cryptophycin. compounds and vinblastine caused dose-dependent increases in the percentage of cells observed in mitosis. The increase in the mitotic index was closely correlated. with decreases in cell proliferation, i.e. the concentrations of both cryptophycin compounds and vinblastine that caused 50% of the cells to accumulate in mitosis was virtually the same as the concentration which inhibited cell proliferation by 50%. The IC₅₀s for the cryptophycin compounds and vinblastine for these effects were 0.2 and 8nM, respectively.

### Effects of cytochalasin B. vinblastine and cryptophycins on the cytoskeleton

Aortic smooth muscle (A-10) cells were grown on glass coverslips and treated with PBS, 2µM cytochalasin B, 100nM vinblastine or 10nM cryptophycin compounds. After 24 hours, microtubules and vimentin intermediate filaments were visualized by indirect immunofluorescence and microfilaments were stained using TRITC- phalloidin. The morphological effects of each drug were examined. Untreated cells displayed extensive microtubule networks complete with perinuclear microtubule organizing centers. Vimentin intermediate filaments were also evenly distributed throughout the cytoplasm, while bundles of microfilaments were concentrated along the major axis of the cell. Cytochalasin B caused complete depolymerization of microfilaments along with the accumulation of paracrystalline remnants. This compound did not affect the distribution of either mierotubules or intermediate filaments. Both vinblastine and the cryptophycin compound caused marked depletion of microtubules. Neither compound affected microfilawent organization; however, vimentin intermediate filaments collapsed, forming concentric rings around the nuclei of cells treated with either vinblastine or a cryptophycin compound.

### Effects of cryptophycins and vinblastine on taxol-stabilized microtubules

A-10 cells were treated for 3 hours with 0 or 10µM taxol before the addition of PBS, 100nM vinblastine or 10nM cryptophycin compound. After 24 hours, microtubule organization was examined by immunofluorescence as described above. Compared with those in control cells, microtubules in taxol-treated cells were extensively bundled, especially in the cell polar regions. As before, vinblastine caused, complete depolymerization of microtubules in non-pretreated cells. However, pretreatment with taxol prevented microtubule depolymerization in response to vinblastine. Similarly, taxol pretreatment completely stabilized microtubules against cryptophycin-induced depolymerization.

### Reversibility of microtubule depolymerization by vinblastine and cryptophycin

A-10 cells were treated with either 100nM vinblastine or 10nM cryptophycins for 24 hr, resulting in complete microtubule depolymerization. The cells were then washed and incubated in drug- free medium for periods of 1 hour or 24 hours. Microtubules repolymerized rapidly after the removal of vinblastine, showing significant levels of microtubules after 1 hour and complete morphological recovery by 24 hour. In contrast, microtubules did not reappear in cells treated with cryptophycin compounds at either 1 hour or 24 hours after removal of the compound.

### Reversibility of cryptophycins-, vinblastine- and taxol-inhibition of cell proliferation

SKOV3 cells were treated for 24 hours with previously determined IC₅₀ doses of vinblastine, cryptophycin compounds or taxol (i.e. values determined in experiments summarized in Table 5). During this time the cell density increased from 0.4 to 0.5 ± 0.05 absorbance units (Figure 3), indicating a 25% increase in cell cumber for all three treatments. Removal of the drugs resulted in rapid growth of the vinblastine-treated cells, such that their numbers were increased approximately 3-fold in 24 hours. In contrast, cells treated with cryptophycin compounds or taxol remained arrested, increasing only 0.2- to 0.4-fold in the 24 hours following removal of the drug. The proliferative capacity of cryptophycins or taxol-treated cells was subsequently restored since the cells then doubled in the next 24 hours.

### Effects of combinations of vinblastine and cryptophycins on cell proliferation

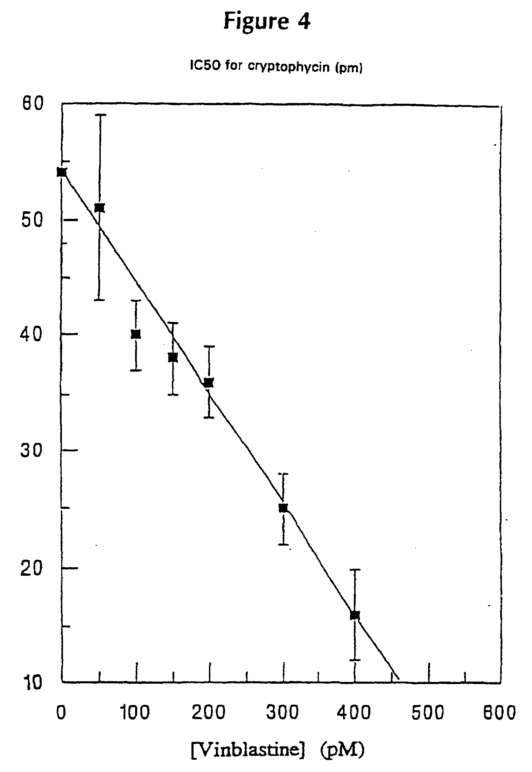

SKOV3 cells were treated with combinations of cryptophycins and vinblastine for 48 hours. The percentages of surviving cells were then determined and the IC₅₀s for each combination was calculated. The effects of these combinational treatments, as well as single drug treatments, are depicted as an isobologram (Figure 4). The IC₅₀s for combinations of cryptophycin compounds and vinblastine fell very close to the line of additivity, indicating that these two drugs induce only additive inhibitions of cell proliferation.

### Toxicity of cryptophycins, vinblastine and taxol toward SKOV3 and SKVLB1 cells

SKVLB1 cells are resistant to natural product anticancer drugs because of their over expression of P-glycoprotein¹². The abilities of taxol, vinblastine and cryptophycin compounds to inhibit the growth of SKOV3 and SKVLB1 cells are summarized in Table 5. Taxol caused dose-dependent inhibition of the proliferation of both cell lines with IC₅₀s for SKOV3 and SKVLB1 cells of 1 and 8000nM, respectively. Vinblastine also inhibited the growth of both cell lines, with IC₅₀s of 0.35 and 4200nM for SKOV3 and SKVLB1 cells, respectively. Cryptophycins demonstrated IC₅₀s of 7 and 600pM for SKOV3 and SKVLB1 cells, respectively. The resulting resistance factors for SKVLB1 cells to the compounds are calculated as the IC₅₀s for SKVLB1. IC₅₀s for SKOV3 cells are also indicated in Table 5.

Thus it is demonstrated that the present invention provides novel cryptophycin compounds, as well as previously-disclosed cryptophycin compounds, which are potent inhibitors of cell proliferation, acting by disruption of the microtubule network and inhibition of mitosis. The cryptophycin compounds disrupt microtubule organization and thus normal cellular functions, including those of mitosis.

Classic anti-microtubule agents, such as colchicine and *Vinca* alkaloids, arrest cell division at mitosis. It seemed appropriate to compare the effect of one of these agents on cell proliferation with the cryptophycin compounds. For this purpose, the *Vinca* alkaloid vinblastine was selected as representative of the classic anti-microtubule agents. Accordingly, the effect of cryptophycin compounds and vinblastine on the proliferation and cell cycle progression of the Jurkat T-cell leukemia cell line was compared. Both compounds caused parallel dose-dependent inhibitions of cell proliferation and accumulation of cells in mitosis.

Since antimitotic effects are commonly mediated by disruption of microtubules in the mitotic spindles, the effects of cryptophycin compounds on cytoskeletal structures were characterized by fluorescence microscopy. Immunofluorescence staining of cells treated with either a cryptophycin compound or vinblastine clearly demonstrated that both compounds caused the complete loss of microtubules. Similar studies with SKOV3 cells demonstrate that the anti-microtubule effects of cryptophycin compounds are not unique to the smooth muscle cell line. Neither drug affected the levels or distribution of microfilament bundles, as was readily induced by cytochalasin B, indicating that the loss of microtubules may not be due to a non-specific mechanism, e.g. activation of proteases or loss of energy charge. Both vinblastine and cryptophycin compounds also promote marked collapse of vimentin intermediate filaments, such that brightly staining rings were formed around the cell nucleus.

Removal of vinblastine from the culture medium resulted in rapid repolymerization of microtubules. In contrast, cells treated with cryptophycin compounds remained depleted of microtubules for at least 24 hours after the compound was removed from the cultures.

The present invention demonstrates that cryptophycin compounds circumvent P-glycoprotein-mediated multiple drug resistance. Transport by P-glycoprotein limits the ability of natural product anticancer drugs to inhibit the growth of mmor cells with acquired or *de novo* drug resisrance.¹³⁻¹⁵ *Vinca* alkaloids, while very useful in the initial course of chemotherapy, are extremely good substrates for transport by P-glycoprotein, and so are of very limited usefulness against P-glycoprotein-mediated MDR tumors. Therefore, identification of agents which overcome multiple drug resistance may, should lead to the development of useful and novel anticancer agents. The cryptophycin compounds of the present invention appear to be such agents since they are poor substrates for P- glycoprotein-mediated transport. This fact is reflected in the low cell resistance factor for cryptophycin compounds compared with vinblastine, taxol and other natural product drugs.

### Total Synthesis of Cryptophycins

The structures of the novel synthesized compounds, viz. Cryptophycins **51, 52, 53, 55, 56, 57, 58,** and **61** were confirmed in a straightforward manner using methodology that is well-known to those trained in the art. Mass spectral data were consistent with the molecular compositions. Proton and carbon NMR data were very similar to those of cryptophycin **1** and related naturally-occurring and semi-synthetic analogs.

The following examples demonstrate the total synthesis of cryptophycin compounds as well as their use as therapeutic agents in accordance with the invention.

### Example 2 Synthesis of Cryptophycin 51 S-trans-3-Penten-2-ol (A)

A mixture of racemic *trans*-3-penten-2-ol (933mg, 11mmol), trifluoroethyl laurate (4.14g, 15mmol), and porcine pancreatic lipase (PPL, 2.0g) in 25mL of anhydrous diethyl ether was stirred for 80 hours. The PPL was then filtered off and washed with ether three times. The ether filtrate was evaporated and the sticky oil was then subjected to short-path vacuum distillation. The *S-trans*-3-penten-2-ol (**A**) was condensed in a liquid nitrogen cooled trap (383mg). ¹H NMR (CDCl₃) δ 5.57 (4-H; dq, -15.3/6.0), 5.47 (3-H; ddd, -15.3/6.4/1.2), 4.19 (2-H; 1:4:6:4:1 pentuplet, 6.4), 2.24 (OH; bs), 1.63 (5-H₃; d, 6.0), 1.19 (1-H₃; d, 6.4). ¹³C NMR (CDCl₃) δ 135.5 (3), 125.5 (4), 68.7 (2), 23.3 (5), 17.5 (1).

### S-trans-2-(2-Propynloxy)-3-pentene (B)

To a vigorously stirred mixture of *S*-enantiomer **A** (628mg, 7.3mmol), tetrabutylammonium hydrogen sulfate (138mg, 0.41mmol), and 40% NaOH in water (5mL) at 0°C was added dropwise propargyl chloride (767mg, 10.3mmol, 745µL). Vigorous stirring was continued overnight after which time the mixture was neutralized by HCl at 0°C and the propargyl ether extracted into pentane. The extract was evaporated and the propargyl ether was purified on a short silica gel column (2 % diethyl ether/pentane) to give 778mg of propargyl ether **B**, [α]_{D} -118.9° (c 2.0, CHCl₃); ¹H NMR (CDCl₃) δ 5.70 (4-H; dq, 18.5/6.5), 5.31 (3-H; ddd, 18.5/7.2/1.4), 4.15 (1'-H; dd, -15.6/2.1), 4.01 (1'-H; dd, -15.6/2.1), 4.01 (2-H; m), 2.38 (3'-H; t, 2.1), 1.73 (5-H; dd, 6.5/1.4), 1.25 (1H; d, 6.3).

### (3R,4R)-4-Methylhept-5(E)-en-1-yn-3-ol (C)

An aliquot of butyl lithium hexane solution (2.5M, 5.1mL, 12.8mmol) was evaporated *in vacuo* and the residue cooled to -90°C. A solution of propargyl ether **B** (454mg, 3.66mmol) in 10mL of THF was slowly added. After allowing the temperature to increase to room temperature overnight, the reaction mixture was quenched with NH₄Cl solution. Extraction with ether three times, evaporation of the dried extract, and purification of the residue on a silica gel column (5% EtOAc/hexane) gave 322mg of alcohol **C** (71% yield), [α]_{D} +32.9° (c 3.0, CHCl₃); IR (NaCl) νₘₐₓ 3306, 2968, 1455, 1379, 1029, 975 cm⁻¹. ¹H NMR (CDCl₃) 6 5.61 (6-H; dq, 15.3/6.3), 5.38 (5-H; dd, 15.3/7.7), 4.13 (3-H; bs), 2.45 (1-H; d, 1.5), 2.38 (4-H; m), 2.20 (OH; bd, 3.3), 1.68 (7-H; d, 6.2), 1.09 (4-CH₃; d, 6.8). ¹³C NMR (CDCl₃) δ 131.5 (5), 127.9 (6), 83.5 (2). 73.6 (1), 66.2 (3), 43.4 (4), 18.1 (7), 15.7 (4-Me).

### (3S,4R)-3-tert-Butyldimethylsilyloxy-4-methylhept-5E-enal (D)

To a stirred solution of alcohol **C** (248mg, 2mmol) and imidazole (340mg, 5mmol) in 3mL of dry DMF was added *tert*-butyldimethylsilyl chloride (452mg, 3mmol). After stirring the mixture overnight, 10mL of 10% NaOH was added to destroy the excess *tert*-butyldimethylsilyl chloride. The product was extracted into ether and the extract washed successively with water, 0.5 N HCl, and water, dried and evaporated. Purification of the residue by chromatography on silica gel with hexane gave 457mg of (3*R*,4*R*)-3-*tert*-butyldimethylsilyloxy-4-methylhept-5(*E*)-en-1-yne (96% yield), ¹H NMR (CDCl₃) δ 5.50 (6-H; dq, 15.3/6.1), 5.38 (5-H; dd, 15.3/7.5), 4.16 (3-H; dd, 5.7/1.7), 2.37 (1-H; d, 1.7), 2.35 (4-H; m), 1.68 (7-H; d, 6.1), 1.07 (4-Me; d, 6.8), 0.90 (CMe₃; s), 0.12 (SiMe; s), 0.09 (SiMe; s).

Using the same procedure the corresponding TBDPS derivative, (3*R*,4*R*)-3-*tert*-butyldiphenylsilyloxy-4-methylhept-5(*E*)-en-1-yne, was formed in 92% yield, [α]_{D} + 32.9° (c 3.0, CHCl₃). ¹H NMR (CDCl₃) δ 7.72/7.38 (2Ph-H₅), 5.32 (6-H; m), 5.25 (5-H; dd, 16.2/7.3), 4.29 (3-H; dd, 5.2/2.0), 2.38 (4-H; m), 2.33 (1-H; d, 2.0), 1.64 (7-H; d, 5.3), 1.11 (4-Me; d, 6.9), 1.06 (CMe₃). ¹³C NMR (CDCl₃) δ 136.1/ 135.9 /133.6 /129.7/129.6/127.5/127.3 (Ph), 132.4 (5), 126.1 (6). 83.3 (2). 73.5 (1), 68.0 (3). 43.6 (4), 26.9 (CMe₃), 19.4 (CMe₃), 18.0 (7), 14.7 (4-Me).

2-Methylbutene (1.15mL 2M solution in THF, 2.3mmol) was added to 1.1mL of BH₃ THF solution (1M, 1.1mmol) at -25°C and the mixture was stirred in an ice bath for two hours, The temperature was then cooled to -50°C and a solution of the TBS derivative (238mg, 1mmol) in 1mL of THF was added all at once. The cooling bath was removed and the reaction mixture was allowed to warm to and remain at room temperature for one hour. Then 2.2 M KH₂PO₄/K₂HPO₄ solution (4.8mL) and 30% H₂O₂ (0.8mL) were added at 0°C. One hour later, the THF was evaporated and the residue was extracted into ether. The dried ether extract was evaporated and the residue chromatographed on silica gel (1% EtOAc/hexane) to give 194mg of aldehyde **D** (76% yield). ¹H NMR (CDCl₃) δ 9.78 (1-H; t, 2.3), 5.46 (6-H; dq, 15.3/6.1), 5.34 (5-H; dd, 15.3/7.5). 4.13 (3-H; m), 2.47 (2-H; m), 2.31 (4-H; m), 1.66 (7-H; br d, 6.1), 0.99 (4-Me; d, 6.8), 0.87 (CMe₃; s). 0.07 (SiMe; s), 0.04 (SiMe; s).

The *tert*-butyldiphenylsilyl ether (TBDPS) derivative of the aldehyde was formed in 83% yield, ¹H NMR (CDCl₃) δ 9.52 (1-H; t, 2.4), 7.69/7.40 (2Ph-H₅), 5.28 (6-H; m), 5.22 (5-H; dd, 16.2/6.2), 4.19 (3-H; m), 2.42 (2-H; m), 2.29 (4-H; m), 1.60 (7-H; d, 5.4), 1.07 (CMe₃), 1.02 (4-Me; d, 6.9). ¹³C NMR (CDCl₃) δ 202.0 (1), 136.1/133.6/133.3/130.2/129.7/127.7/127.6 (Ph), 132.3 (5), 126.2 (6), 72.8 (3), 47.6 (2), 42.2 (4), 27.1 (CMe₃), 19.6 (CMe₃), 18.3 (7), 14.9 (4-Me).

### Methyl (5S,6R)-5-tert-Butyldimethylsilyloxy-6-methyl-7-oxonona-2E,7 E-dienoate (E)

To a stirred solution of aldehyde **D** (0.74g, 2.9mmol) and trimethyl phosphonoacetate (632mg, 3.5mmol) in 5mL of THF cooled to -78°C was added tetramethylguanidine (435µL, 3.5mmol). After 30 minutes the cooling bath was removed and the mixture was stirred for another four hours. The mixture was neutralized with 1N HCl and the product was extracted into ether. Evaporation of the dried ether extract left a residue which was chromatographed on silica gel (5% EtOAc/hexane) to give 0.814g of E (90% yield). ¹H NMR (CDCl₃,) δ 6.93 (3-H;dt, 15.6/7.8), 5.62 (2-H; dd, 15.6/1.2), 5.37 (8-H, m), 5.37 (7-H, m), 3.71 (OCH₃, s), 3.61 (5-H, m), 2.29 (4-H₂, m), 2.22 (6-H, m), 1.66 (9-H₃; br d, 6.1), 0.99 (6-Me; d, 6.8), 0.88 (CMe₃; s). 0.03 (SiMe; s), 0.01 (SiMe; s).

The *tert*-butyldiphenylsilyl ether (TBDPS) derivative of the aldehyde was formed in 90% yield, ¹H NMR (CDCl₃) δ 7.68/7.38 (2Ph-H₅), 6.75 (3-H;dt, 15.6/7.4). 5.62 (2-H; d, 15.6), 5.34 (8-H, m), 5.29 (7-H, m), 3.70 (5-H, m), 3.68 (OCH₃, s), 2.28 (4-H₂, m), 2.20 (6-H, m), 1.62 (9-H₃; d, 5.3), 1.08 (CMe₃), 0.99 (6-Me; d, 6.9). ¹³C NMR (CDCl₃) δ 166.7 (1), 146.4 (3), 136.0/134.2/133.8/129.62/129.56/127.5/127.4 (Ph), 132.5 (7), 125.8 (8). 122.6 (2), 76.2 (5), 51.3 (OCH₃), 41.7 (6), 36.8 (4), 27.0 (CMe₃), 19.4 (CMe₃), 18.1 (9), 14.7 (6-Me).

### Methyl (5S,6R)-5-tert-Butyldimethylsilyloxy-6-methyl-7-oxohept-2(E)-enoate (F)

Ozone was passed through a solution of methyl ester **E** (328mg, 1.0mmol) and 97µL of pyridine in 15mL of CH₂Cl₂ at -78°C and the progress of the ozonolysis was monitored by TLC analysis. After the methyl ester had been consumed, about 500mg of zinc dust and 1mL of glacial acetic acid were added. The temperature was slowly increased to 25°C. The mixture was filtered and the filtrate was washed successively with saturated CuSO₄ and NaHCO₃ solutions. After evaporation of the solvent, the crude aldehyde **F** (249mg. 83 %) was used in the next step without further purification. ¹H NMR (CDCl₃) δ 9.96 (7-H; t, 2.3), 6.96 (3-H; dt, 15.7/7.6), 5.90 (2-H; dd, 15.7/0.7), 4.05 (5-H; m), 3.74 (OMe; s), 2.51 (6-H; m). 2.45 (4-H₂; m), 1.09 (6-Me; d, 6.9), 0.88 (CMe₃; s), 0.04 (SiMe; s), 0.03 (SiMe; s).

### Methyl (5S,6R)-5-t-butyldimethylsilyloxy-6-methyl-8-phenyl-octa-2E,7 E-dienoate (G)

To a stirred solution of aldehyde **F** (25.0mg, 0.08mmol) in 1.5mL of THF at -78°C was added 0.80mL of a cold (-78°C) mixture of benzyltriphenylphosphonium chloride (268mg, 0.69mmol, in 6.9mL of THF) and n-butyl lithium (280µL, 2.5M in hexace). After 15 min, the cold bath was removed and stirring was continued for 2 h. The reaction was quenched with saturated ammonium chloride solution and the THF was evaporated. The concentrate was extracted with hexane twice and the combined extract was washed with brine, dried and evaporated. The residual oil, a 5:1 mixture of the E and Z isomers, was dissolved in 1.5mL of benzene containing thiophenol (0.02M) and 1,1'azobis(cyclohexanecarbordtrile) (VAZO, 0.006M) and the mixture was refluxed for 5h. After cooling to RT, hexane (15mL) was added and the organic solution was washed successively with 10% NaOH and brine, dried (MgSO₄), and evaporated.
Chromatography of the residue on silica gel (2% EtOAc/hexane) led to 24mg (80%) of **G**, [α]_{D} +68.2° (c 1.5, CHCl₃); EIMS *m*/*z* 374 (<1%; M⁺), 359 (1; M⁺-CH₃), 317 (10; M⁺-^{*t*}Bu), 275 (10), 243, (73), 143 (20), 115 (10), 97 (64), 89 (31), 73 (100); HREIMS *m*/*z* 374.2232 (C₂₂H₃₄O₃Si, Δ +4.5mmu), 359.2031 (C₂₁H₃₁O₃Si, Δ+1.1mmu), 317.1579 (C₁₈H₂₅O₃Si, Δ-0.6mmu); UV (MeOH) λₘₐₓ (ε) 206 (33500), 252 (20100) nm; IR νₘₐₓ 2952, 2855, 1725, 1657, 1435, 1257, 1168, 1097, 970, 836, 775 cm⁻¹; ¹H NMR δ 7.2-7.4 (Ph-H₅; m), 6.96 (3-H; ddd, 15.6/7.8/7.5), 6.37 (8-H; d, 15.9). 6.16 (7-H; dd, 15.9/8.1), 5.84 (2-H; d, 15.6), 3.75 (5-H; ddd, 10.2/6.0/4.2), 3.72 (OMe; s), 2.44 (6-H; m), 2.36 (4-H₂; m), 1.10 (6-Me; d, 6.9), 0.91 (Si-CMe₃; s), 0.06 (Si-Me; s), 0.05 (Si-Me; s); ¹³C NMR δ 166.8 (1), 146.4 (3), 137.6 (Ph 1'), 131.9 (8), 130.4 (7), 128.5 (Ph 3'/5'), 127.0 (Ph 4'), 126.0 (Ph 2'/6'), 122.9 (2), 75.0 (5), 51.4 (OMe), 42.8 (6), 37.6 (4), 25.9 (Si-CMe₃), 18.1 (Si-CMe₃), 16.2 (6-Me), -4.4 (Si-Me), -4.5 (Si-Me). Calcd for C₂₂H₃₄O₃Si: C, 70.52; H, 9.17. Pound: C, 70.72; H, 9.42.

### (5S,6R)-5-t-Butyldimethylsilyloxy-6-methyl-8-phenylocta-2E,7 E-dienoic acid (H)

To a solution of ester **G** (159mg, 0.43mmol) in 7mL acetone was added 5mL of 1N aq LiOH. The mixture was stirred at 25°C for 3h, diluted with 20mL of Et₂O, and acidified to ≈ pH 4 with 1N HCl. The organic layer was separated and washed with 20mL portions of brine and water, dried (MgSO₄) and evaporated. Chromatography of the residual oil on silica gel with 40% EtOAc in hexane containing 0.5% AcOH resulted in pure acid **H** as a pale yellow mobile oil (145mg, 95% yield): [α]_{D} +87.0° (c 1.4, CHCl₃); EIMS *m*/*z*; 343 (1; M⁺-OH), 303 (5), 275 (9), 257 (4), 229 (62), 213 (16), 171 (22), 143 (37), 131 (16), 115 (23), 97 (100), 91 (44); HREIMS *m*/*z* 343.2107 (C₂₁H₃₁O₂Si, Δ-1.3mmu), 229.1220 (C₁₅H₁₇O₂, Δ+0.9mmu); UV λₘₐₓ (ε) 206 (24500), 252 (15600) nm; IR νₘₐₓ 3300-2800 (br), 2956, 2856, 1697, 1651, 1419, 1256, 1097, 836, 693 cm⁻¹; ¹H NMR δ 10.4 (CO₂H; bs, W_{1/2}≈100), 7.2-7.4 (Ph-H₅; m), 7.09 (3-H; ddd, 15.6/7.6/7.6), 6.39 (8-H; d, 15.9), 6.16 (7-H; dd, 15.9/8.1), 5.85 (2-H; d, 15.6), 3.78 (5-H; ddd, 6.0/6.0/4.2), 2.46 (6-H; m), 2.40 (4-H₂; m), 1.12 (6-Me; d, 6.9), 0.92 (Si-CMe₃; s), 0.07 (SiMe₂; s); ¹³C NMR δ 171.6 (1), 149.1 (3), 137.5 (Ph 1'), 131.8 (8), 130.5 (7), 128.5 (Ph 3'/5'), 127.1 (Ph 4'), 126.1 (Ph 2'/6'), 122.7 (2), 74.9 (5), 42.9 (6). 37.6 (4). 25.8 (Si-CMe₃), 18.1 (Si-CMe₃), 16.1 (6-Me), -4.4 (Si-Me), -4.5 (Si-Me).

### 2,2,2-Trichloroethyl Ester of 3-(3-Chloro-4-methoxyphenyl)-D-alanine (I)

A sample of the D-chlorotyrosine BOC derivative (160mg, 0.35mmol) was dissolved in 3mL neat trifluoroacetic acid and allowed to stand at room temperature for 1h. Removal of the excess reagent under reduced pressure returned the desired amine **I** as the trifluoroacetate salt (165mg, 100% yield), [α]_{D} +1.7° (c 5.2, CHCl₃); IR νₘₐₓ 3400-2500 (br), 1760, 1680, 1500, 1200, 1130, 1070, 805, 710 cm⁻¹; ¹H NMR δ 8.07 (NH₂; br m, W_{1/2}≈45), 7.27 (5-H; s), 7.12 (9-H; d, 8.1), 6.88 (8-H; d. 8.1), 4.86/4.67 (CH₂CCl₃; AB q, -12.0), 4.41 (2-H; bs, W_{1/2}≈20), 3.86 (OMe; s). 3.33 (3-H; dd, -14.4/3.6), 3.22 (3-H'; dd, -14.4/6.6); ¹³C NMR δ 167.6 (1), 155.0 (7). 130.9 (5), 128.8 (9), 125.4 (4), 123.1 (6), 112.7 (8), 93.4 (CCl₃), 75.3 (CH₂CCl₃), 56.1 (OMe), 54.2 (2), 34.9(3).

### Compound J

To a stirred solution of **H** (25mg, 0.07mmol) in 3mL of anhydrous DMF under argon was added successively pentafluorodiphenylphosphinate (FDPP, 32mg, 0.08mmol), trifluoroacetate salt **I** (35mg, 0.07mmol) and diisopropylethylamine (DIEA, 27mg, ≈ 36µL, 0.21mmol, ≈ 3 equiv). Stirring was continued at 25°C for 1h and then the reaction mixture was extracted with 20mL of Et₂O. The ether extract was washed with 10mL of 1N HCl, followed by 10mL of sat'd NaHCO₃, 20mL of brine and -20mL of water, dried (MgSO₄), and evaporated. The residual pale yellow oil was subjected to chromatography on silica gel (15% EtOAc in hexane) to give **J** as a colorless oil (32mg, 65% yield): [α]_{D} +11.8° (c 1.2, CHCl₃); EIMS *m*/*z*; 644/646/648/650 (7/8/6/3; M⁺-'Bu), 570/572/574 (46/100/21), 536/538 (18/15), 394/396 (67/29), 275 (20). 155/157 (29/9); HREIMS *m*/*z* 644.0981 (C₂₉H₃₄Cl₄NO₅Si, Δ-2.13mmu); UV λₘₐₓ (ε) 204 (54900), 230 (23200), 248 (19200), 284 (3500) nm; IR νₘₐₓ 3290, 2980, 2850, 1760, 1680, 1640, 1505, 1380, 1270, 1169, 990, 720 cm⁻¹; ¹H NMR *unit A* δ 7.2-7.4 (Ph-H₅; m), 6.87 (3-H; ddd, 15.0/7.8/7.5), 6.37 (8-H; d, 16.2), 6.18 (7-H; dd, 16.2/8.1), 5.82 (2-H; d, 15.0), 3.75 (5-H; ddd, 9.9/6.0/4.8), 2.46 (6-H; m), 2.36 (4-H₂; m), 1.11 (6-Me; d, 6.9), 0.91 (SiCMe₃; s), 0.07 (SiMe; s), 0.06 (SiMe; s); *unit B* δ 7.19 (5-H; d, 2.1), 7.04 (9-H; dd, 8.4/2.1), 6.85 (8-H; d, 8.4), 5.85 (NH; d, 7.8), 5.08 (2-H; ddd, 7.8/6.0/5.7), 4.81/4.74 (CH₂CCl₃; AB q, -11.7), 3.87 (OMe; s), 3.22 (3-H; dd, -14.1/5.7), 3.12 (3-H'; dd, -14.1/6.0). ¹³C NMR *unit A* δ 165.1 (1), 143.0 (3), 137.6 (9), 132.0 (8), 130.4 (7), 128.5 (11/13), 127.0 (12), 126.0 (10/14), 124.7 (2), 75.0 (5), 42.6 (6), 37.6 (4), 25.9 (Si-CMe₃), 18.1 (Si-CMe₃), 16.5 (6-Me), -4.3 (Si-Me), -4.6 (Si-Me); *unit B δ* 170.1 (1), 154.3 (7), 131.1 (5), 128.5 (4/9), 122.6 (6), 112.2 (8), 94.2 (CCl₃), 74.8 (CH₂CCl₃), 56.1 (OMe), 53.0 (2), 36.5 (3).

### (1'R,5S,6R)-N-1'-(carbo-2",2",2"-trichloroethoxy)-2'-(3-chloro-4-met hoxyphenyl)ethyl-5-t-butyldimethylsilyloxy-6-methyl-8-phenyl-octa-2E,7E-dienamide (K)

To a solution of **J** (50mg, 0.07mmol) in 4mL MeCN was added 400µL of 50% aq HF and the mixture stirred for 1h at 25°C. Extraction into 30mL of Et₂O, followed by washing the ether extract with 30mL portions of sat'd NaHCO₃, brine and water, drying (MgSO₄) and evaporation, gave alcohol **K** as a colorless foam (40mg, 95% yield): [α]_{D} - 6.1° (c 1.3, CHCl₃); EIMS *m*/*z* (rel intensity) 587/589/591/593 (M +, < 1%), 552/554/556 (1/2/0.5), 456/458/460/462 (1/2/1/0.2), 342/344/346 (7/8/4), 212/214 (15/5), 195/197 (6/2), 155/157 (99/34), 131 (100), 91 (77); HREIMS *m*/*z* 587.0721 (C₂₇H₂₉³⁵Cl₄NO₅, Δ+7.9mmu); UV λₘₐₓ 204 (56500), 230 (22100), 248 (18100), 284 (3600) nm; IR νₘₐₓ 3400, 3300, 2980, 1780, 1680, 1640, 1505, 1270, 1180, 1090, 1000, 770 cm⁻¹. ¹H NMR *unit A* δ 7.2-7.4 (Ph-H₅; m), 6.92 (3-H; ddd, 15.3/7.8/7.5), 6.46 (8-H; d, 15.9), 6.14 (7-H; dd, 15.9/8.4), 5.90 (2-H; d, 15.3), 3.65 (5-H; ddd 7.8/5.6/4.0), 2.39 (6-H/4-H₂; bm), 1.78 (OH; bs, W_{1/2} ≈ 40 Hz), 1.14 (b-Me; d, 6.9); *unit B δ* 7.18 (5-H; d, 1.8), 7.03 (9-H; dd, 8.4/1.8), 6.84 (8-H; d, 8.4), 5.97 (NH; d, 7.8), 5.06 (2-H; ddd, 7.8/6.0/5.7), 4.79/4.72 (CH₂CCl₃; AB q, -12.0), 3.86 (OMe; s), 3.20 (3-H; dd, -14.1/5.7), 3.10 (3-H'; dd, -14.1/6.0). ¹³C NMR *unit A δ* 165.3 (1), 142.6 (3), 137.0 (9), 131.7 (8), 131.0 (7), 128.5 (11/13), 127.3 (12), 126.1 (10/14), 125.0 (2), 73.8 (5), 43.2 (6), 37.2 (4), 16.8 (6-Me); *unit B* δ 170.2 (1), 154.2 (7), 131.0 (5), 128.4 (9), 128.3 (4), 122.5 (6), 112.2 (8), 94.2 (CCl₃), 74.7 (CH₂CCl₃), 56.1 (OMe), 53.0 (2), 36.5 (3).

### 3-(tert-Butoxycarbonyl)amino-2,2-dimethylpropanoic Acid (M)

To a solution of 3-amino-2,2-dimethylpropan-1-ol (L) (3.0g, 29mmol) in 51mL of a 10 % solution of triethylamine in MeOH was added di-*tert*-butyl dicarbonate (6.7g, 31mmol) and the mixture was stirred at 25°C for 1h. After removal of solvent, the residue was dissolved in CH₂Cl₂ (30mL) and the solution was washed twice with 1M KHSO₄ (pH 2) and once with saturated NaCl solution, and dried (MgSO₄). Removal of solvent *in vacuo* afforded 5.8g (93% yield) of 3-(*tert*-butoxycarbonyl)amino-2,2-dimethylpropan-1-ol as a white solid which was directly used for the next step without further purification (>95% pure by NMR analysis), mp 70.5-71.5°C; IR *ν*_{*max*} 3350, 1685, 1456 cm⁻¹; ¹H NMR δ 4.87 (NH; br s), 3.72 (OH; br s), 3.19 (1-H₂; d, 5.1), 2.95 (3-H₂; d, 6.0), 1.44 (CMe₃; s), 0.85 (2-Me₂; s); ¹³C NMR (CDCl₃) δ 157.6 (BOC CO), 79.7 (CMe₃), 68.1 (1), 47.1 (3). 36.7 (2), 28.3 (CMe₃), 22.4 (2-Me₂).

To a solution of alcohol 3-(*tert*-butoxycarbonyl)amino-2,2-dimethylpropan-1-ol (5.3g, 25.9mmol) and sodium periodate (16.6g, 77.7mmol) in carbon tetrachloride (52mL), acetonitrile (52mL) and water (78mL) was added ruthenium trichloride hydrate (122mg), and the mixture was stirred at 25°C for 1h. The mixture was filtered through Celite and a saturated solution of potassium carbonate in water (50mL) was added. The water layer was separated, washed with ether (20mL), acidified with HCl to pH 2 at 0°C and extracted with CH₂Cl₂ (30mL x 3). The combined extracts were washed with saturated NaCl solution and dried (MgSO₄). Removal of solvent *in vacuo* yielded a residue that was first subjected to flash reversed-phase chromatography on a C18 silica (ODS 120Å, 50 to 90% MeOH) and then crystallized from ether to give 3.7g (66% yield) of **M** as a white solid, mp 106-108°C; EIMS *m*/*z* (rel intensity) 217 (0.1). 161 (11). 98 (25), 88 (71), 57 (100); HREIMS *m*/*z* 217.1292 (C₁₀H₁₉NO₄, Δ+2.2mmu); IR νₘₐₓ 3450-2500, 1710, 1694, 1510 cm⁻¹; ¹H NMR of major conformer δ 5.03 (NH; br s), 3.26 (3-H₂; m), 1.45 (CMe₃; s), 1.24 (2-Me₂; s); ¹³C NMR (CDCl₃) δ 183.2 (1), 156.3 (BOC CO), 79.6 (CMe₃), 49.5/47.9 (2/3), 28.4 (CMe₃), 22.9 (2-Me₂).

### Allyl (2S)-2-Hydroxy-4-methylpentanoate (N)

To a solution of 2.66g of L-leucic acid (20mmol) and 1.74g of sodium bicarbonate (20mmol) in 30mL water at 0°C was added 30mL of a CH₂Cl₂ solution of 6.44g of tetrabutylammonium chloride (20mmol) and 1.74mL of allyl bromide (20mmol). After vigorously stirring the mixture for 24h, the CH₂Cl₂ was evaporated. About 50mL water was added and the aqueous layer was extracted four times with Et₂O. The ether solution was dried over anhydrous sodium sulfate and then evaporated. The residue was passed through a short Si column to give 3.21g of allyl ester **N** (93% yield) as a colorless oil, [α]_{D} -8.4° (c 1.1, CHCl₃); IR νₘₐₓ 3464, 2957, 1732, 1203, 1140, 1087 cm⁻¹; ¹H NMR δ 5.92 (allyl 2-H; m), 5.34 (allyl 3-H_{*z*}; dd, 17.4/1.1), 5.28 (allyl 3-H_{*E*}; dd, 10.5/1.1), 4.67 (allyl 1-H₂; d, 5.7), 4.23 (2-H; br s), 2.64 (OH; br s), 1.89 (4-H; m), 1.57 (3-H₂; m), 0.96 (5-H₃; d, 6.5), 0.95 (4-Me; d, 6.7); ¹³C NMR δ 175.3 (1), 131.4 (allyl C-2), 118.6 (3). 68.9 (2), 65.7 (allyl C-1), 43.2 (3), 24.1 (4), 23.0 (5), 21.3 (4-Me).

### Allyl (2S)-2-[3'(tert-Butoxycarbonyl)amino-2',2'-dimethylpropanoyloxy]-4- methylpentanoate (O)

To a solution of 0.8g of **M** (3.7mmol), 0.76g of **N** (4.4mmol), and 92mg DMAP in 10mL of dry CH₂Cl₂ at 0°C was added 0.84g of DCC (4.1mmol) in CH₂Cl₂. The mixture was stirred at 25°C for 3h and filtered. The filtrate was washed with saturated aqueous sodium bicarbonate, dried (Na₂SO₄), and evaporated *in vacuo.* Flash chromatography (silica gel, 5% EtOAc/hexane) afforded 1.0g (92% yield) of pure **O** as a colorless oil, R_{f} 0.68 (17:83 EtOAc/hexane), [α] _{D} -29.4° (c 18.1, CHCl₃); EIMS *m*/*z* (rel intensity) 371 (2, M⁺), 242 (13), 184 (12), 126 (20), 84 (100); HREIMS *m*/*z* 371.2317 (C₁₉H₃₃NO₆, Δ-0.9mmu); IR (neat) νₘₐₓ 3385, 2963, 1731, 1720, 1513 cm⁻¹; ¹H NMR (300 MHz, CDCl₃) *unit C δ* 5.39 (NH; obscured br s), 3.33 (3-H; dd, -13.5/7.4), 3.27 (3-H'; dd, -13.5/5.9), 2.78 (2-H, m), 1.44 (CMe₃; s), 1.23 (2-Me; s), 1.22 (2-Me; s); *unit D* δ 5.91 (allyl 2-H, ddt, 16.6/10.3/6.0 Hz), 5.34 (allyl 3-H_{*z*}; bd, 16.6), 5.27 (allyl 3-H_{*E*}; bd, 10.3), 5.08 (2-H; dd, 9.6/3.6), 4.65 (allyl 1-H₂; m), 1.6-1.9 (3-H₂/4-H; m), 0.94 (5-H₃; d, 6.3), 0.94 (4-Me; d, 7.3). ¹³C NMR *unit C* δ 176.5 (1), 156.3 (BOC CO), 79.0 (CMe₃), 48.6 (3), 44.0 (2), 28.4 (CMe₃), 22.2/23.0 (2-Me₂); *unit D* δ 170.6 (1), 131.4 (allyl C-2), 119.1 (allyl C-3), 70.9 (2), 66.0 (allyl C-1), 39.5 (3), 24.8 (4), 23.0 (5), 21.5 (4-Me).

### (2S)-2-[3'(tert-Butoxycarbonyl)amino-2',2'-dimethylpropanoyloxy]-4-methylpentanoic Acid (P)

To 10mL of a solution of 180mg (0.49mmol) of **O** and 60mg (0.05mmol) of tetrakis(triphenylphosphine)palladium in dry THF (under argon atmosphere) was slowly added 470µL (5.4mmol) of dry morpholine over 10 min. After stirring for 50 min, 40mL of ether was added and the solution was washed with 1N HCl (40mL) and then extracted with saturated sodium bicarbonate (2 x 30mL). The aqueous extract was acidified with 0.5N HCl and extracted with ether (40mL). The ether extract was washed with water (2 x 30mL), dried (MgSO₄) and evaporated *in vacuo* to give **P** as a colorless mobile oil (152mg, 95%); [α]_{D} -22.2° (c 2.2, CHCl₃); EIMS *m*/*z* (rel intensity) 331 (1, M⁺), 275 (1), 258 (4), 231 (9), 202 (36), 174 (13), 144 (31), 126 (16), 114 (14), 98 (54), 88 (50), 84 9100); HREIMS *m*/*z* 331.2004 (C₁₆H₂₉NO₆, Δ -1.0mmu). ¹H NMR (CDCl₃) *unit C* δ 5.41 (NH; dd, 5.7/5.4), 3.30 (3-H₂; m), 2.68 (2-H; m), 1.43 (CMe₃; br s), 1.22 (2-Me; s), 1.21 (2-Me; s); *unit D* δ 6.47 (1-OH; br s, W_{1/2}≈35), 5.09 (2-H; dd, 9.3/2.7), 1.7-1.9 (3-H₂/4-H; m), 0.97 (5-H₃; d, 6.0), 0.94 (4-Me; d, 6.0). ¹³C NMR (CDCl₃) *unit C* δ 176.5 (1), 156.5 (BOC CO), 79.3 (CMe₃), 48.6 (3), 44.0 (2), 28.3 (CMe₃), 23.0 (2-Me), 22.2 (2-Me); *unit D* δ 175.4 (1), 70.6 (2), 39.5 (3), 24.8 (4), 23.0 (5), 21.4 (4-Me).

### Compound O

To a solution of alcohol **K** (80mg, 0.14mmol), acid **P** (68mg, 0.21mmol) and DMAP (4mg) in dry CH₂Cl₂ (4mL) stirred at 0°C under an argon atmosphere was added DCC (44mg, 0.21mmol) in dry CH₂Cl₂ (1mL). The mixture was stirred at 0°C for 30 minutes, during which time a white precipitate developed, and then allowed to warm to room temperature and stirred for a further 4 hours. The precipitate was filtered off and the filtrate diluted with Et₂O (40mL) and washed successively with dilute HCl (1M, 40mL), saturated NaHCO₃ (40mL) and brine (40mL). The ethereal layer was dried (MgSO₄) and evaporated *in vacuo* to give a waxy solid. Chromatography (silica, EtOAc:hexane, 1:3) led to pure **Q** as a colorless, viscous oil (103mg, 84%), [α]_{D} -3.1° (c 2.9, CHCl₃); EIMS *m*/*z* 800/802/804/806 (<1, M⁺-C₅H₈O₂), 415/417/419/421 (5/3/3/2), 342/344/346 (7/9/4), 286/288/290 (2/6/2), 207 (34), 178 (22), 155/157 (66/24), 131 (36), 91 (70), 70 (100); HREIMS *m*/*z* 800.2179 (C₃₈H₄₈N₂O₈³⁵Cl₄, Δ-1.4mmu); UV (MeOH) λₘₐₓ (ε) 204 (51200), 230 (18500), 248 (17200), 282 (2200) nm; IR (NaCl) νₘₐₓ 3376, 2965, 1755, 1728, 1712, 1678, 1504, 1258, 1150, 1067, 732 cm⁻¹. ¹H NMR (CDCl₃) δ *unit A:* 7.28-7.33 (10-H/14-H/11-H/13-H; m), 7.22 (12-H; m), 6.78 (3-H; ddd, 15.8/6.4/6.3), 6.40 (8-H; d, 15.8), 6.01 (7-H; dd, 15.8/8.7), 5.88 (2-H; d, 15.8), 5.06 (5-H; bm, W_{1/2}≈20 Hz), 2.62 (6-H; m), 2.53 (4-H₂; bm, W_{1/2}≈15 Hz), 1.12 (6-CH₃; d, 6.8); *unit B* 7.18 (5-H; d, 2.0), 7.05 (9-H; dd, 8.5/2.0). 6.83 (8-H; d, 8.5), 6.49 (NH; d, 7.9), 5.06 (2-H; bm, W_{1/2}≈20 Hz), 4.79/4.70 (CH₂CCl₃; AB q, -11.7), 3.85 (OCH₃; s), 3.20 (3-H_{b}; dd, -14.1/5.8), 3.07 (3-Hₐ; dd, -14.1/6.7); *unit C* 5.38 (NH; bt, 6.5), 3.27 (3-H₂; d, 6.5), 1.20 (2-CH₃; s), 1.15 (2-CH₃'; s); *unit D* 4.92 (2-H; dd, 10.0/3.8), 1.72 (4-H; bm, W_{1/2}≈20 Hz), 1.67 (3-H_{b}; ddd, -14.1/10.0/5.0/), 1.56 (3-Hₐ; ddd, -14.1/9.1/3.8), 1.43 (CO₂CMe₃; s), 0.86 (4-CH₃; d, 6.4), 0.82 (5-H₃; d, 6.4). ¹³C NMR (CDCl₃) δ *unit A* 165.4 (1), 139.3 (3), 136.9 (9), 131.7 (8), 130.1 (7), 128.6 (11/13), 127.5 (12), 126.2 (10/14), 125.4 (2), 76.5 (5), 41.1 (6), 33.4 (4), 16.7 (6-Me); *unit B* 170.0 (1), 154.1 (7). 131.2.(5), 128.8 (4). 128.5 (9), 122.3 (6), 112.1 (8), 94.3 (CH₂CCl₃), 74.6 (CH₂CCl₃), 56.1 (7-OMe), 53.2 (2). 36.6 (3); *unit C* 176.9 (1). 156.4 (CO₂CMe₃), 79.1 (CO₂CMe₃), 48.7 (3), 44.0 (2), 22.8 (2-Me), 22.3 (2-Me'); *unit D* 170.7 (1), 71.4 (2), 39.5 (3), 28.4 (CO₂CMe₃), 24.8 (4), 23.0 (4-Me), 21.4 (5).

### Amino Acid R

To the amino acid **Q** (100mg, 0.11mmol) was added activated Zn dust (400mg, excess) and AcOH (4mL). The heterogenous mixture was subject to sonication for 45 minutes, stirred for a further 90 minutes at room temperature, and then poured onto a pad of Celite. The organic material was Washed from the Celite pad with CH₂Cl₂. The solvent was removed *in vacuo*, leaving the carboxylic acid as a colorless simorphous solid.

Without purification the crude acid was dissolved in trifluoroacetic acid (TFA, 5mL) and allowed to sit at room temperature for 1 hour. After this time excess TFA was removed *in vacuo* and the resulting amorphous solid was then subjected to chromatographic purification (Sep-Pak™, silica, initially CH₂Cl₂ then 10% MeOH/CH₂Cl₂), yielding the trifluoroacetate ammonium salt of the desired compound. Repeated lyophilization of an aqueous solution of the salt resulted in the free amino acid **R** as a colorless amorphous solid (68mg, 91% over two steps); IR (NaCl) *ν*ₘₐₓ 3300, 3200, 2965, 1693, 1606, 1504, 1441, 1259, 1201, 1146, 1066, 727 cm⁻¹. ¹H NMR (CD₃OD) *δ unit A:* 7.33 (10-H/14-H; d, 7.4), 7.28 (11-H/13-H; t, 7.4), 7.18-7.23 (12-H; m), 6.69 (3-H; ddd, 15.6/7.7/7.0), 6.43 (8-H; d, 15.8), 6.04 (7-H; dd, 15.8/8.9), 6.00 (2-H; d. 15.6), 5.01 (5-H; ddd, 9.1/6.9/3.1). 2.64 (4-H_{b}; bm, W_{1/2} ≈ 30 Hz), 2.60 (6-H; bm, W_{1/2}≈20 Hz), 2.49 (4-Hₐ; ddd, 15.8/9.1/7.7), 1.13 (6-Me; d, 6.7); *unit B* 7.18-7.23 (5-H; m), 7.11 (9-H; dd, 8.3/1.6), 6.92 (8-H; d, 8.3), 4.59 (2-H; bm, W_{1/2}≈20 Hz), 3.81 (OCH₃; s). 3.14 (3-H_{b}; dd, -13.7/4.3), 2.96 (3-Hₐ; m, W_{1/2}≈20 Hz); *unit C* 2.96 (3-H₂; bm, W_{1/2}≈20 Hz), 1.31 (2-CH₃; s), 1.25 (2-CH₃'; s); *unit D* 4:90 (2-H; dd, 9.6/4.0), 1.66 (4-H; bm, W_{1/2} = 25 Hz), 1.59 (3-H_{b}; ddd, -14.4/9.6/4.8), 1.53 (3-Hₐ; ddd, -14.4/9.1/4.0), 0.81 (4-Me; d, 6.5), 0.74 (5-H₃; d, 6.5). ¹³C NMR (CD₃OD) *δ unit A* 167.7 (1), 140.7 (3), 138.4 (9), 133.0 (8), 131.7 (7), 129.6 (11/13), 128.5 (12), 127.3 (10/14), 127.1 (2), 78.4 (5), 43.1 (6), 35.7 (4), 17.4 (6-Me); *unit B* 179.8 (1), 155.2 (7), 132.3 (4), 132.1 (5), 130.1 (9), 123.0 (6), 113.4 (8), 56.6 (7-OMe), 56.6 (2), 37.8 (3), *unit C* 176.8 (1), 48.2 (3), 42.2 (2), 23.3 (2-Me), 23.3 (2-Me'); *unit D* 172.0 (1), 73.4 (2), 40.7 (3), 26.0 (4), 23.1 (4-Me), 21.8 (5).

### Cryptophycin 51

To a stirred solution of amino acid **T** (75mg, 0.11mmol) in anhydrous DMF (20mL) at room temperature under argon was added diisopropylethylamine (DIEA, 44mg, 60µL, 0.34mmol, ≈ 3 equiv.) followed by pentafluorodiphenylphosphinate (FDPP, 55mg, 0.14mmol, ≈ 1.3 equiv.) in DMF (2mL). The mixture was stirred for 12 hours, Et₂O (40mL) was added, and the ether layer was washed successively with HCl (1M, 40mL), brine (40mL) and H₂O (40mL), dried (MgSO₄) and evaporated under reduced pressure. The residual waxy solid was further purified by reverse phase chromatography (ODS, 10µ, 30% H₂O/MeCN, 3mL min⁻¹) to give Cryptophycin **51** as a colorless amorphous solid (45mg, 61%), [α]_{D} +26.4° (c 0.25, CHCl₃); EIMS *m*/*z* 652/654 (M⁺, 3/1), 632/634 (3/2), 426/428 (51/15), 227 (64), 195/197 (64/22), 155/157 (71/15), 131 (59), 91 (100); HREIMS *m*/*z* 652.2936 (C₃₆H₄₅N₂O₇³⁵Cl, Δ-2.1mmu); UV (MeOH) λₘₐₓ (ε) 204 (52000), 228 (20400), 250 (13400), 284 (2800) nm; IR (NaCl) νₘₐₓ 3376, 3270, 2960, 1747, 1721, 1659, 1536, 1514, 1259, 1150, 1066, 1013, 980, 694 cm⁻¹. ¹H NMR (CDCl₃) δ *unit A* 7.32 (10-H/14-H; dd, 8.0/1.5), 7.29 (11-H/13-H; t, 8.0), 7.24 (12-H; bm, W_{1/2}≈15 Hz), 6.77 (3-H; ddd, 15.2/10.8/4.3), 6.40 (8-H; d, 15.8), 6.01 (7-H; dd, 15.8/8.8), 5.76 (2-H; dd, 15.2/1.1), 5.04 (5-H; ddd, 11.1/6.4/1.9), 2.54 (4-H_{b}/6-H; bm, W_{1/2}≈15 Hz), 2.37 (4-Hₐ; ddd, -14.3/11.1/10.8 ), 1.13 (6-Me; d, 6.8); *unit B* 7.20 (5-H; d, 2.0), 7.05 (9-H; dd, 8.4/2.0), 6.84 (8-H; d, 8.4), 5.61 (NH; d, 7.8), 4.74 (2-H; ddd, 7.8/7.6/5.4), 3.87 (OMe; s), 3.11 (3-H_{b}; dd, -14.2/5.4), 3.06 (3-Hₐ; dd, -14.2/7.6); *unit C* 7.24 (NH; bm, W_{1/2}≈15 Hz), 3.40 (3-H_{b}; dd, -13.5/8.5), 3.12 (3-Hₐ; dd, -13.5/3.6), 1.22 (2-Me; s), 1.15 (2-Me', s); *unit D* 4.85 (2-H; dd, 10.2/3.6), 1.66 (3-H_{b}; ddd, -14.0/10.2/4.6), 1.61 (4-H; bm W_{1/2}≈20.0 Hz), 1.33 (3-Hₐ; ddd, -14.0/9.0/3.6), 0.74 (4-Me; d, 6.6), 0.72 (5-H₃; d, 6.6). ¹³C NMR (CDCl₃) δ *unit A* 165.1 (1), 142.2 (3), 136.7 (9), 131.7 (8), 130.1 (7), 128.6 (11/13), 127.5 (12), 126.1 (10/14), 124.6 (2), 77.0 (5), 42.2 (6), 36.5 (4), 17.3 (6-Me); *unit B* 170.3 (1), 154.1 (7), 130.9 (5), 129.5 (4), 128.3 (9), 122.5 (6), 112.3 (8), 56.1 (7-OMe), 54.2 (2), 35.3 (3); *unit C* 178.0 (1), 46.5 (3), 42.7 (2), 22.8 (2-Me), 22.6 (2-Me'); *unit D* 170.6 (1), 71.5 (2), 39.5 (3), 24.5 (4), 22.7 (4-Me), 21.2 (5).

### Example 3 Synthesis of Cryptophycin 52 and Cryptophycin 53

To a stirred solution of Cryptophycin **51** (75mg, 0.12mmol) in anhydrous dichloromethane (7.5mL) at 0°C under argon was added a solution of *m*-chloroperbenzoic acid (*m*CPBA. 50mg, 0.23mmol, ≈2 equiv. based on 80% active oxygen) in dichloromethane (1mL). After 30 minutes the reaction mixture was allowed to warm to room temperature and stirred for a further 12 hours. The solvent was then removed under reduced pressure to give a 1.8:1 mixture of Cryptophycins **52** and **53** (by NMR analysis), respectively, as an amorphous solid. The mixture of regioisomeric epoxides was dissolved in minimal acetonitrile and subjected to reverse phase chromatography (YMC-ODS. 10µ, 250mm x 22.5mm, 30% H₂O/MeCN, 6mL min⁻¹) to separate Cryptophycin **52** (37mg, 48 %) and Cryptophycin **53** (19mg, 25 %).

### Spectral data for Cryptophycin 52

[α]_{D} +19.9° (c 0.5, CHCl₃); EIMS *m*/*z* 668/670 (4/2, M⁺), 445 (35), 244 (12), 227 (22), 195/197 (66/27). 184 (45), 155/157 (38/10), 91 (100); HREIMS *m*/*z* 668.2873 (C₃₆H₄₅N₂O₈³⁵Cl, Δ-0.9mmu), 445.2497 (C₂₅H₃₅NO₆, Δ-3.3mmu); UV (MeOH) λₘₐₓ (ε) 204 (35100), 218 (20900) nm; IR (NaCl) νₘₐₓ 3415, 3270, 2960, 1748, 1721, 1650, 1536, 1504, 1260, 1192, 1150, 1066, 1013, 800, 698 cm⁻¹. ¹H NMR (CDCl₃) *δ unit A* 7.33-7.38 (11-H/12-H/13-H; bm, W_{1/2} ≈ 25 Hz), 7.24 (10-H/14-H; m, W_{1/2} ≈ 15 Hz), 6.76 (3-H; ddd, 15.1/10.8/4.3), 5.71 (2-H; dd, 15.1/1.7), 5.20 (5-H; ddd, 11.0/5.0/1.8), 3.68 (8-H; d, 1.9), 2.92 (7-H; dd, 7.5/1.9), 2.57 (4-H_{b}; ddd, -14.6/1.8/1.7), 2.45 (4-Hₐ; ddd, -14.6/11.0/10.8), 1.78 (6-H; bm, W_{1/2} ≈ 15 Hz), 1.14 (6-Me; d, 6.9); *unit B* 7.18 (5-H; d, 2.2), 7.04 (9-H; dd, 8.4/2.2), 6.83 (8-H; d, 8.4), 5.56 (NH; d, 7.9), 4.73 (2-H; ddd, 7.9/7.4/5.3). 3.87 (OMe; s), 3.09 (3-H_{b}; dd, -14.6/5.3), 3.05 (3-Hₐ; dd, -14.6/7.4); *unit C* 7.20 (NH; dd, 8.6/3.2). 3.41 (3-H_{b}; dd, -13.4/8.6), 3.10 (3-Hₒ; dd, -13.4/3.2), 1.22 (2-Me; s), 1.15 (2-Me'; s); *unit D* 4.82 (2-H; dd, 10.2/3.5), 1.73 (3-H_{b}; bm, W_{1/2}≈20 Hz), 1.66 (4-H; bm, W_{1/2}≈20 Hz), 1.31 (3-Hₐ; ddd, -13.8/9.1/3.5), 0.84 (4-Me; d, 6.6), 0.82 (5-H₃; d, 6.6); ¹³C NMR (CDCl₃) *δ unit A* 164.9 (1), 141.8 (3), 136.7 (9), 128.7 (11/13), 128.3 (12), 125.6 (10/14), 124.7 (2), 75.9 (5), 63.0 (7), 59.0 (8), 40.7 (6), 36.9 (4), 13.5 (6-Me), *unit B* 170.3 (1), 154.1 (7), 130.9 (5), 129.5 (4), 128.5 (9), 122.6 (6), 112.4 (8), 56.1 (7-OMe), 54.3 (2), 35.3 (3), *unit C* 178.0 (1), 46.5 (3), 42.8 (2), 22.8 (2-Me), 22.8 (2-Me'), *unit D* 170.5 (1), 71.2 (2), 39.3 (3), 24.6 (4), 22.7 (4-Me), 21.2 (5).

### Spectral data for Cryptophycin 53

[α]_{D} +20.8° (c 1.7, CHCl₃); EIMS *m*/*z* 668/670 (5/4, M⁺), 445 (32), 244 (15), 227 (24), 195/197 (64/21), 184 (60), 155/157 (33/9), 91 (100); HREIMS *m*/*z* 668.2853 (C₃₆H₄₅N₂O₈ ³⁵Cl, Δ1.1mmu); UV (MeOH) λₘₐₓ (ε) 204 (38700), 218 (22900) nm; IR (NaCl) νₘₐₓ 3415, 3280, 2917, 2849, 1748, 1722, 1660, 1504, 1465, 1260, 1190, 1150, 1066, 755 cm⁻¹. ¹H NMR (CDCl₃) δ *unit A* 7.29-7.36 (11-H/12-H/13-H, bm, W_{1/2}≈20 Hz), 7.23 (10-H/14-H; dd, 8.3/1.7), 6.77 (3-H; ddd, 15.1/10.9/4.3), 5.81 (2-H; dd, 15.1/1.3), 5.17 (5-H; ddd, 11.2/4.9/1.8), 3.58 (8-H; d, 1.7), 2.90 (7-H; dd, 7.8/1.7), 2.67 (4-H_{b}; ddd, 14.7/11.2/10.9), 2.56 (4-Hₐ; dddd, 14.7/4.3/1.8/1.3), 1.67-1.78 (6-H; bm, W_{1/2}≈45), 1.03 (6-CH₃; d, 7.1); *unit B* 7.21 (5-H; d, 2.1), 7.07 (9-H; dd, 8.5/2.1), 6.84 (8-H; d, 8.4), 5.90 (2-NH; d, 7.9), 4.75 (2-H; ddd, 7.9/7.9/4.9), 3.85 (7-OCH₃; s), 3.14 (3-H_{b}; dd, 14.5/4.9), 3.03 (3-Hₐ; dd, 14.5/7.9); *unit C* 7.29-7.36 (3-NH; bm, W_{1/2}≈25), 3.43 (3-H_{b}; dd, 13.7/8.8), 3.10 (3-Hₐ; dd, 13.7/3.4), 1.23 (2-CH₃; s), 1.17 (2-CH₃'; s); *unit D* 4.92 (2-H; dd, 10.3/3.2), 1.67-1.78 (3-H_{b}/4-H; bm, W_{1/2}≈45), 1.48 (3-Hₐ; ddd, 13.9/8.8/3.2), 0.89 (4-CH₃; d, 6.6), 0.86 (5-H₃; d, 6.6). ¹³C NMR (CDCl₃) δ *unit A* 165.1 (1), 142.0 (3), 137.0 (9), 128.5 (11/13), 128.5 (12), 125.3 (10/14), 124.6 (2), 76.7 (5), 63.2 (7), 56.2 (8), 40.8 (6), 36.7 (4), 13.4 (6-Me); *unit B* 170.4 (1), 154.0 (7), 130.8 (5), 129.7 (4), 128.2 (9), 122.5 (6), 112.3 (8), 56.1 (7-OMe), 54.4 (2), 35.3 (3); *unit C* 177.9 (1), 46.4 (3), 42.7 (2), 23.0 (2-Me), 22.7 (2-Me'); *unit D* 170.5 (1), 71.3 (2), 39.2 (3), 24.7 (4), 22.8 (4-Me), 21.3 (5).

### Example 4 Synthesis of Cryptophycin 55

To a solution of Cryptophycin **52** (6mg, 0.009mmol) in 0.6mL of 2:1 1,2-dimethoxyethane/water was added 2µL of 12 N HCl. The solution was allowed to stir at room temperature for 20 h, neutralized with potassium carbonate, filtered through a 5µ filter, and evaporated. The acetonitrile-soluble material was purified by reversed-phase HPLC on C18 (250 x 10mm column) using 4:1 MeOH/H₂O to obtain 3.0mg of Cryptophycin **55** (48%). [α]_{D} +42.5° (c 1.1, CHCl₃); EIMS *m*/*z* 704/706/708 (M⁺ < 1), 668/670 (1.5/0.5, M⁺-HCl), 445 (6), 226 (8), 195/197 (16/5), 184 (10), 155/157 (33/11), 135 (100), 91 (99), 77 (30); HREIMS *m*/*z* 668.2873 (M⁺-HCl, C₃₆H₄₅N₂O₈ ³⁵Cl, Δ-0.8mmu); UV (MeOH) λₘₐₓ (ε) 204 (48400), 218 (29200), 284 (1600) nm; IR (NaCl) νₘₐₓ 3410, 3286, 2959, 1748, 1723, 1666, 1538, 1504, 1455, 1257, 1178, 1066, 753 cm⁻¹. ¹H NMR (CDCl₃) δ *unit A* 7.35-7.42 (10-H/11-H/12-H/13-H/14-H; m), 6.78 (3-H; ddd, 15.1/10.6/4.5), 5.78 (2-H; dd, 15.1/1.7), 5.16 (5-H; ddd, 11.1/8.3/2.1), 4.65 (8-H; d, 9.7), 4.01 (7-H; bd, 9.7), 2.69 (4-H_{b}; dddd, -14.5/4.5/2.1/1.7), 2.50 (6-H; bm, W_{1/2}*≈*15), 2.38 (4-Hₐ: ddd, -14.5/11.1/10.6), 1.53 (7-OH, s), 1.04 (6-Me, d, 7.1); *unit B* 7.21 (5-H; d, 2.2), 7.07 (9-H; dd, 8.5/2.2), 6.85 (8-H; d, 8.5), 5.57 (2-MH; d, 7.8), 4.74 (2-H; ddd, 7.8/7.6/5.2), 3.88 (7-OCH₃; s), 3.13 (3-H_{b}; dd, 14.5/5.2), 3.05 (3-Hₐ; dd, 14.5/7.6); *unit C* 7.21 (3-NH; m), 3.38 (3-H_{b}; dd, 13.5/8.3), 3.17 (3-Hₐ; dd, 13.5/4.1), 1.23 (2-CH₃; s), 1.17 (2-CH₃'; s), *unit D* 4.93 (2-H; dd, 10.1/3.5), 1.78 (3-H_{b}; ddd, 13.5/10.1/5.0), 1.72 (4-H; bm, W_{1/2}≈20), 1.43 (3-Hₐ; ddd, 13.5/8.8/3.5), 0.92 (4-CH₃; d, 6.6), 0.92 (5-H₃, d, 6.4). ¹³C NMR (CDCl₃) *δ unit A* 165.1 (C-1), 142.4 (C-3), 138.4 (C-9), 129.0 (C-11/13), 128.3 (C-12), 128.0 (C-10/14), 124.6 (C-2), 76.1 (C-5), 74.1 (C-7), 62.0 (C-8), 38.4 (C-6), 36.5 (C-4), 8.6 (6-Me); *unit B* 170.3 (C-1), 154.1 (C-7), 130.9 (C-5), 129.6 (C-4), 129.2 (C-9), 122.6 (C-6), 112.3 (C-8), 56.1 (7-OMe), 54.3 (C-2), 35.3 (C-3); *unit C* 177.8 (C-1), 46.5 (C-3), 42.8 (C-2), 22.9 (2-Me), 23.0 (C-2-Me'); *unit D* 170.3 (C-1), 71.3 (C-2), 39.7 (C-3), 24.8 (C-4), 22.7 (4-Me), 21.6 (C-5). The corresponding diol, Cryptophycin **56** (2.8mg, 44% yield), was also obtained.

### Example 5 Synthesis of Cryptophycin 57

A small amount of PtO₂ (≈ 1mg) was added to a flask containing 0.5mL of CH₂Cl₂. The air in the flask was evacuated, H₂ was introduced, and the mixture was stirred at room temperature for 20 minutes. A solution containing 10.2mg of Cryptophycin **52** (0.015mmol) in CH₂Cl₂ (0.3mL) was added and the mixture stirred at room temperature for a further 30 minutes. The catalyst was removed by filtration through Celite/cotton and the solvent was removed *in vacuo*. Reverse phase HPLC of the residue (ODS, 10µ, 250 x 22.5mm, MeCN/H₂O (3:1), 5mL min⁻¹) yielded pure Cryptophycin **57** (9.1mg, 89%). [α]_{D} +3.4 (c=4.5, CHCl₃); EIMS *m*/*z* 670/672 (M⁺, 9/3), 447 (10), 246 (63), 229 (20), 195/197 (78/25), 184 (58), 155/157 (39/13), 128 (21), 91 (100), 77 (23); HREIMS *m*/*z* 670.3037 (C₃₆H₄₇N₂O₈ ³⁵Cl, Δ-1.6mmu); UV (MeOH) λₘₐₓ (ε) 204 (31400), 218 (12000), 284 (1200) mn; ¹H NMR (CDCl₃) δ *unit A:* 7.30-7.37 (11/12/13-H, bm), 7.23 (10/14-H, bdd, 7.9, 1.9), 5.03 (5-H, ddd, 9.0, 5.6, 3.4), 3.66 (8-H, d, 2.1), 2.89 (7-H, dd, 7.7, 2.1), 2.27 (2-H_{b}, ddd, 14.3, 8.7, 6.2), 2.04 (2-Hₐ, ddd, 14.3, 8.8, 6.8), 1.64-1.75 (b-H/4-H₂, bm), 1.61 (3-H₂, bm, W_{1/2}≈25), 1.11 (b-CH₃, d, 7.1), *unit B:* 7.19 (5-H, d, 2.1), 7.04 (9-H, dd, 8.3, 2.1), 6.83 (8-H, d, 8.3), 5.55 (2-NH, d, 8.3), 4.65 (2-H, ddd, 8.3, 7.3, 5.3), 3.87 (7-OCH₃, s), 3.16 (3-H_{b}, dd, 14.3, 7.3), 3.08 (3-Hₐ, dd, 14.3, 5.3), *unit C:* 6.91 (3-NH, dd, 6.4, 6.4), 3.41 (3-H_{b}, dd, 13.5, 6.4), 3.30 (3-Hₐ, dd, 13.5, 6.4), 1.21 (2-CH₃, s), 1.13 (2-CH₃', s), *unit D:* 4.80 (2-H, dd, 9.8, 4.1), 1.64-1.75 (3-H_{b}/4-H, bm), 1.34 (3-Hₐ, ddd, 15.4, 10.1, 4.1), 0.86 (4-CH₃, d, 6.5), 0.84 (5-H₃, d, 6.5); ¹³C NMR (CDCl₃) δ *unit A*: 172.6 (1), 136.9 (9), 128.7 (11/13), 128.5 (12). 125.6 (10/14), 76.8 (5), 63.4 (7), 59.2 (8), 40.2 (6), 36.2 (2), 32.2 (4), 21.4 (3), 13.6 (6-Mc), *unit B:* 170.4 (1), 154.0 (7), 131.1 (5), 130.0 (4), 128.5 (9), 122.5 (6), 112.2 (8), 56.1 (7-OMe), 54.3 (2), 35.3 (3), *unit C:* 177.6 (1), 47.0 (3), 43.1 (2), 22.5 (2-Me'), 22.4 (2-Me), *unit D:* 171.7 (1), 72.0 (2), 39.0 (3), 24.6 (4), 22.8 (4-Me), 21.8 (5).

### Example 6 Synthesis of Cryptophycin 58

To a stirred solution of Cryptophycin **57** (5.5mg, 0.008mmol) in 3mL of ethanol free chloroform at ≈-60°C was added TMSCl (Used as obtained from Aldrich, ≈ 4.5mg, ≈5.2µL, ≈0.04mmol). The reaction mixture was stirred for 20 minutes, by which time the indicated no starting material remained. The volatile components were then removed under reduced pressure to leave an amorphous solid. This material was taken up in acetonitrile and subject to HPLC (ODS, 10µ, 250 x 22.5mm, MeCN/H₂O (3:1), 5mL min⁻¹) to return pure Cryptophycin **58** (5.4mg, 93%) as the major product. [α]_{D} +7.2 (c=2.1, CHCl₃); EIMS *m*/*z* 706/708/710 (M⁺, 27/23/8), 670/672 (M⁺-HCl, 14/13), 583 (54), 581 (53), 485 (23), 483 (21), 447 (34), 294 (21), 282 (39), 246 (57), 195/197 (87/27), 184 (73), 155/157 (45/10), 128 (30), 91 (95), 77 (30), 69 (100); HREIMS *m*/*z* 706.2844 (C₃₆H₄₈N₂O₈³⁵Cl₂, Δ-5.6mmu), *m*/*z* 670.3670 (M⁺-HCl, C₃₆H₄₇N₂O₈ ³⁵Cl, Δ-4.9mmu); UV (MeOH) λₘₐₓ (ε) 204 (331900), 218 (11800), 284 (1800) nm; ¹H NMR (CDCl₃) δ *unit A:* 7.34-7.42 (10/11/12/13/14-H, bm), 5.01 (5-H, ddd, 9.6, 8.3, 2.5), 4.65 (8-H, d, 9.6), 4.00 (7-H, dd, 9.6, 1.9), 2.42 (6-H, ddq, 8.3, 1.9, 7.0), 2.29 (2-H_{b}, ddd, 14.3, 9.4, 4.5), 2.06 (2-Hₐ, ddd, 14.3, 8.3, 7.5), 1.62-1.82 (3-H₂/4-H₂, bm), 0.99 (6-CH₃, d, 7.0), *unit B:* 7.20 (5-H, d, 2.1), 7.06 (9-H, dd, 8.3, 2.1), 6.84 (8-H, d, 8.3), 5.62 (2-NH, d, 8.3), 4.61 (2-H, ddd, 8.3, 7.7, 5.4), 3.87 (7-OCH₃, s), 3.17 (3-H_{b}, dd, 14.3, 7.7), 3.11 (3-Hₐ, dd, 14.3, 5.4), *unit C:* 6.97 (3-NH, dd, 6.4, 6.2), 3.43 (3-H_{b}, dd, 13.4, 6.2), 3.31 (3-Hₐ, dd, 13.4, 6.4), 1.23 (2-CH₃, s), 1.16 (2-CH₃', s), *unit D:* 4.93 (2-H, dd, 10.0, 4.0), 1.86 (3-H_{b}, ddd, 14.0, 10.0, 5.5), 1.58 (3-Hₐ, ddd, 14.0, 8.3, 4.0), 0.97 (4-CH₃, d, 6.8), 0.94 (5-H₃, d, 6.6); ¹³C NMR (CDCl₃) δ *unit A:* 172.8 (1), 138.7 (9), 129.0 (12), 128.9 (11/13), 128.0 (10/14), 76.5 (5), 73.8 (7), 62.1 (8), 38.1 (6), 35.9 (2), 31.8 (4), 21.4 (3), 8.7 (6-Me), *unit B:* 170.6 (1), 153.9 (7), 131.0 (5), 130.2 (4), 128.5 (9), 122.4 (6), 112.2 (8), 56.1 (7-OMe), 54.4 (2), 35.0 (3), *unit C:* 177.2 (1), 47.0 (3), 43.2 (2), 22.5 (2-Me'), 22.4 (2-Me), *unit D:* 171.8 (1), 72.0 (2), 39.4 (3), 24.9 (4), 22.9 (4-Me), 21.7 (5).

### Example 7 Synthesis of Cryptophycin 61

To a solution of Cryptophycin **53** (5mg, 0.007mmol) in 0.5mL of dry benzene was added triphenylphosphine sulfide (4mg, 0.014mmol) followed by 0.65µL of trifluoroacetic acid as a solution in dry benzene (100µL). The solution was allowed to stir at room temperature for 6 h, neutralized with sodium bicarbonate, filtered and evaporated. The residue was partitioned between water and CH₂Cl₂. The CH₂Cl₂-soluble material was purified by reversed-phase HPLC on C18 using 4:1 MeCN/H₂O to obtain pure Cryptophycin **61** (1.9mg, 37 %). [α]_{D} +28.4 (c=0.7, CHCl₃); EIMS *m*/*z* 684/686 (M⁺, not observed), 652/654 (M⁺-S, 5/4). 426/428 (90/29), 294 (10), 227 (100), 195/197 (57/20), 184 (20), 155/157 (34/9), 131 (45), 129 (44), 91 (76), 77 (27); HREIMS *m*/*z* 652.2973 (M⁺-S, C₃₆H₄₅N₂O₇³⁵Cl, Δ-5.8mmu); UV (MeOH) λₘₐₓ (ε) 204 (26700), 218 (11600), 284 (820) nm; IR (NaCl) νₘₐₓ 3410, 3271, 2958, 1749, 1724, 1670, 1503, 1463, 1258, 1176, 1066, 758 cm⁻¹. ¹H NMR (CDCl₃) δ *unit A* 7.29-7.34 (11/12/13-H; m). 7.25 (10/14-H, bd, 6.6), 6.73 (3-H, ddd, 15.2/10.6/4.5), 5.66 (2-H; dd, 15.2/1.7), 5.22 (5-H, ddd, 11.2/4.2/2.0), 3.68 (8-H, d, 5.1), 3.01 (7-H, dd, 8.4/5.1), 2.52 (4-H_{b}, dddd, -14.4/4.5/2.0/1.7), 2.41 (4-Hₐ, ddd, -14.4/11.2/10.6), 1.68-1.74 (6-H, m), 1.14 (6-Me, d, 6.9); *unit B* 7.18 (5-H, d, 2.2), 7.04 (9-H, dd, 8.4/2.2), 6.84 (8-H, d, 8.4), 5.45 (NH, d, 7.8), 4.75 (2-H, ddd, 7.8/7.3/5.4), 3.87 (OMe, s), 3.09 (3-H_{b}, dd, -14.5/5.4), 3.05 (3-Hₐ, dd, -14.5/7.3); *unit C* 7.17 (NH, dd, 8.3, 3.9), 3.39 (3-H_{b}, dd, -13.5/8.3), 3.14 (3-Hₐ, dd, -13.5/3.9), 1.23 (2-Me, s), 1.16 (2-Me', s); *unit D* 4.86 (2-H, dd, 10.2/3.4), 1.77 (3-H_{b}, ddd, -14.0/10.2/4.9), 1.68-1.74 (4-H, m), 1.42 (3-Hₐ, ddd, -14.0/8.7/3.4), 0.92 (4-Me, d, 6.6), 0.88 (5-H₃, d, 6.4). ¹³C NMR (CDCl₃) δ *unit A* 164.9 (1), 141.7 (3), 138.3 (9), 128.8 (11/13), 128.0 (12), 126.7 (10/14), 124.7 (2), 76.6 (5), 45.8 (7), 43.9 (8), 43.9 (6), 36.6 (4), 16.0 (6-Me), *unit B* 170.2 (1), 154.1 (7), 130.9 (5), 129.4 (4), 128.3 (9), 122.8 (6), 112.4 (8), 56.1 (7-OMe), 54.2 (2), 35.3 (3), *unit C* 177.9 (1), 46.5 (3), 42.7 (2), 22.9 (2-Me), 22.8 (2-Me'), *unit D* 170.4 (1), 71.3 (2), 39.4 (3), 24.7 (4), 22.7 (4-Me), 21.4 (5).

### Example 8 Synthesis of Cryptophycin 97

To a solution of the cyclic depsipeptide, Cryptophycin **53** (9mg, 0.013mmol), dissolved in dimethyl sulfoxide (1mL), was added sodium azide (40mg) and concentrated sulfuric acid (4µL). The mixture was then allowed to stir at 75-85°C for 2 days. After this time the reaction mixture was cooled to room temperature, diluted with Et₂O (15mL) and the organic layer washed with brine (2 x 20mL) and H₂O (20mL). The ethereal extract was then dried (MgSO₄) and the solvent removed *in vacuo* to return an amorphous, colorless solid, that was predominantly the azido-alcohol, Cryptophycin **86**. Purification was achieved by reverse phase chromatography (ODS, 10µ, 250 x 10mm, 25% H₂O/MeCN, 3mL min⁻¹) to return the pure azido-alcohol, Cryptophycin **86**, as an amorphous colorless solid (7.4mg, 77%).

### Spectral Data for Cryptophycin 86

[α]_{D} -22.0 (c=3.0, CHCl₃); MS (EI) *m*/*z* 482/484 (highest observed ion, M⁺-229, 8/3), 625/627 (14/5), 259 (7), 195/197 (100/34), 184 (14), 155/157 (82/70), 91 (23), 77 (22); HRMS, obsd *m*/*z* 482.1791, C23H₃₁N₂O₇³⁵Cl (Δ 2.9mmu); MS (FAB) *m*/*z* (magic bullet matrix) 712/714 (M⁺+H, 79/36), 686/688 (31/12), 232 (74), 184 (100). ¹H NMR (CDCl₃) *δ unit A:* 7.37-7.42 (10/11/12/13/14-H, bm, W_{1/2}≈20), 6.77 (3-H, ddd, 15.2, 10.6. 4.4), 5.77 (2-H, dd, 15.2, 1.3), 5.45 (5-H, ddd, 11.0, 4.2, 2.0), 4.55 (8-H, d, 5.7), 3.75 (7-H, dd, 7.3, 5.7), 2.55 (4-H_{b}, dddd, 14.5, 4.4, 2.0, 1.3), 2.43 (4-Hₐ, ddd, 14.5, 11.0, 10.6), 2.34 (7-OH, s), 1.80 (6-H, ddq, 7.3, 4.2, 7.0), 0.99 (6-Me, d, 7.0), *unit B:* 7.20 (5-H, d, 2.2), 7.06 (9-H, dd, 8.4, 2.2), 6.84 (8-H, d, 8.4), 5.76 (NH, d, 7.7), 4.74 (2-H, ddd, 7.7, 7.5, 5.5), 3.87 (OCH₃, s), 3.10 (3-H_{b}, dd, 14.5, 5.5), 3.06 (3-Hₐ, dd, 14.5, 7.5), *unit C:* 7.22 (NH, dd, 8.4, 3.7), 3.40 (3-H_{b}, dd, 13.6, 8.4), 3.14 (3-Hₐ, dd, 13.6, 3.7), 1.23 (2-CH₃, s), 1.16 (2-CH₃, s), *unit D:* 4.85 (2-H, dd, 9.5, 5.1), 1.71 (3-H_{b}, ddd, 13.6, 9.5, 5.9), 1.59 (4-H, bm, W_{1/2}=25), 1.50 (3-Hₐ, ddd, 13.6, 7.9, 5.1), 0.89 (4-CH₃, d, 6.6), 0.85 (5-H₃, d, 6.6); ¹³C NMR (CDCl₃) *δ unit A:* 165.3 (1), 143.0 (3), 135.1 (9), 129.1 (12), 128.9 (11/13), 128.6 (10/14), 124.3 (2), 75.1 (7), 74.6 (5), 67.8 (8), 39.3 (6), 34.7 (4), 11.9 (6-Me), *unit B:* 170.5 (1), 154.1 (7), 130.9 (5), 129.7 (4), 128.3 (9), 122.5 (6), 112.4 (8), 56.1 (7-OMe), 54.3 (2), 35.3 (3), *unit C:* 177.8 (1), 46.5 (3), 42.8 (2), 22.9 (2-Me), 22.7 (2-Me'), *unit D*: 170.2 (1), 71.4 (2), 39.4 (3), 24.7 (4), 22.6 (4-Me), 21.8 (5).

### Cryptophycin 97

To a solution of the cyclic depsipeptide, Cryptophycin **86** (5.5mg, 0.008mmol), dissolved in Et₂O/CH₂Cl₂ (3:1, 0.5mL), was added an ethereal solution (0.5mL) of triphenylphosphine (3mg, 0.011mmol). The mixture was then allowed to stir at room temperature for three days. After this time the solvent was removed *in vacuo* and the residue dissolved in CH₂Cl₂ and subject to HPLC purification (CN column, 10µ, 250 x 10mm, 80% EtOAc/CH₂Cl₂, 3mL min⁻¹) to return pure Cryptophycin **97** as an amorphous colorless solid (4.2mg, 82 %).
UV (MeOH) λₘₐₓ (ε) 202 (24400), 218 (9400), 284 (2200) nm; MS (EI) *m*/*z* 667/669 (M⁺, 11/3), 639/641 (41/16), 442 (21), 226 (17), 195 (43), 196 (32), 197 (100), 198 (71), 199 (11), 182/184 (25/16), 155/157 (63/22), 146 (30), 91 (40), 77 (29); HRMS, obsd *m*/*z* 667.3061, C₃₆H₄₆N₃O₇³⁵Cl (Δ-3.6mmu). ¹H NMR (CDCl₃) δ *unit A* 7.35 (11-H/13-H; m, W_{1/2} =15 Hz), 7.28 (12-H, m), 7.16 (10-H/14-H; m, W_{1/2}=15 Hz), 6.74 (3-H; ddd, 15.2/9.0/6.1), 5.69 (2-H; d, 15.2), 5.21 (5-H; ddd, 9.2/4.3/4.2), 2.19 (8-H, bs), 2.51 (4-H₂, m), 2.11 (7-H, bd, 6.5), 1.48 (6-H, m), 1.13 (6-Me, d, 6.9); *unit B:* 7.18 (5-H, d, 2.1), 7.04 (9-H, dd, 8.4, 2.1), 6.83 (8-H, d, 8.4), 5.53 (NH, m), 4.73 (2-H, ddd, 7.6, 5.6, 5.4), 3.87 (OMe, s), 3.09 (3-H_{b}, dd, 14.7, 5.4), 3.04 (3-Hₐ, dd, 14.7, 7.6), *unit C:* 7.20 (NH, m), 3.40 (3-H_{b}, dd, 13.5, 8.6), 3.11 (3-Hₐ, dd, 13.5, 3.3), 1.22 (2-Me, s), 1.15 (2-Me, s), *unit D:* 4.84 (2-H, dd, 10.1, 3.7), 1.71 (3-H_{b}, m), 1.67 (4-H, bm, W_{1/2}≈25), 1.35 (3-Hₐ, m), 0.86 (4-Me, d, 6.7), 0.84 (5-H₃, d, 6.5); ¹³C NMR (CDCl₃) *δ unit A:* 165.0 (1), 142.1 (3), 139.2 (9), 128.8 (11/13), 127.5 (12), 125.4 (10/14), 124.6 (2), 76.6 (5), 42.5 (6), 42.1 (7), 40.5 (8), 36.6 (4), 14.8 (6-Me), *unit B:* 170.2 (1), 154.1 (7), 130.9 (5), 129.5 (4), 128.2 (9), 122.6 (6), 112.4 (8), 56.1 (7-OMe), 54.3 (2), 35.3 (3), *unit C:* 177.9 (1), 46.5 (3), 42.7 (2), 22.8 (2-Me/2-Me'), *unit D:* 170.5 (1), 71.3 (2), 39.5 (3), 24.6 (4), 22.7 (4-Me), 21.3 (5).

### Comparative Example 9 Synthesis of Cryptophycins 121-123 and 127

### Compound AJ

To a solution of BOC-*L*-leucine monohydrate (1.245g, 5mmol) in 30mL anhydrous CH₂Cl₂ was added solid EDC (1-(3-dimethylaminopropyl)-3-ethylcarbodiimide hydrochloride)(0.53g, 2.75mmol) under N₂ with stirring at 0°C. After stirring at 0°C for 1.5h the mixture was concentrated below 5°C and the residue was diluted with 35mL cold EtOAc and washed successively with two portions (10mL) each of ice-cold solutions of 5% aqu. KHSO₄, 5% aqu. NaHCO₃ and brine. The organic phase was separated, dried (MgSO₄) at 5°C and evaporated below 5°C. The residual oil was diluted with ice-cold THF (5mL) and added to a solution of compound **K** (295mg, 0.5mmol) in ice-cold anhydrous THF (5mL). A few crystals of DMAP were then added to the mixture which was stirred and allowed to reach 25°C overnight. 5% aqu. NaHCO₃ solution (5mL) was added and the resulting two-phase mixture was stirred vigorously at 25°C for 2h. EtOAc (40mL) was added, the aqueous phase was extracted with additional EtOAc (2x 20mL) and the combined organic layers were washed with water and brine, dried (NaSO₄), filtered and evaporated. The residue was filtered through a plug of silica gel with 25% EtOAC in hexanes to yield compound AJ as a colourless oil (385mg, 96% yield): [α]_{D} 13.6° (c= 0.63, CHCCl₃); EIMS *m*/*z* (rel. intensity) 805/803/801(M+, 1%), 568/570/572/574 (9/10/6/2), 523/525/527 (5/6/1), 418/420/422/424 (15/15/6/2), 341/343/345/347 (58/70/35/9), 307/309/311 (29/22/10), 224/227/228/229 (10/100/31/14), 208/210/211/2'12 (20/83/45/54); HREIMS *m*/*z* 800.2218 (C₃₅H₄₈ ³⁵Cl₄N₂O₈, Δ-5.3mmu); ¹H NMR (CDCl₃) *unit A* δ 7.21-7.35 (Ph-H₅; m), 6.77 (3-H; ddd, 6.7/6.7/15.3), 6.4 (8-H; d, 15.8), 6.05 (7-H; dd, 8.7/15.8), 5.9 (2-H; d, 15.3), 5.0 (5-H; m), 2.6 (6-H; m), 2.55 (4-H₂; m), 1.17(6-Me; d, 6.7); *unit B δ* 7.22 (5-H; s), 7.05 (9-H: d, 8.1), 6.84 (8-H; d, 8.1), 6.55 (NH; d, 7.8), 5.0 (2-H; ddd, 5.5/7.1/7.8), 4.68-4.8 (CH₂CCl₃; ABq, 11.9), 3.86 (OMe; s), 3.2 (3-H; dd, 5.5/14.0), 3.06 (3-H': dd. 7.1/14.0); *unit D δ* 4.8 (NH; d), 4.2 (2-H; m), 1.65 (4-H; m), 1.55 (3-H; m), 1.4 (CMe₃); s). 1.37 (3-H'; m), 0.85 (4-Me; d, 6.5), 0.8 (5-H; d, 6.5); ¹³C NMR *unit A δ* 165.4(1), 139.3 (3), 137.0 (9), 131.5 (8), 130.5 (7), 128.5 (11/13), 127.3 (12), 126.2 (10/12), 125.8 (2), 76.2 (5), 40.8 (6), 33.4 4), 16.8 (6-Me); *unit B δ* 170.0 (1), 154.2 (7), 131.0 (5), 129.0. (9), 122.5 (6), 112.2 (8), 94.4 (CCl₃), 74.7 (CH₂CCl₃), 56.1 (OMe), 53.3 (2), 36.5 (3), *unit D δ* 173.2 (1), 155.7 (BOC-CO), 80.0 (CMe₃), 52.4 (2), 41.2 (3), 28.3 (CMe₃), 24.8 (4), 22.8 (4-Me), 21.6 (5).

### Compound AK

Compound AJ (115mg, 0.134mmol) was dissolved in TFA (3mL) and left at 25°C for 1 h. The solvent was removed and the residue was evaporated repeatedly from CH₂Cl₂ then from toluene to leave an amorphous solid. This solid was dissolved in anhydrous THF (5mL) and sufficient diethylisopropylamine was added to adjust the pH of the solution to pH 8-9 when an aliquot of the solution was spotted onto moist pH paper. The solution was cooled to 0°C with stirring and a solution of compound AL in THF (3mL) was added. To prepare compound AL compound Z (55mg, 0.27mmol) was dissolved in THF (3mL) and diethylisopropylamine (0.046mL, 0.27mmol) was added. To this mixture, after cooling to -15°C, pivaloylchloride (0.033mL, 0.27mmol) was added dropwise and the solution was stirred at -15 °C for 10 min and at 0°C for 20 min. The resulting suspension was then transferred into the solution of compound AJ in THF. The resulting mixture was stirred at 0°C and allowed to reach 25°C overnight. After 12 hrs at 25°C 5% aqu. NaHCO₃ solution was added and the resulting mixture was stirred vigorously at 25°C for 1.5h. EtOAC (40mL) was added and the phases were separated. The aqueous phase was extracted with additional EtOAC (2x 10mL). The combined organic extracts were washed with 5 % aqu. NaHCO₃ solution (20mL), 5% aqu. KHSO₄ solution (20mL) and brine, dried (NaSO₄) and evaporated. The residual oil was chromatographed over silica gel eluting with 35% EtOAc in hexanes to yield compound AK as a colourless foam (98mg, 83% yield): [α]_{D} -8.3 (c 0.88, CHCl3); ¹H NMR (CDCl₃) *unit A* δ 7.28-7.35 (Ph-H₄; m), 7.22(H-12;m), 6.75 (H-3; ddd, 6.4/6.4/15.4), 6.4(H-8; d, 15.9), 6.04 (H-7; dd, 8.6/15.9), 5.95 (H-2; d, 15.4), 5.0 (H-5; m), 2.6 (H-4; m), 1.1 (6-Me; d, 6.8); *unit B δ* 7.22 (H-5, d, 1.5), 7.15 (NH; d, 7.6), 7.05 (H-9; dd, 1.5/8.2), 6.85 (H-8; d, 8.2), 5.0 (H-2; m), 4.8-4.68 (CH₂CCl₃; ABq, 12), 3.86 (OMe; s), 3.2 (H-3; m), 3.1 (H-3'; dd, 7.2/14.1); *unit C* δ 5.0 (NH; m), 3.2 (H2-3; m), 2.55 (H-2; m), 1.1 (2-Me; d, 7.1); *unit D* δ 6.12 (NH; m), 4.4 (H-2; m), 1.65 (H-4; m), 1.55 (H-3; m), 1.4 (H-3'; m), 0.86 (4-Me; d, 6.8), 0.81 (5-H; d, 6.8); ¹³C NMR (CDCl₃) *unit A* δ 165.8(1), 138.9(3), 131.5(8), 130.4(7), 128.6(11/13), 127.4(12), 126.2(10/14), 125.8 (2), 76.3(5), 33.6(4), 16.6(6-Me); *unit B* δ 170.2(1), 154.1(7), 136.9(4), 131.2(5), 128.6(9), 122.3(6), 112.2(8), 94.4(CCl3), 74.5(CH₂CCl₃), 56.1(OMe), 53.4(2), 36.6(3); *unit C δ* 175.4(1), 156.3(BOC-CO), 79.5(OCMe₃), 43.6(3), 41.3(2), 15.1(2-Me); *unit D δ* 172.6(1), 51.3(2), 40.5(3), 24.5(4), 22.7(4-Me), 21.5(5).

### Cryptophycin 121

Compound AK (73mg, 0.082mmol) was dissolved in AcOH (3.5mL), activated Zn dust (400mg) was added and the resulting suspension was sonicated for 45 min. After stirring at 25°C for an additional 1.5h the mixture was diluted with CH₂Cl₂ (5mL) and filtered through Celite^{R}. The solids were washed with additional CH₂Cl₂ (10mL) and the resulting filtrate was evaporated. The residue, without further purification, was dissolved in TFA (3mL), kept at 25°C for 1 h and the solvent was removed *in vacuo*. The residue was evaporated repeatedly from CH₂Cl₂, then from toluene until a colourless solid was obtained. This solid was dissolved in dry DMF (3mL), FDPP (43mg, 0.108mmol) was added followed by a sufficient amount of diethylisopropylamine to adjust the pH of the solution to pH 8-9 when an aliquot was spotted onto moist pH paper. After stirring at 25°C for 16 hrs, the mixture was diluted with ether (40mL) and washed with 5% aqu. KHSO₄ solution (2 x 1mL), 5% aqu. NaHCO₃ (15mL) and brine. After drying (Na₂SO₄) and evaporation the residue was purified by reversed-phase HPLC on C-18 silica (Econosil^{R} C-18, 22x 250mm) eluting with CH₃CN/water 65:35 at 6mL/min. The fraction eluting at t_{R} 48 min was collected and evaporated to leave an amorphous solid (26mg, 50% yield): [α]_{D} +46.5° (c 0.81, CHCl₃); EIMS *m*/*z* 637/639 (M⁺, 1%), 449/451 (2/1), 420 (5), 411/413 (7/5), 227/228 (9/7), 195 (10), 184 (15), 167/168/169 (40/86/29), 155/157 (100/26), 91 (85), 69 (86); HREIMS *m*/*z* 637.2864 (C₃₅ H₄₄ ³⁵Cl N₃ O₆, Δ + 5.5mmu); ¹H NMR *unit A δ* 7.21- 7.35 (Ph-H₅; m), 6.75 (H-3; ddd, 4.2/10.8/16), 6.4 (H-8; d, 16), 6.04 (H-7; dd, 8.8/16), 5.75 (H-2; d, 16), 5.1 ((H-5; m), 2.55 (H-4; m; H-6; m), 2.35 (H-4'; m); *unit B δ* 7.18 (H-5; d, 2), 7.05 (H-9; dd, 2.0/8.3), 6.85 (H-8; d, 8.3), 5.7 (NH; d, 7.2), 4.7 (H-2; ddd, 4.9/7.2/7.7), 3.86 (OMe; s), 3.1 (H-3; dd, 4.9/14.4), 3.0 (H-3'; dd,7.7/ 14.4); *unit C* δ 7.25 (NH; m), 3.5 (H-3; m), 3.4 (H-3'; m), 2.55 (H-2; m), 1.2 (2-Me; d, 7.2); *unit D* δ 5.8 (NH; d, 7.2), 4.4 (H-2; m), 1.55 (H-4; m), 1.38 (H₂-3; dd, 7.2/7.7), 0.76 (4-Me; d, 6.6), 0.74 (H-5; d, 6.6): ¹³ C NMR (CDCl₃) *unit A δ* 165.1 (1), 141.9 (3), 136.8 (9), 131.7 (8), 130.2 (7), 128.5 (11/13), 127.3 (14), 126.1 (10/12), 125.1 (2), 76.5 (5), 42.3 (6), 36.3 (4), 17.2 (6-Me); *unit B* δ 171.0 (1), 154.1 (7), 130.8(5), 129.6 (4), 128.4 (9), (122.5(6), 112.4 (8), 56.2 (OMe), 54.4 (2), 35.4 (3); *unit C* δ 175.8 (1), 41.0 (3), 38.6 (2), 14.8 (2-Me); *unit D* δ 173.2 (1), 51.1 (2), 40.9 (3), 24.7 (4), 23.4 (4-Me), 21.5 (5)

### Example 10 Structure-Activity Relationships (SAR) and In Vivo Evaluation

The cytotoxicities of Cryptophycins **1, 3** and **8** and the new cryptophycins against the human tumor cell lines KB and LoVo are shown in Table 4. Cryptophycin **51** and cryptophycin **3** show comparable cytotoxicities (IC₅₀'s 3.5-5.8 nM). Cryptophycin **52** (IC₅₀'s 43-70pM), however, is slightly less cytotoxic than Cryptophycin **1** (IC₅₀'s 9-29pM). and Cryptophycin **55** (33-47pM) is slightly less cytotoxic than Cryptophycin **8** (IC₅₀'s 9-19pM). The cytotoxicity IC₅₀'s for Cryptophycins **117, 122, 125, 127, 128** and **132** are comparable with those for Cryptophycins **1, 8, 52,** and **55;** however, the data does not presently admit to more meaningful comparisons.

Cryptophycin **52** is active *in vivo*, but requires roughly three times the total dosage compared with Cryptophycin **1**. The *in vivo* activity of Cryptophycin **52** against five solid tumors of murine origin and three human solid tumors is summarized in Table 7. Similarly Cryptophycin **55** was active *in vivo*, but also required three times the total dosage compared with Cryptophycin **8**. The *in vivo* activity of Cryptophycin **55** against six solid tumors of murine origin and four human solid tumors is summarized in Table 8. The *in vivo* data appears to correlate with the *in vitro* data.

Cryptophycins **117, 125, 128** and **132** are active *in vivo* against a pancreatic adenocarcinoma of murine origin (Panc 03). Cryptophycins **117** and **128** appear to be more potent, that is they require smaller total doses, than Cryptophycins **1** and **8,** respectively, whereas Cryptophycins **125** and **132** are less potent, that is they require higher total doses, than Cryptophycins **1** and **8,** respectively. The data is summarized in Table 7.

T/C values that are less than 42 % are considered to be active by NCI. standards; T/C values that are less than 10% are considered to have excellent activity and potential clinical activity by NCI standards. Gross log kill is defined as T-C/3.2 Td where T is the median time in days for the tumors of the treated group to reach 750mg, C is the median time in days for the tumors of the control group to reach 750mg, and Td is the tumor volume doubling time (T.H. Corbett *et al*., Cytotoxic Anticancer Drugs: Models and Concepts for Drug Discovery and Development, pp 35-87; Kluwer: Norwell, 1992). Gross log kill values of >2.8, 2.0-2.8, 1.3-1.9, 0.7-1.2, and <0.7 with duration of drug treatment of 5-20 days are scored ++++, +++, ++, + and - (inactive), respectively. An activity rating of +++ to ++++, which is indicative of clinical activity, is needed to effect partial or complete regression of 100-300mg size masses of most transplanted solid tumors of mice. Cryptophycin **52** shows T/C values ranging from 0 to 14% for murine tumors and 4.1 to 16 for human tumors and gross log kill values ranging from 1.1 to 2 for murine tumors and 0 to 3.2 for human tumors. Cryptophycin **1** shows T/C values ranging from 0 to 27% and log kill values ranging from < 1 to 2. Cryptophycin **55** shows T/C values ranging from mostly 0 to 4.4% and log kill values ranging from 2.1 to >4.6 (cures) for all but one trial, the Colon 26 experiment which showed a gross log kill value of 1.2. Cryptophycin **8** shows T/C values of mostly 0% and log kill values >2.8 (several cures) as shown in Table 10. Cryptophycins **117** and **125** appear to have T/C and gross log kill values that are comparable with those of other epoxide-type cryptophycins, viz Cryptophycins **1** and **52,** whereas Cryptophycins **128** and **132** have T/C and gross log kill values that are comparable with those of other chlorophycin-type cryptophycins, viz Cryptophycins **8** and **55.**

**Table 1**

| *In Vivo Activity of Cryptophycin **1*** | | | | | | | |
|---|---|---|---|---|---|---|---|
| *Exp #* | *SC Tumor* | *# of Inj. IV* | *mg*/*kg Total Dose* | *% Body Wt. Loss at Nadir* | *T*/*C* | *Log Kill* | *Cures* |
| *1560* | *Colon 38* | *8* | *10.3* | *Gain* | *6%* | *1.5* | *0*/*5* |
| *1694* | *Panc 03* | *8* | *16.0* | *Gain* | *0%* | **2.0** | *0*/*5* |
| *1636* | *Colon 51* | *7* | *28.1* | *-11%* | *7%* | *1.3* | *0*/*5* |
| *1720* | *Mam 16*/*C* | *5* | *13.2* | *-1%* | *5%* | *1.4* | *0*/*5* |
| *1733* | *Mam 16*/*Taxol* | *5* | *16.5* | *0%* | *2%* | *1.8* | *0*/*5* |
| *1833* | *M17*/*0* (Adr. *Sens.)* | *5* | *5*.*4* | *-10%* | *23%* | *<1* | *0*/*5* |
| *1749* | *Panc 02* | *5* | *11.0* | *-5%* | *20%* | *1.1* | *0*/*5* |
| *1596* | *Human Sm Cell L. DMS273 SCID* | *6* | *7.3* | *0%* | *27%* | *<1* | *0*/*5* |
| *1806* | *MX-1 Human Breast* | *8* | *12* | *-3%* | *3%* | *2.0* | *0*/*5* |
| *1823* | *H125 Human Adenosq-lung* | *8* | *14.4* | *-15*% *1*/*5 dead* | *9%* | *1.1* | *0*/*5* |
| *1841* | *LNCaP Human Prostate* | *6* | *6.5* | *-6%* | *26%* | *<1* | *0*/*5* |

**Table 2**

| *In Vivo Activity of Cryptophycin Analogs* | | | | | | | | |
|---|---|---|---|---|---|---|---|---|
| *Exp #* | *Agent* | *SC Tumor* | *# of Inj*. *IV* | *mg*/*kg TD* | *% Body Wt*. *Loss at Nadir* | *T*/*C* | *Log Kill* | *Cures* |
| *1813* | *Cryptophycin-2* | *P03* | *10* | *37* | *-2%* | *44%* | *<1* | *0*/*5* |
| *1843* | *Cryptophycin-3* | *P03* | *4* | *28*/*5* | *-9%* | *54%* | *<1* | *0*/*5* |
| *1769* | *Cryptophycin-5* | *C38* | *15* | *45* | *-2%* | *>100%* | *None* | *0*/*5* |
| *1825* | *Cryptophycin-8* | *P03* | *11* | *106* | *-6%* | *4%* | *4.6* | *0*/*5* |
| *1885* | *Cryptophycin-8* | *Mam 16*/*C* | *7* | *21.3* | *-4.5%* | *6%* | *2.5* | *0*/*5* |
| *1887 B* | *Cryptophycin-8* | *C38* | *6* | *30* | *-2%* | *0%* | *2.8* | *1*/*5* |
| *1900* | *Cryptophycin-8* | *Colon 51* | *9* | *67.5* | *-1%* | *7%* | *1.8* | *0*/*5* |
| *1843* | *Cryptophycin-15* | *P03* | *5* | *18* | *-7%* | *83%* | *None* | *0*/*5* |
| *1878* | *Cryptophycin-16* | *P03* | *9* | *82* | *-1%* | *89%* | *None* | *0*/*5* |
| *1813* | *Cryptophycin-21* | *P03* | *9* | *27* | *-11%* *(1*/*5 dead)* | *61%* | *None* | *0*/*5* |
| *1843* | *Cryptophycin-35* | *P03* | *7* | *23* | *-2%* | *11%* | *1.3* | *0*/*5* |

**Table 3.**

| Cytotoxicities of antimitotic agents for SKOV3 and SKVLB1 cells | | | |
|---|---|---|---|
| Cells were treated with varying concentrations of the compounds indicated below for 48 hours. Cell numbers were then determined as indicated in the Methods section and the IC₅₀ for each compound was calculated. Values represent the mean ± SEM for three experiments. | | | |

| | Cell Line | | |
|---|---|---|---|
| Compound | SKOV3 | SKVLB1 | Resistance Factor |
| | IC50 (nM) | | |
| Viiiblastine | 0.35 ± 0.25 | 4200 ± 1700 | 12,000 |
| Taxol | 1 ± 0.4 | 8000 ± 2000 | 8,000 |
| Cryptophycias | 0.007 ± 0.002 | 0.60 ± 0.19 | 86 |

**Table 4**

| ***In Vitro* Cytotoxicity Data of Cryptophycin** | | |
|---|---|---|
| **Cryptophycin** | **RB IC**_{**50**} **nM** | **LoVo IC**_{**50**} **nM** |
| **1** | **0.0092** | **0.0104** |
| **3** | **3.1-4.6** | **1.9-5.8** |
| **8** | **0.019** | **0.0091** |
| **51** | **3.5** | **5.2** |
| **52** | **0.043** | **0.070** |
| **53** | **4.2** | **5.5** |
| **55** | **0.033** | **0.047** |
| **57** | **0.064** | **0.27** |
| **58** | **0.048** | **0.20** |
| **61** | **21** | **15** |
| **81** | **5.5** | **5.7** |
| **82** | **310** | **45** |
| **90** | **0.34** | **0.30** |
| **91** | **30** | **34** |
| **97** | **3.6** | **5.7** |
| **110** | **4.6** | **5.3** |
| **111** | **4.8** | **3.2** |
| **112** | **5.4** | **6.2** |
| **115** | **0.039** | **0.048** |
| **117** | **0.036** | **0.031** |
| **118** | **2.3** | **1.2** |
| **119** | **4.4** | **6.1** |
| **120** | **4.6** | **6.4** |
| **122** | **0.021** | **6.9** |
| **125** | **0.05** | **0.006** |
| **127** | **0.176** | **0.075** |
| **128** | **0.023** | **2.5** |
| **132** | **0.082** | **0.037** |

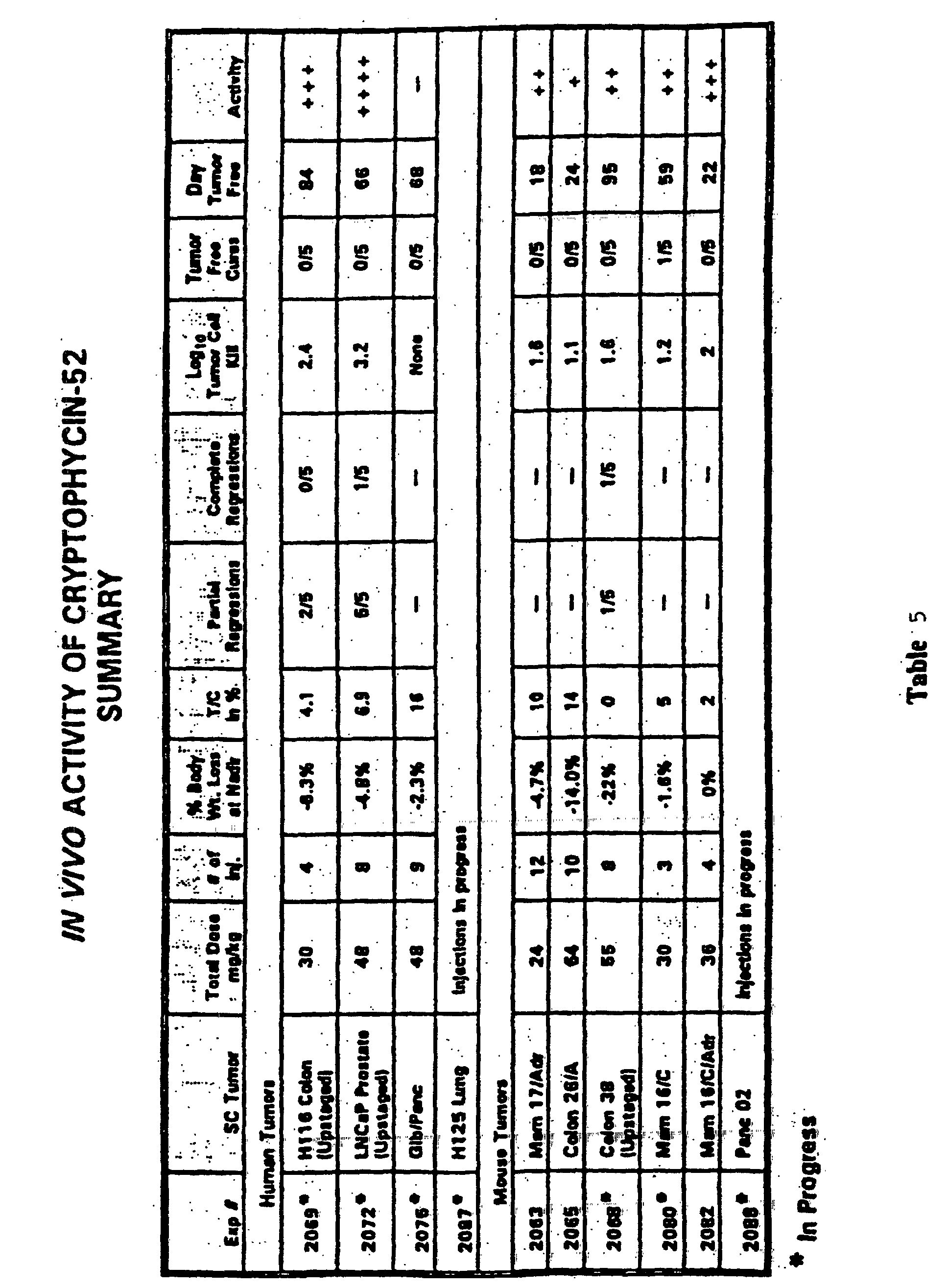

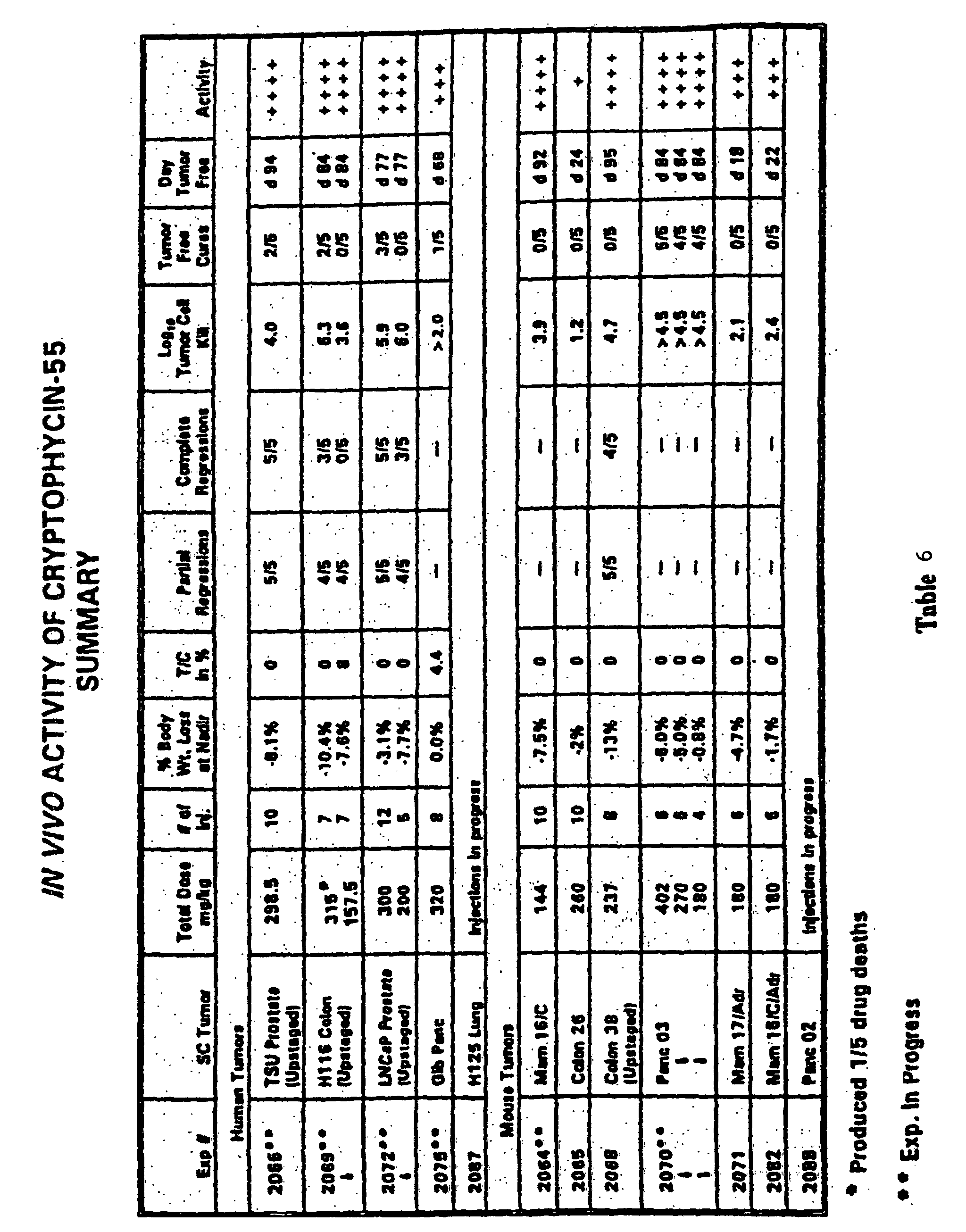

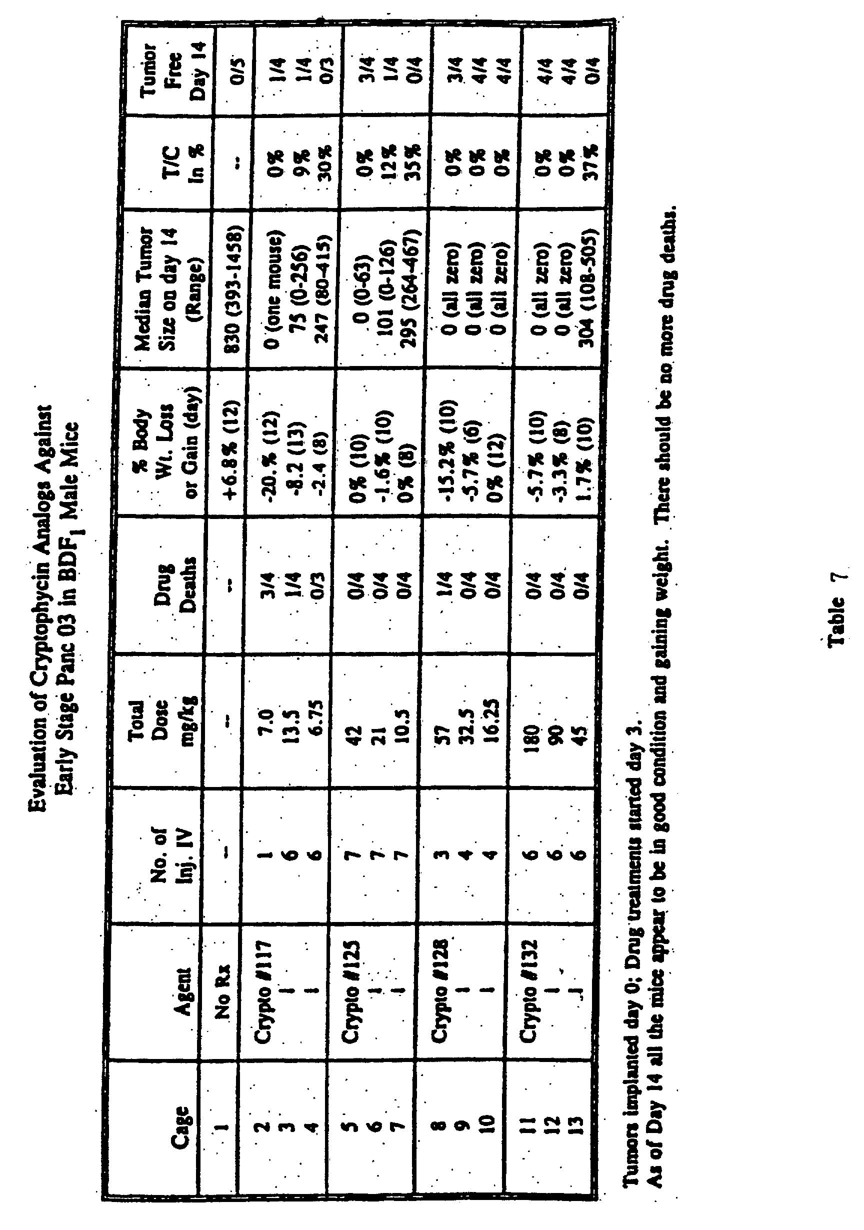

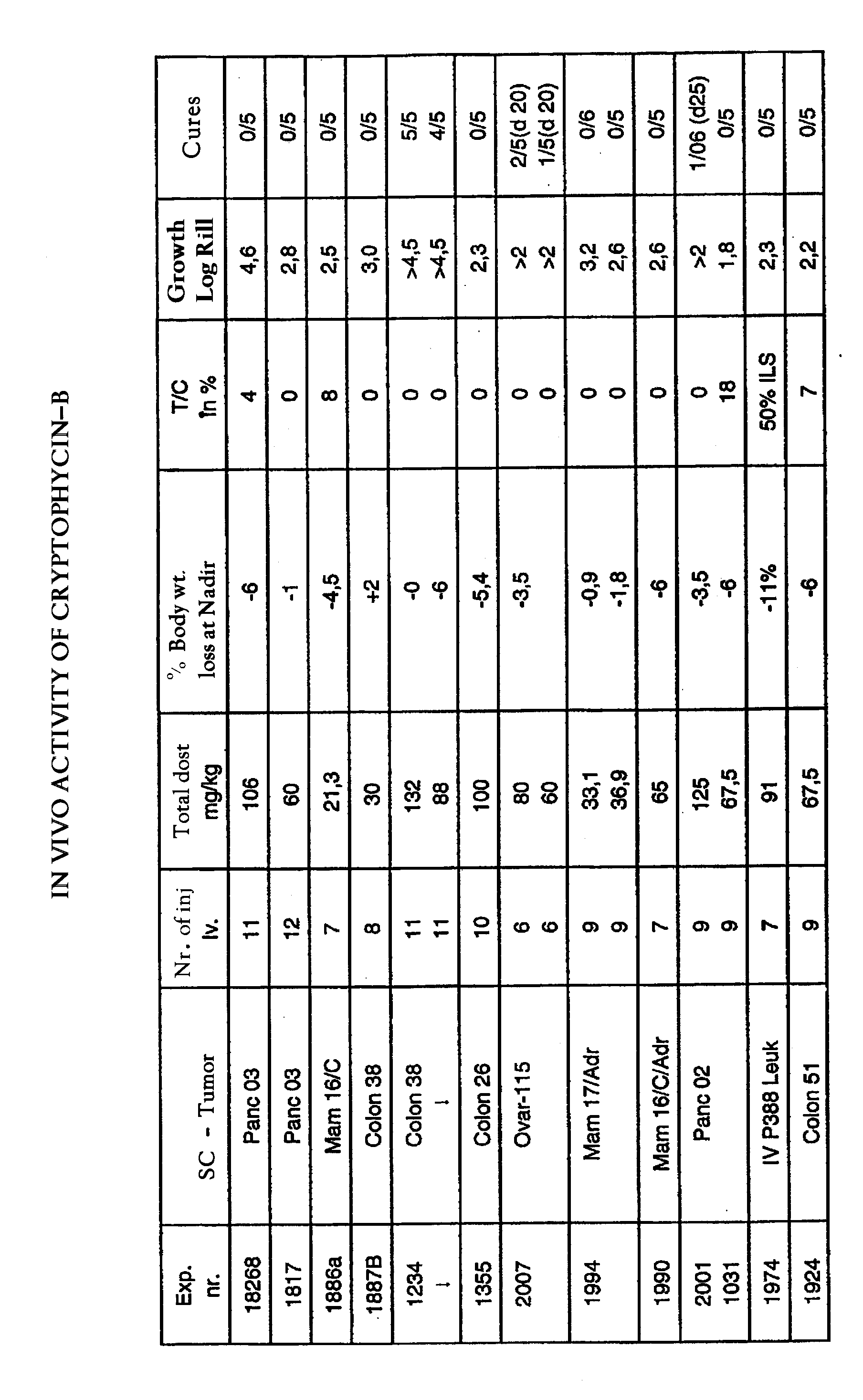

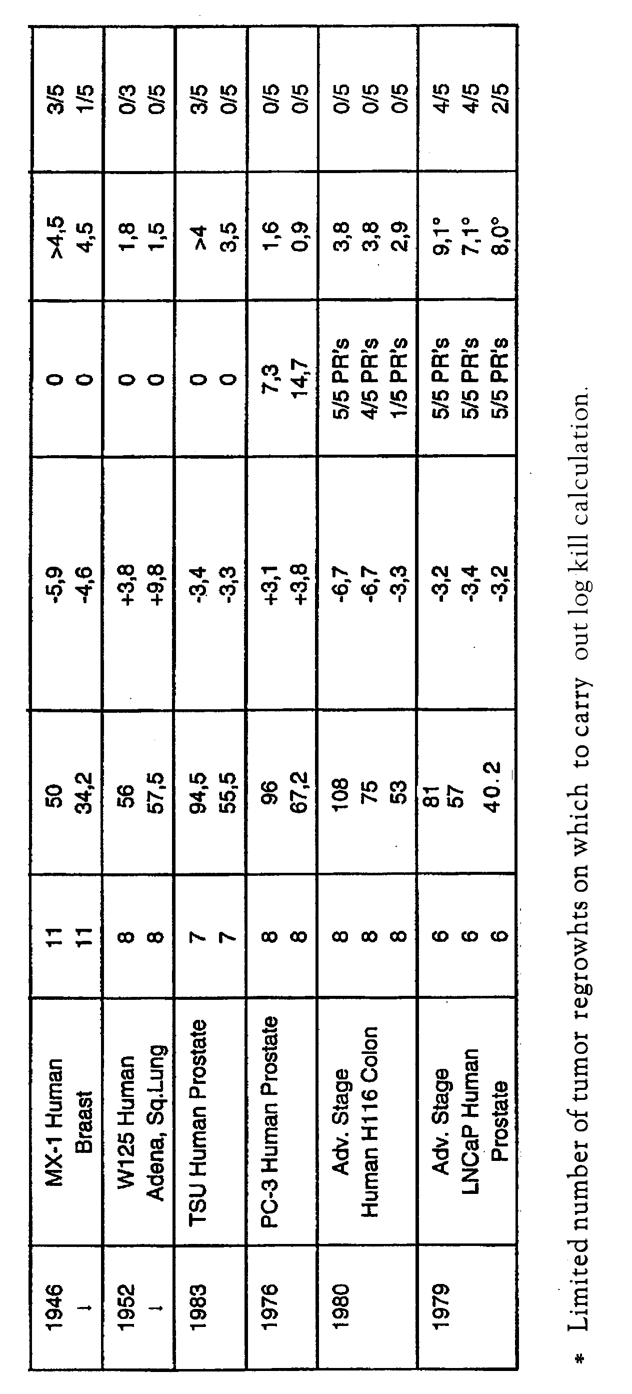

.

### References

1. Eglof, G., Organic Chemistry; An Advanced Treatise, Gilmar *et al*. (ed.), pp. 31-46, John Wiley & Sons (1943).
2. Kemp, *et al*., Organic Chemistry, Worth Publishers, Inc. (1980).
3. Patterson, G. M. L. *et al*., *J. Phycol*. **27**:530-536 (1991).
4. Corbett, T.H. *et al*., Cytotoxic Anticancer Drugs; Models and Concepts for Drug Discovery and Development, pp 35-87, Kluwer Academic Publishers: Norwell, 1992.
5. Valeriote, F.A. *et al*., Discovery and Development of Anticancer Agents, Kluwer Academic Publishers: Norwell, 1993; in press.
6. Schwartz, R.E. *et al*., *J. Ind. Microbiol*. **5**:113-124 (1990).
7. Hirsch, C.F. *et al.,* U.S. Patent 4,946,835, issued August 7, 1990.
8. Sesin, D.F., U.S. Patent 4,845,085, issued July 4, 1989.
9. Sesin, D.F. and Liesch, J.M., U.S. Patent 4,868,208, issued September 19, 1989.
10. Sesin, D.F., U.S. Patent 4,845,086, issued July 4, 1989.
11. Skehan, P. *et al*., *J*. *Natl*. *Cancer Inst*. **82**:1107-1112 (1990).
12. Bradley, G. *et al*., *Cancer Res*. **49**:2790-2796 (1989).
13. Endicott, J.A. *et al*., *Ann. Rev*. *Biochem*. **58**:137-171 (1989).
14. Beck, W.T., *Biochem*. *Pharm*. **36**:2879-2887 (1987).
15. Moscow, J.A. *et al*., *J*. *Natl*. *Cancer Inst*. **80**:14-20 (1988).
16. Trimurtulu, G. *et al*., *J*. *Am. Chem. Soc*. **116**:4729-4737 (1994).
17. Smith. C.D. *et al*., *Cancer Res*. **54**:3779-3784 (1994).

## Claims

1. A cryptophycin represented by the structure: 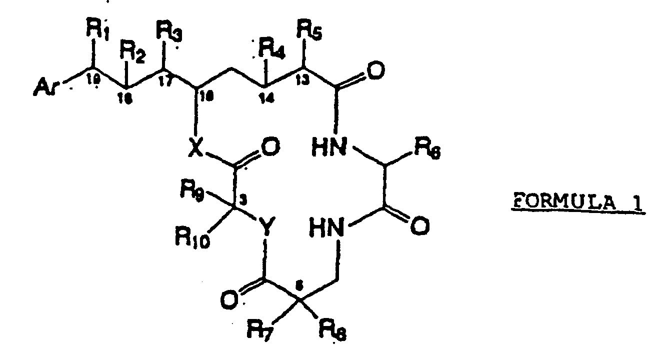 wherein
Ar is methyl or phenyl or an aromatic group having 4n+2 pi electrons in a monocyclic or bicyclic conjugated system or
a phenyl group substituted with a single group or a heteroaromatic group;
R₁ is a halogen, SH, amino, monoalkylamino, dialkylamino, trialkylammonium, alkylthio, dialkylsulfonium, sulfate or phosphate;
R₂ is OH or SH; or
R₁ and R₂ may be taken together to form an. epoxide ring, an aziridine ring, an episulfide ring, a sulfate ring or a monoalkylphosphate ring; or
R₁ and R₂ may be taken together to form a double bond between C₁₈ and C₁₉;
R₃, R₇ and R₈ are each alkyl.groups of one to five carbon atoms including linear and non.linear hydrocarbon chains;
R₄ and R₅ are H; or
R₄ and R₅ may be taken together to form a double bond between C₁₃ and C₁₄;
R6 is a benzyl, hydroxybenzyl, alkoxybenzyl, halohydraxybenzyl, dihalohydroxybenzyl, haloalkoxybenzyl, or dihaloalkoxybenzyl group;
R₉ and R₁₀ are each independently H or an alkyl group of one to five carbon atoms including linear and non linear hydrocarbon chains; and
X and Y are each independently O, NH or alkylamino.

2. The cryptophycin of claim 1, wherein R₈ is ethyl, propyl, isopropyl, butyl, isobutyl, pentyl or isopentyl.

3. The cryptophycin of claim 1, wherein R₇ is ethyl, propyl, isopropyl, butyl, isobutyl, pentyl or isopentyl.

4. The cryptophycin of claim 1, wherein R₃ is ethyl, propyl, isopropyl, butyl, isobutyl, pentyl or isopentyl.

5. The cryptophycin of claim 1, wherein R₉ is methyl, ethyl, propyl, butyl, isobutyl, pentyl or isopentyl.

6. The cryptophycin of claim 1, wherein R₁₀ is methyl, ethyl, propyl, butyl, isobutyl, pentyl or isopentyl.

7. The compound of claim 1, wherein at least one of the groups attached to C₃, C₆, C₁₀, C₁₆, C₁₇ and C₁₈ have R stereochemistry.

8. The compound of claim 1, wherein at least one of the groups attached to C₃, C₆, C₁₀, C₁₆, C₁₇ and C₁₈ have *S* stereochemistry.

9. The compound of claim 1, wherein Ar is phenyl, R₁ and R₂ are taken together to form a double bond between C₁₈ and C₁₉, R₃, R₇ and R₈ are methyl, R₄ and R₅ are taken together to form a double bond between C₁₃ and C₁₄, R₆ is 3-chloro-4-methoxyhenryl, R₉ is isobutyl, R₁₀ is hydrogen, and X and Y are oxygen.

10. The compound of claim 1, wherein Ar is phenyl, R₁ and R₂ are taken together to form an *R*,*R*-epoxide ring, R₃, R₇ and R₈ are methyl, R₄ and R₅ are taken together to form a double bond between C₁₃ and C₁₄, R₆ is 3-chloro-4-methoxybenzyl, R₉ is isobutyl, R₁₀ is hydrogen, and X and Y are oxygen.

11. The compound of claim 1, wherein Ar is phenyl, R₁ and R₂ are taken together to form an *S,S*-epoxide ring, R₃, R₇ and R₈ are methyl, R₄ and R₅ are taken together to form a double bond between C₁₃ and C₁₄, R₆ is 3-chloro-4-methoxybenzyl, R₉ is isobutyl, R₁₀ is hydrogen, and X and Y are oxygen.

12. The compound of claim 1, wherein Ar is phenyl, R₁ is *S*-chloro, R₂ is *R*-hydroxyl, R₃, R₇ and R₈ are methyl, R₄ and R₅ are taken together to form a double bond between C₁₃ and C₁₄, R₆ is 3-chloro-4-methoxybenzyl, R₉ is isobutyl, R₁₀ is hydrogen, and X and Y are oxygen.

13. The compound of claim 1, wherein Ar is phenyl, R₁ and R₂ are taken together to form an *R*,*R*-epoxide ring, R₃, R₇ and R₈ are methyl, R₄ and R₅ are hydrogen, R₆ is 3-chloro-4-methoxybenzyl, R₉ is isobutyl, R₁₀ is hydrogen, and X and Y are oxygen.

14. The compound of claim 1, wherein Ar is phenyl, R₁ is *S*-chloro, R₂ is *R*-hydroxyl, R₃, R₇ and R₈ are methyl, R₄ and R₅ are hydrogen, R₆ is 3-chloro-4-methoxybenzyl, R₉ is isobutyl, R₁₀ is hydrogen, and X and Y are oxygen.

15. The compound of claim 1, wherein Ar is phenyl, R₁ and R₂ are taken together to form an *R*,*R*-episulfide ring, R₃, R₇ and R₈ are methyl, R₄ and R₅ are taken together to form a double bond between C₁₃ and C₁₄, R₆ is 3-chloro-4-methoxybenzyl, R₉ is isobutyl, R₁₀ is hydrogen, and X and Y are oxygen.

16. A compound having the structure of Formula 1, wherein Ar is phenyl, R₁ and R₂ are taken together to form an R,R-aziridine ring, R₃, R₇ and R₈ are methyl, R₄ and R₅ are taken together to form a double bond between C₁₃ and C₁₄, R₆ is 3-chloro-4-methoxybenzyl, R₉ is isobutyl, R₁₀ is hydrogen, and X and Y are oxygen.

17. A method for producing a cryptophycin compound according to any one of claims 1-16 comprising the following steps:
selecting an allylically substituted E alkene;
rearranging the allylically E alkene via a stereospecific Wittig rearrangement;
converting this compound to a first δ-amino acid or δ-hydroxy acid;
coupling the first acid to a second α-amino acid to form a first subunit;
coupling a third β-amino acid to a fourth α-hydroxy acid or α-amino acid to form a second subunit; and
coupling the first subunit to the second subunit to form a cryptophycin.

18. A pharmaceutical composition useful for inhibiting the proliferation of a hyperproliferative mammalian cell comprising an effective amount of a compound according to any of claims 1 to 16 together with a pharmaceutically acceptable carrier.

19. The pharmaceutical composition of claim 18 further comprising at least one additional anti-neoplastic agent.

20. Use of a cryptophycin compound according to any of claims 1 to 16 in the preparation of a medicament for inhibiting the proliferation of a mammalian cell.

21. Use according to claim 20 which medicament further comprises at least one additional anti-neoplastic agent.

22. Use of a canpound according to any of claims 1 to 16 in the preparation of a medicament for inhibiting the proliferation of a hyperproliferative mammalian cell having a multiple. drug resistant phenotype.

23. Use according to claim 22, which medicament further comprises at least one additional anti-neoplastic agent.

24. The pharmaceutical composition of claim 18 further comprising at least one additional therapeutic agent directed to inhibiting said proliferation.

## Patentansprüche

1. Kryptophycin mit der folgenden Struktur: 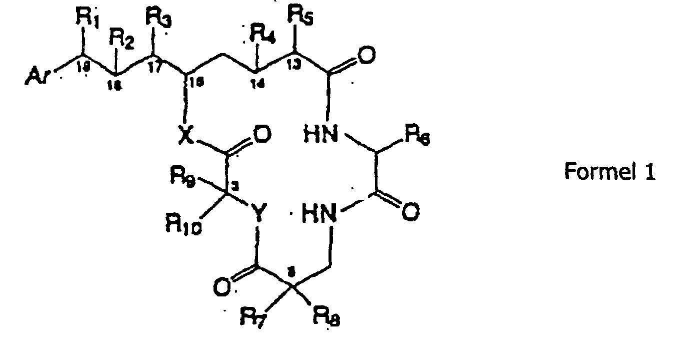 wobei
Ar eine Methyl- oder Phenyl- oder aromatische Gruppe mit 4n+2 π-Elektronen in einem monocyclischen oder bicyclischen konjugierten System oder eine mit einer einzigen Gruppe oder einer heteroaromatischen Gruppe substituierte Phenylgruppe ist;
R₁ ein Halogenatom, SH, Amino, Monoalkylamino, Dialkylamino, Trialkylammonium; Alkylthio, Dialkylsulfonium, Sulfat oder Phosphat ist;
R₂ OH oder SH ist; oder
R₁ und R₂ zusammen einen Epoxidring, einen Aziridinring, einen Episulfid-Ring, einen Sulfatring oder einen Monoalkylphosphatring bilden können; oder
R₁ und R₂ zusammen eine Doppelbindung zwischen C₁₈ und C₁₉ bilden können; R₃, R₇, R₈ jeweils Alkylgruppen mit 1 bis 5 Kohlenstoffatomen, einschließlich linearer oder nichtlinearer Kohlenwasserstoffketten, sind;
R₄ und R₅ H sind; oder
R₄ und R₅ zusammen eine Doppelbindung zwischen C₁₃ und C₁₄ bilden können; R₆ eine Benzyl-, Hydroxybenzyl-, Alkoxybenzyl-, Halogenhydroxybenzyl-, Dihalogenhydroxybenzyl-, Halgenalkoxybenzyl- oder Dihalogenalkoxybenzylgruppe ist;
R₉ und R₁₀ jeweils unabhängig H oder eine Alkylgruppe mit 1 bis 5 Kohlenstoffatomen, einschließlich linearer und nichtlinearer Kohlenwasserstoffketten, sind; und
X und Y jeweils unabhängig O, NH oder Alkylamino sind.

2. Kryptophycin nach Anspruch 1, wobei R₈ Ethyl, Propyl, Isopropyl, Butyl, Isobutyl, Pentyl oder Isopentyl ist.

3. Kryptophycin nach Anspruch 1, wobei R₇ Ethyl, Propyl, Isopropyl, Butyl, Isobutyl, Pentyl oder Isopentyl ist.

4. Kryptophycin nach Anspruch 1, wobei R₃ Ethyl, Propyl, Isopropyl, Butyl, Isobutyl, Pentyl oder Isopentyl ist.

5. Kryptophycin nach Anspruch 1, wobei R₉ Methyl, Ethyl, Propyl, Isopropyl, Butyl, Isobutyl, Pentyl oder Isopentyl ist.

6. Kryptophycin nach Anspruch 1, wobei R₁₀ Methyl, Ethyl, Propyl, Isopropyl, Butyl, Isobutyl, Pentyl oder Isopentyl ist.

7. Verbindung nach Anspruch 1, wobei zumindest eine der Gruppen, die an C₃, C₆, C₁₀, C₁₆, C₁₇ und C₁₈ gebunden ist, eine R-Stereochemie aufweist.

8. Verbindung nach Anspruch 1, wobei zumindest eine der Gruppen, die an C₃, C₆, C₁₀, C₁₆, C₁₇ und C₁₈ gebunden ist, eine S-Stereochemie aufweist.

9. Verbindung nach Anspruch 1, wobei Ar Phenyl ist, R₁ und R₂ zusammen eine Doppelbindung zwischen C₁₈ und C₁₉ bilden, R₃, R₇ und R₈ Methyl sind, R₄ und R₅ zusammen eine Doppelbindung zwischen C₁₃ und C₁₄ bilden, R₆ 3-Chlor-4-methoxybenzyl ist, R₉ Isobutyl ist, R₁₀ Wasserstoff ist und X und Y Sauerstoff sind.

10. Verbindung nach Anspruch 1, wobei Ar Phenyl ist, R₁ und R₂ zusammen einen R,R-Epoxidring bilden, R₃, R₇ und R₈ Methyl sind, R₄ und R₅ zusammen eine Doppelbindung zwischen C₁₃ und C₁₄ bilden, R₆ 3-Chlor-4-methoxybenzyl ist, R₉ Isobutyl ist, R₁₀ Wasserstoff ist und X und Y Sauerstoff sind.

11. Verbindung nach Anspruch 1, wobei Ar Phenyl ist, R₁ und R₂ zusammen einen S,S-Epoxidring bilden, R₃, R₇ und R₈ Methyl sind, R₄ und R₅ zusammen eine Doppelbindung zwischen C₁₃ und C₁₄ bilden, R₆ 3-Chlor-4-methoxybenzyl ist, R₉ Isobutyl ist, R₁₀ Wasserstoff ist und X und Y Sauerstoff sind.

12. Verbindung nach Anspruch 1, wobei Ar Phenyl ist, R₁ S-Chlor ist, R₂ R-Hydroxyl ist, R₃, R₇ und R₈ Methyl sind, R₄ und R₅ zusammen eine Doppelbindung zwischen C₁₃ und C₁₄ bilden, R₆ 3-Chlor-4-methoxybenzyl ist, R₉ Isobutyl ist, R₁₀ Wasserstoff ist und X und Y Sauerstoff sind.

13. Verbindung nach Anspruch 1, wobei Ar Phenyl ist, R₁ und R₂ zusammen einen R,R-Epoxidring bilden, R₃, R₇ und R₈ Methyl sind, R₄ und R₅ Wasserstoff sind, R₆ 3-Chlor-4-methoxybenzyl ist, R₉ Isobutyl ist, R₁₀ Wasserstoff ist und X und Y Sauerstoff sind.

14. Verbindung nach Anspruch 1, wobei Ar Phenyl ist, R₁ S-Chlor ist, R₂ R-Hydroxyl ist, R₃, R₇ und R₈ Methyl sind, R₄ und R₅ Wasserstoff sind, R₆ 3-Chlor-4-methoxybenzyl ist, R₉ Isobutyl ist, R₁₀ Wasserstoff ist und X und Y Sauerstoff sind.

15. Verbindung nach Anspruch 1, wobei Ar Phenyl ist, R₁ und R₂ zusammen einen R,R-Episulfidring bilden, R₃, R₇ und R₈ Methyl sind, R₄ und R₅ zusammen eine Doppelbindung zwischen C₁₃ und C₁₄ bilden, R₆ 3-Chlor-4-methoxybenzyl ist, R₉ Isobutyl ist, R₁₀ Wasserstoff ist und X und Y Sauerstoff sind.

16. Verbindung mit der Struktur gemäß Formel 1, wobei Ar Phenyl ist, R₁ und R₂ zusammen einen R,R-Aziridinring bilden, R₃, R₇ und R₈ Methyl sind, R₄ und R₅ zusammen eine Doppelbindung zwischen C₁₃ und C₁₄ bilden, R₆ 3-Chlor-4-methoxybenzyl ist, R₉ Isobutyl ist, R₁₀ Wasserstoff ist und X und Y Sauerstoff sind.

17. Verfahren zur Herstellung einer Kryptophycinverbindung nach einem der Ansprüche 1 bis 16, das die folgenden Schritte aufweist:
Auswählen eines Allyl-substituierten E-Alkens;
Umgruppieren des Allyl-substituierten E-Alkens durch eine stereospezifische Wittig-Umgruppierung;
Überführen dieser Verbindung in eine erste δ-Aminosäure oder δ-Hydroxysäure;
Koppeln der ersten Säure mit einer zweiten α-Aminosäure, um eine erste Teileinheit zu bilden;
Koppeln einer dritten β-Aminosäure mit einer vierten α-Hydroxysäure oder α-Aminosäure, um eine zweite Teileinheit zu bilden; und
Koppeln der ersten Teileinheit mit der zweiten Teileinheit, wodurch ein Kryptophycin erzeugt wird.

18. Pharmazeutische Zusammensetzung, die für die Hemmung der Proliferation einer hyperproliferativen Mammaliazelle vorteilhaft ist, die eine wirksame Menge einer Verbindung nach einem der Ansprüche 1 bis 16 zusammen mit einem pharmazeutisch akzeptablen Träger aufweist.

19. Pharmazeutische Zusammensetzung nach Anspruch 18, die ferner mindestens ein weiteres antineoplastisches Mittel aufweist.

20. Verwendung einer Kryptophycinverbindung nach einem der Ansprüche 1 bis 16 bei der Herstellung eines Medikamentes für die Hemmung der Proliferation einer Mammaliazelle.

21. Verwendung nach Anspruch 20, wobei das Medikament ferner mindestens ein weiteres antineoplastisches Mittel aufweist.

22. Verwendung einer Zusammensetzung nach einem der Ansprüche 1 bis 16 bei der Herstellung eines Medikamentes für die Hemmung der Proliferation einer hyperproliferativen Mammaliazelle mit einem mehrfach arnzeimittelresistenten Phenotyp.

23. Verwendung nach Anspruch 22, wobei das Medikament ferner mindestens ein weiteres antineoplastisches Mittel aufweist.

24. Pharmazeutische Zusammensetzung nach Anspruch 18, die ferner mindestens ein weiteres therapeutisches Mittel aufweist, das auf die Hemmung dieser Proliferation ausgerichtet ist.

## Revendications

1. Cryptophycine de structure suivante : 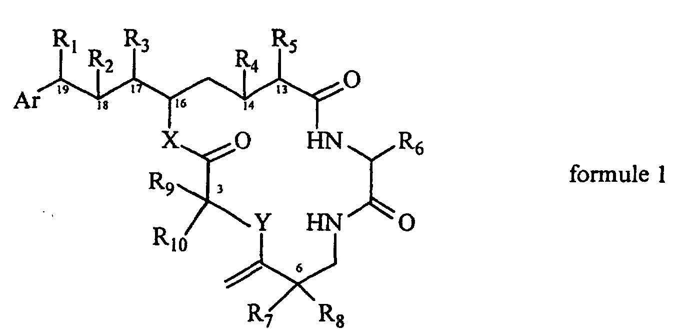 dans laquelle
Ar représente un groupe méthyle ou phényle, un groupe aromatique contenant 4n+2 électrons π dans un système conjugué monocyclique ou bicyclique, ou un groupe phényle portant un unique substituant, ou encore un groupe hétéroaromatique ;
R₁ représente un atome d'halogène ou un groupe sulfanyle -SH, amino, monoalkylamino, dialkylamino, trialkylammonium, alkylthio, dialkylsulfonium, sulfate ou phosphate ;
R₂ représente un groupe hydroxyle -OH ou sulfanyle -SH ;
ou R₁ et R₂ représentent conjointement un cycle époxyde, aziridine, épi-sulfure, sulfate ou monoalkyl-phosphate ;
ou encore R₁ et R₂ représentent conjointement une double liaison entre les atomes de carbone n° 18 et 19 ;
R₃, R₇ et R₈ représentent chacun un groupe alkyle comportant de 1 à 5 atomes de carbone, y compris les groupes à chaîne hydrocarbonée linéaire et les groupes à chaîne hydrocarbonée non linéaire ;
R₄ et R₅ représentent des atomes d'hydrogène, ou bien R₄ et R₅ représentent conjointement une double liaison entre les atomes de carbone n° 13 et 14 ;
R₆ représente un groupe benzyle, hydroxybenzyle, alcoxybenzyle, halogénohydroxybenzyle, dihalogénohydroxybenzyle, halogénoalcoxybenzyle ou dihalogénoalcoxybenzyle ;
R₉ et R₁₀ représentent chacun, indépendamment, un atome d'hydrogène ou un groupe alkyle comportant de 1 à 5 atomes de carbone, y compris les groupes à chaîne hydrocarbonée linéaire et les groupes à chaîne hydrocarbonée non linéaire ;
et X et Y représentent chacun, indépendamment, un atome d'oxygène, un groupe imino -NH- ou un groupe alkyl-amino.

2. Cryptophycine conforme à la revendication 1, dans laquelle R₈ représente un groupe éthyle, propyle, isopropyle, butyle, isobutyle, pentyle ou isopentyle.

3. Cryptophycine conforme à la revendication 1, dans laquelle R₇ représente un groupe éthyle, propyle, isopropyle, butyle, isobutyle, pentyle ou isopentyle.

4. Cryptophycine conforme à la revendication 1, dans laquelle R₃ représente un groupe éthyle, propyle, isopropyle, butyle, isobutyle, pentyle ou isopentyle.

5. Cryptophycine conforme à la revendication 1, dans laquelle R₉ représente un groupe méthyle, éthyle, propyle, butyle, isobutyle, pentyle ou isopentyle.

6. Cryptophycine conforme à la revendication 1, dans laquelle R₁₀ représente un groupe méthyle, éthyle, propyle, butyle, isobutyle, pentyle ou isopentyle.

7. Composé conforme à la revendication 1, dans lequel au moins l'un des substituants liés aux atomes de carbone n° 3, 6, 10, 16, 17 et 18 se trouve en configuration *R*.

8. Composé conforme à la revendication 1, dans lequel au moins l'un des substituants liés aux atomes de carbone n° 3, 6, 10, 16, 17 et 18 se trouve en configuration *S*.

9. Composé conforme à la revendication 1, dans lequel Ar représente un groupe phényle, R₁ et R₂ représentent conjointement une double liaison entre les atomes de carbone n° 18 et 19, R₃, R₇ et R₈ représentent des groupes méthyle, R₄ et R₅ représentent conjointement une double liaison entre les atomes de carbone n° 13 et 14, R₆ représente un groupe 3-chloro-4-méthoxybenzyle, R₉ représente un groupe isobutyle, R₁₀ représente un atome d'hydrogène, et X et Y représentent des atomes d'oxygène.

10. Composé conforme à la revendication 1, dans lequel Ar représente un groupe phényle, R₁ et R₂ représentent conjointement un cycle *R,R*- époxyde, R₃, R₇ et R₈ représentent des groupes méthyle, R₄ et R₅ représentent conjointement une double liaison entre les atomes de carbone n° 13 et 14, R₆ représente un groupe 3-chloro-4-méthoxybenzyle, R₉ représente un groupe isobutyle, R₁₀ représente un atome d'hydrogène, et X et Y représentent des atomes d'oxygène.

11. Composé conforme à la revendication 1, dans lequel Ar représente un groupe phényle, R₁ et R₂ représentent conjointement un cycle *S,S-*époxyde, R₃, R₇ et R₈ représentent des groupes méthyle, R₄ et R₅ représentent conjointement une double liaison entre les atomes de carbone n° 13 et 14, R₆ représente un groupe 3-chloro-4-méthoxybenzyle, R₉ représente un groupe isobutyle, R₁₀ représente un atome d'hydrogène, et X et Y représentent des atomes d'oxygène.

12. Composé conforme à la revendication 1, dans lequel Ar représente un groupe phényle, R₁ représentent un substituant *S*-chloro, R₂ représente un substituant *R*-hydroxyle, R₃, R₇ et R₈ représentent des groupes méthyle, R₄ et R₅ représentent conjointement une double liaison entre les atomes de carbone n° 13 et 14, R₆ représente un groupe 3-chloro-4-méthoxybenzyle, R₉ représente un groupe isobutyle, R₁₀ représente un atome d'hydrogène, et X et Y représentent des atomes d'oxygène.

13. Composé conforme à la revendication 1, dans lequel Ar représente un groupe phényle, R₁ et R₂ représentent conjointement un cycle *R,R*-époxyde, R₃, R₇ et R₈ représentent des groupes méthyle, R₄ et R₅ représentent des atomes d'hydrogène, R₆ représente un groupe 3-chloro-4-méthoxybenzyle, R₉ représente un groupe isobutyle, R₁₀ représente un atome d'hydrogène, et X et Y représentent des atomes d'oxygène.

14. Composé conforme à la revendication 1, dans lequel Ar représente un groupe phényle, R₁ représentent un substituant *S*-chloro, R₂ représente un substituant *R*-hydroxyle, R₃, R₇ et R₈ représentent des groupes méthyle, R₄ et R₅ représentent des atomes d'hydrogène, R₆ représente un groupe 3-chloro-4-méthoxy-benzyle, R₉ représente un groupe isobutyle, R₁₀ représente un atome d'hydrogène, et X et Y représentent des atomes d'oxygène.

15. Composé conforme à la revendication 1, dans lequel Ar représente un groupe phényle, R₁ et R₂ représentent conjointement un cycle *R,R*-épisulfure, R₃, R₇ et R₈ représentent des groupes méthyle, R₄ et R₅ représentent conjointement une double liaison entre les atomes de carbone n° 13 et 14, R₆ représente un groupe 3-chloro-4-méthoxybenzyle, R9 représente un groupe isobutyle, R₁₀ représente un atome d'hydrogène, et X et Y représentent des atomes d'oxygène.

16. Composé possédant la structure représentée par la formule 1, dans lequel Ar représente un groupe phényle, R₁ et R₂ représentent conjointement un cycle *R,R*-aziridine, R₃, R₇ et R₈ représentent des groupes méthyle, R₄ et R₅ représentent conjointement une double liaison entre les atomes de carbone n° 13 et 14, R₆ représente un groupe 3-chloro-4-méthoxybenzyle, R₉ représente un groupe isobutyle, R₁₀ représente un atome d'hydrogène, et X et Y représentent des atomes d'oxygène.

17. Procédé de préparation d'une cryptophycine conforme à l'une des revendications 1 à 16, comportant les étapes suivantes :
- choisir un E-alcène à substitution allylique ;
- faire subir à cet E-alcène à substitution allylique un réarrangement stéréospécifique de Wittig ;
- convertir le composé ainsi obtenu en un premier acide δ-aminé ou δ-hydroxylé ;
- coupler ce premier acide avec un deuxième acide α-aminé, pour obtenir une première sous-unité ;
- coupler un troisième acide β-aminé avec un quatrième acide α-aminé ou α-hydroxylé, pour obtenir une deuxième sous-unité ;
- et coupler la première sous-unité avec la deuxième sous-unité, de manière à former une cryptophycine.

18. Composition pharmaceutique, utilisable pour inhiber la prolifération d'une cellule hyperproliférante de mammifère et contenant, en une quantité efficace, un composé conforme à l'une des revendications 1 à 16, associé à un véhicule admissible en pharmacie.

19. Composition pharmaceutique conforme à la revendication 18, qui contient en outre au moins un agent antinéoplasique supplémentaire.

20. Emploi d'une cryptophycine conforme à l'une des revendica-tions 1 à 16 dans la préparation d'un médicament servant à inhiber la prolifération d'une cellule de mammifère.

21. Emploi conforme à la revendication 20, ledit médicament contenant en outre au moins un agent antinéoplasique supplémentaire.

22. Emploi d'un composé conforme à l'une des revendications 1 à 16 dans la préparation d'un médicament servant à inhiber la prolifération d'une cellule hyperproliférante de mammifère qui présente un phénotype de multirésistance aux médicaments.

23. Emploi conforme à la revendication 22, ledit médicament contenant en outre au moins un agent antinéoplasique supplémentaire.

24. Composition pharmaceutique conforme à la revendication 18, qui contient en outre au moins un agent thérapeutique supplémentaire qui sert aussi à inhiber ladite prolifération.
